(19) Europäisches Patentamt
European Patent Office
Office européen des brevets

(11) EP 3 650 440 A1

(12) EUROPEAN PATENT APPLICATION
published in accordance with Art. 153(4) EPC

(43) Date of publication:
**13.05.2020 Bulletin 2020/20**

(21) Application number: **19842579.5**

(22) Date of filing: **26.08.2019**

(51) Int Cl.:
***C07C 279/18*** (2006.01) ***A61K 31/235*** (2006.01)
***A61P 3/04*** (2006.01) ***A61P 3/10*** (2006.01)
***A61P 43/00*** (2006.01)

(86) International application number:
**PCT/JP2019/033215**

(87) International publication number:
**WO 2020/045326 (05.03.2020 Gazette 2020/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2018 JP 2018157901**

(71) Applicant: **Scohia Pharma, Inc.**
**Fujisawa-shi, Kanagawa 251-8555 (JP)**

(72) Inventors:
- **HARA, Ryoma**
**Fujisawa-shi, Kanagawa 2518555 (JP)**
- **ASANO, Kohei**
**Fujisawa-shi, Kanagawa 2518555 (JP)**
- **NIIDA, Ayumu**
**Fujisawa-shi, Kanagawa 2518555 (JP)**
- **KASAI, Shizuo**
**Fujisawa-shi, Kanagawa 2518555 (JP)**
- **MAEKAWA, Tsuyoshi**
**Fujisawa-shi, Kanagawa 2518555 (JP)**

(74) Representative: **Wesela-Bauman, Grzegorz**
**JWP**
**Rzecznicy Patentowi Dorota Rzazewska sp.k.**
**Sienna Center**
**Ul. Zelazna 28/30**
**00-833 Warszawa (PL)**

# (54) BENZOIC ESTER COMPOUND

(57) The present invention provides a benzoic acid ester compound that has an enteropeptidase inhibitory effect, and use of the compound as a medicament for the treatment or prevention of obesity, diabetes mellitus, or the like.

A compound represented by the formula (I) or a salt thereof has an enteropeptidase inhibitory effect and is useful as a medicament for the treatment or prevention of obesity, diabetes mellitus, or the like:

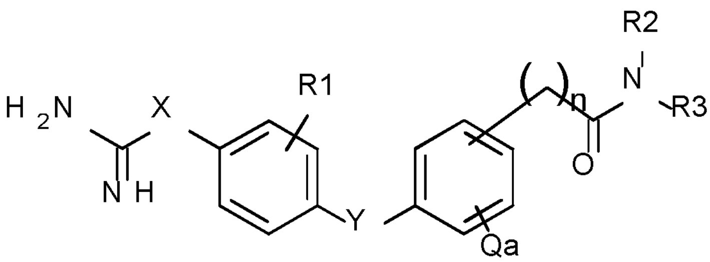

EP 3 650 440 A1

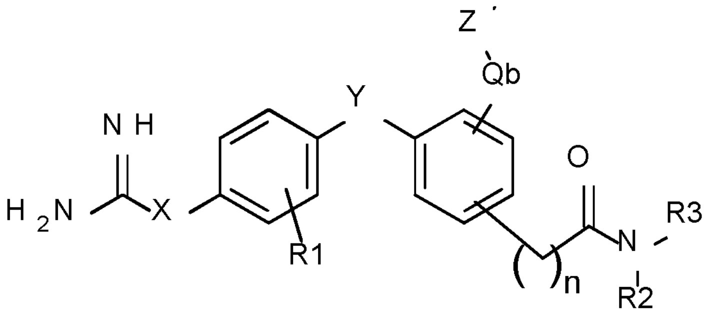

(I)

[in the formula, each symbol is as defined in the specification] .

SEQUENCE LISTING

<110> SCOHIA PHARMA, Inc

<120> Benzoic acid ester Compounds

<130> G2061WO

<150> JP2018-157901
<151> 2018-08-27

<160> 1

<170> PatentIn version 3.5

<210> 1
<211> 13
<212> PRT
<213> Artificial Sequence

<220>
<223> Synthetic Peptide

<220>
<221> MISC_FEATURE
<222> (1)..(1)
<223> Xaa stands for Abu (2-Aminobutyric acid)

<220>
<221> MOD_RES
<222> (1)..(1)
<223> modified with 5FAM

<220>
<221> MOD_RES
<222> (11)..(11)
<223> modified with CPQ2

<400> 1

```
Xaa Gly Asp Asp Asp Lys Ile Val Gly Gly Lys Lys Lys
1               5                   10
```

## Description

## Technical Field

The present invention relates to a benzoate compound that has an enteropeptidase inhibitory effect and is useful in the treatment or prevention of obesity, diabetes mellitus, or the like, and a medicament comprising the same.

## Background of Invention

Enteropeptidase is a serine protease that converts trypsinogen secreted from the pancreas after meal to trypsin. Trypsin in a state activated by enteropeptidase then activates protease precursors such as chymotrypsinogen, procarboxypeptidase, and proelastase. These activated proteases decompose dietary proteins into amino acid units. The resulting amino acids are absorbed into the small intestine. Thus, enteropeptidase inhibitors are capable of suppressing the decomposition or absorption of proteins and are useful as a drug for treating obesity.

Examples of substances having an effect related to an enteropeptidase inhibitor include the following.

(1) A compound or a salt thereof which has an enteropeptidase inhibitory effect and is useful in the treatment or prevention of a disease related to obesity or abnormal fat metabolism, the compound being represented by the following formula:

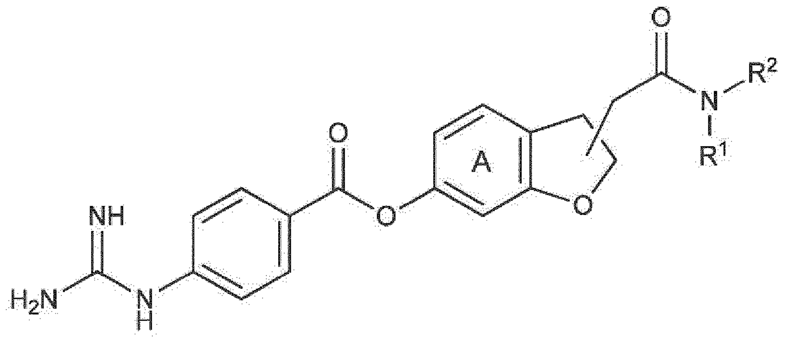

(I)

wherein

ring A represents a benzene ring optionally substituted by 1 to 3 substituents selected from a halogen atom, a $C_{1-6}$ alkyl group optionally having a substituent and a $C_{1-6}$ alkoxy group optionally having a substituent;
$R^1$ represents a hydrogen atom or a $C_{1-6}$ alkyl group substituted by COOH; and
$R^2$ represents a $C_{1-6}$ alkyl group substituted by one or two COOH and optionally further substituted by $SO_3H$ (Patent Literature 1: WO2015/122187).

(2) A compound or a salt thereof which is a condensed heterocyclic compound having an enteropeptidase inhibitory effect and is useful as a medicament for the treatment or prevention of obesity, diabetes mellitus, or the like, the compound being represented by the following formula:

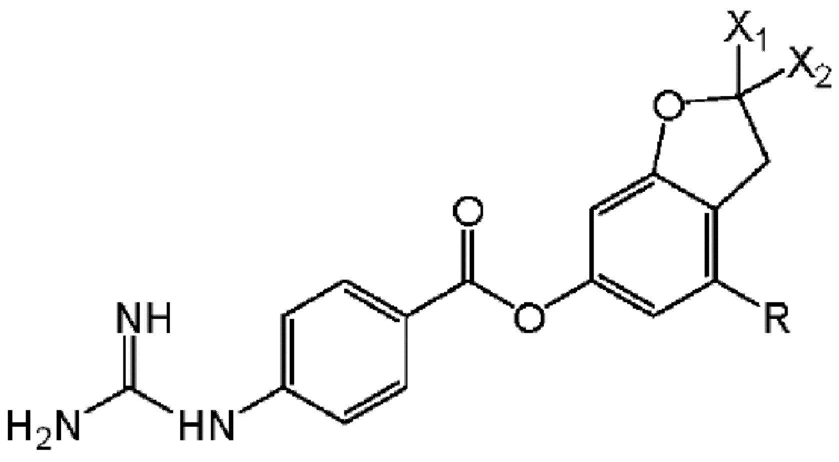

(I)

wherein

R represents

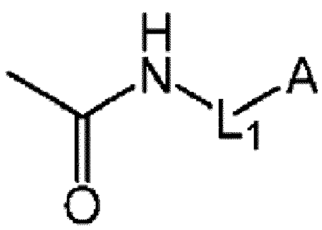

or

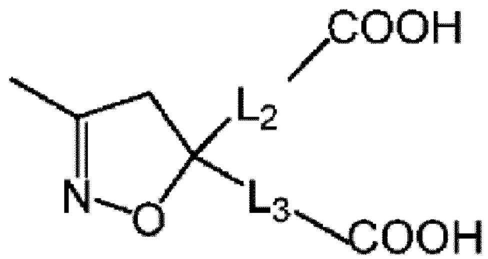

$L_1$ represents a $C_{1-6}$ alkylene group;
$L_2$ and $L_3$ are the same or different and each represent a bond or a $C_{1-6}$ alkylene group;
A represents $-S(O)_2OH$ or $-P(O)(OH)_2$; and
$X_1$ and $X_2$ are the same or different and each represent H or a $C_{1-6}$ alkyl group
(Patent Literature 2: WO2016/158788).

(3) A compound or a salt thereof which has an enteropeptidase inhibitory effect and is a condensed heterocyclic compound useful in the treatment or prevention of obesity, diabetes mellitus, or the like and is useful as a medicament comprising the compound, the compound being represented by the following formula:

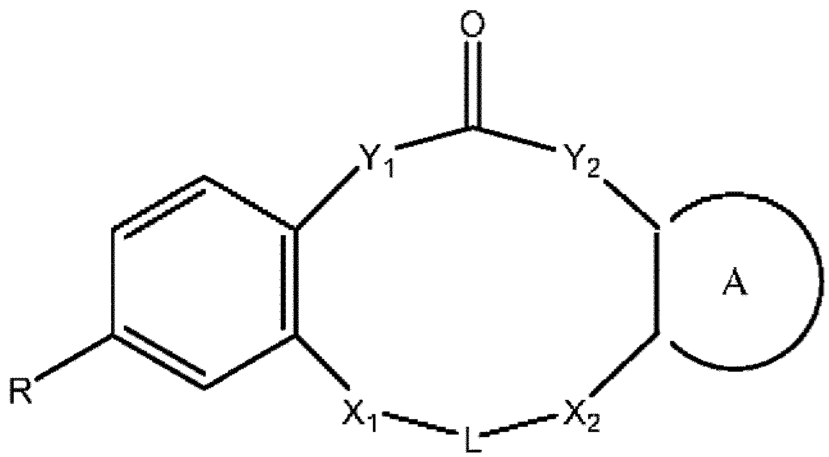

(I)

wherein

ring A represents an optionally substituted 5- or 6-membered aromatic ring, wherein a substituent on the ring A optionally forms an optionally substituted ring together with the atoms constituting the ring A;
L represents a bond or a $C_{1-6}$ alkylene group;
$X_1$ and $X_2$ are the same or different and each represent -O- or a bond;
R represents a guanidino group or an amidino group;
one of $Y_1$ and $Y_2$ is -O-, and the other moiety is a bond,
when R is a guanidino group, $Y_1$ represents a bond and $Y_2$ represents -O-, and
when R is an amidino group, $Y_1$ represents -O- and $Y_2$ represents a bond
(Patent Literature 3: WO2016/104630).

(4) A compound which has a serine protease inhibitory effect and is useful in the treatment or prevention of obesity, hyperlipidemia, diabetes mellitus, diabetic complications, or metabolic syndrome, the compound being represented by the following formula:

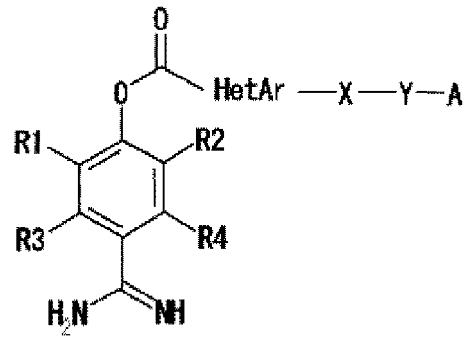

wherein

R1, R2, R3, and R4 each represent H or the like;
HetAr represents an optionally substituted heteroaromatic ring;
X represents optionally substituted lower alkylene or the like;
Y represents carbonyl or the like;
A represents

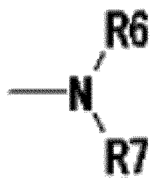

or the like; and
R6 and R7 each represent H, optionally substituted lower alkyl, or the like
(Patent Literature 4: WO2011/071048).

## Summary of Invention

### Technical Problem

An object of the present invention is to provide a benzoate compound that has an excellent enteropeptidase inhibitory effect and is useful in the treatment or prevention of obesity, diabetes mellitus, or the like, and a medicament comprising the same.

### Solution to Problem

The present inventors have conducted diligent studies to attain the object and consequently completed the present invention by finding that a compound represented by the formula (I) given below has an excellent enteropeptidase inhibitory effect.

Specifically, the present invention is as follows:

<1> A compound represented by the formula (I) or a salt thereof:

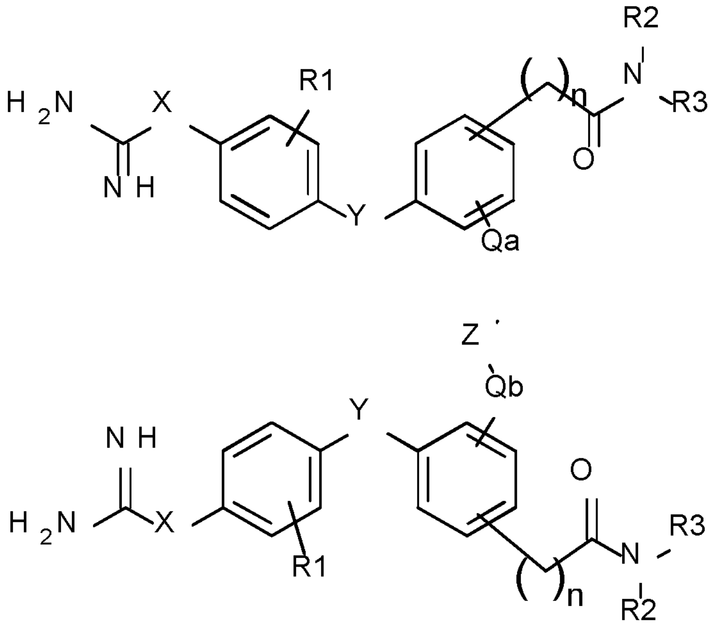

**(I)**

wherein each symbol represents the following meaning:

R1: H, halogen, a $C_{1-6}$ alkyl group optionally having a substituent(s), or a $C_{1-6}$ alkoxy group optionally having a substituent(s);
R2: H or a $C_{1-6}$ alkyl group optionally substituted by a carboxyl group;
R3: a $C_{1-8}$ alkyl group having one substituent selected from the group consisting of a carboxyl group, a sulfonic acid group, and a phosphoric acid group and optionally further substituted by one substituent selected from the group consisting of a carboxyl group, NHR4 and a hydroxy group;
R4: H or a $C_{1-6}$ alkyl group optionally having a substituent(s);
n: an integer of 0 to 2;
X: a bond or NH;
Y: **-C(=O)O- or **-OC(=O)- (wherein ** is bonded to the benzene ring substituted by a guanidino group or a carbamimidoyl group);
Qa and Qb: the same or different, $-(CH_2)_mO-$*, $-(CH_2)_mNR5-$*, $-(CH_2)_mNR5C(=O)-$*, -C(=O)NR5-*, -S-*, or $-SO_2-$* (wherein * is bonded to Z);
R5: H or a $C_{1-6}$ alkyl group optionally having a substituent(s);
m: an integer of 0 to 6;
Z: $-[(CR6aR6b)_q-A]_r-(CR7_aR7b)_s-$;
A: O, S, $SO_2$, NR8, -C(=O)-, -OC(=O)-, -C(=O)O-, - NR8C(=O)-, or C(=O)-NR8;
R6a, R6b, R7a and R7b: the same or different, H, halogen, a hydroxy group, or a $C_{1-6}$ alkyl group optionally having a substituent(s), or a $C_{1-6}$ alkoxy group optionally having a substituent(s);
R8: H or a $C_{1-6}$ alkyl group optionally having a substituent(s);
q: an integer of 1 to 6;
r: an integer of 0 to 50; and
s: an integer of 1 to 6.

<2> The compound according to <1> or a salt thereof, wherein X is NH, and Y is **-C(=O)O-.
<3> The compound according to <1> or <2> or a salt thereof, wherein each of R1 and R2 is a hydrogen atom, R3 is a $C_{1-8}$ alkyl group substituted by two carboxyl groups.
<4> The compound according to any one of <1> to <3> or a salt thereof, wherein each of Qa and Qb is $-(CH_2)_mO-$

*, m is an integer of 0 to 3, Z is $-[(CR6aR6b)_q-O]_r-(CR7aR7b)_s-$, each of q and s is an integer of 1 to 6, and r is an integer of 0 to 10.

<5> The compound according to <1> or a salt thereof, wherein each of Qa and Qb is O, Z is $-[(CH_2)_2-O]_r-(CH_2)_2-$, r is an integer of 0 to 3, X is NH, Y is ** - C(=O)O-, each of R1 and R2 is a hydrogen atom, R3 is a $C_{1-8}$ alkyl group substituted by two carboxyl groups, and n is 0.

<6> (2S)-2-[[3-[2-[2-[2-[3-[[(1S)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof.

<7> (2R) -2-[[3-[2-[2-[2-[3-[[(1R)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof.

<8> (2S)-2-[[3-[2-[2-[3-[[(1S)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof.

<9> (2R) -2-[[3-[2-[2-[3-[[(1R)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof.

<10> (2R)-2-[[3-[2-[3-[[(1R)-1,2-Dicarboxyethyl]carbamoyl]-5-(4-guanidinobenzoyl)oxyphenoxy]ethoxy]-5-(4-guanidinobenzoyl)oxybenzoyl]amino]butanedioic acid or a salt thereof.

<11> (2S)-2-[[3-[2-[3-[[(1S)-1,2-Dicarboxyethyl]carbamoyl]-5-(4-guanidinobenzoyl)oxyphenoxy]ethoxy]-5-(4-guanidinobenzoyl)oxybenzoyl]amino]butanedioic acid or a salt thereof.

<12> (2R) -2-[[2-[2-[2-[2-[[(1R)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof.

<13> (2S)-2-[[2-[2-[2-[2-[[(1S)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof.

<14> (2R)-2-[[2-[2-[2-[[(1R)-1,2-Dicarboxyethyl]carbamoyl]-5-(4-guanidinobenzoyl)oxyphenoxy]ethoxy]-4-(4-guanidinobenzoyl)oxybenzoyl]amino]butanedioic acid or a salt thereof.

<15> 2-[[3-[2-[2-[2-[3-[[1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof, or
2-[[3-[2-[3-[[1,2-dicarboxyethyl]carbamoyl]-5-(4-guanidinobenzoyl)oxyphenoxylethoxyl-5-(4-guanidinobenzoyl)oxybenzoyl]amino]butanedioic acid or a salt thereof.

<16> 2-[[3-[2-[2-[3-[[1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof;
2-[[2-[2-[2-[2-[[1,2-dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof; or
2-[[2-[2-[2-[[1,2-dicarboxyethyl]carbamoyl]-5-(4-guanidinobenzoyl)oxyphenoxy]ethoxy]-4-(4-guanidinobenzoyl)oxybenzoyl]amino]butanedioic acid or a salt thereof.

<17> A medicament comprising a compound according to any one of <1> to <16> or a salt thereof.

<18> The medicament according to <17>, wherein the medicament is an enteropeptidase inhibitor.

<19> The medicament according to <17>, wherein the medicament is an agent for preventing or treating obesity.

<20> The medicament according to <17>, wherein the medicament is an agent for preventing or treating diabetes mellitus.

<21> A method for preventing or treating obesity in a mammal, comprising administering an effective amount of a compound according to <1> or a salt thereof to the mammal.

<22> A method for preventing or treating diabetes mellitus in a mammal, comprising administering an effective amount of a compound according to <1> or a salt thereof to the mammal.

<23> A method for inhibiting enteropeptidase in a mammal, comprising administering an effective amount of a compound according to <1> or a salt thereof to the mammal.

<24> Use of a compound according to <1> or a salt thereof for manufacturing a medicament for preventing or treating obesity.

<25> Use of a compound according to <1> or a salt thereof for manufacturing a medicament for preventing or treating diabetes mellitus.

<26> The compound according to <1> or a salt thereof for use in the prevention or treatment of obesity.

<27> The compound according to <1> or a salt thereof for use in the prevention or treatment of diabetes mellitus.

### Advantageous Effects of Invention

Compound (I) has an excellent enteropeptidase inhibitory effect and is useful in the treatment or prevention of obesity, diabetes mellitus, or the like.

(Detailed Description of the Invention)

Hereinafter, the present invention will be described in detail.

Hereinafter, the definition of each substituent used in the present specification will be described in detail. Each substituent is defined as follows, unless otherwise specified.

In the present specification, examples of the "halogen atom" include fluorine, chlorine, bromine, and iodine.

In the present specification, examples of the "$C_{1-6}$ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl. Examples of the "$C_{1-8}$ alkyl group" include methyl, ethyl, propyl, isopropyl, butyl, isobutyl, pentyl, isopentyl, neopentyl, hexyl, isohexyl, heptyl, and octyl.

In the present specification, examples of the "$C_{1-6}$ alkoxy group" include methoxy, ethoxy, propoxy, isopropoxy, butoxy, isobutoxy, sec-butoxy, tert-butoxy, pentyloxy, and hexyloxy.

In the present specification, examples of the "$C_{1-6}$ alkyl group optionally having a substituent(s)" and the "$C_{1-6}$alkoxy group optionally having a substituent(s)" include a $C_{1-6}$ alkyl group and a $C_{1-6}$ alkoxy group optionally having a substituent(s) selected from the following substituent group A.

[Substituent group A]

(1) a halogen atom,
(2) a nitro group,
(3) a cyano group,
(4) an oxo group,
(5) a hydroxy group, and
(6) an optionally halogenated $C_{1-6}$ alkoxy group.

The number of the substituent described above in the "$C_{1-6}$ alkyl group optionally having a substituent(s)" and the "$C_{1-6}$ alkoxy group optionally having a substituent(s)" is, for example, 1 to 5, preferably 1 to 3. When the number of substituents is two or more, these substituents may be the same or different.

In the present specification, examples of the "optionally halogenated $C_{1-6}$ alkoxy group" include a $C_{1-6}$ alkoxy group optionally having 1 to 7, preferably 1 to 5 halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, and hexyloxy.

Hereinafter, each symbol of the formula (I) will be described.

Both of R1 represent H, a halogen atom, a $C_{1-6}$ alkyl group optionally having a substituent(s), or a $C_{1-6}$ alkoxy group optionally having a substituent(s). Preferably, R1 is H or halogen atom.

Both of R2 represent H or a $C_{1-6}$ alkyl group substituted by a carboxyl group. The $C_{1-6}$ alkyl group optionally substituted by carboxyl group optionally has 1 to 3 carboxyl groups (-COOH) as substituents. Preferably, R2 is H or a methyl group substituted by one carboxyl group.

Both of R3 represent a $C_{1-8}$ alkyl group having one substituent selected from the group consisting of a carboxyl group, a sulfonic acid group ($-SO_2$), and a phosphoric acid group ($-PO_4H_2$) and optionally further substituted by one substituent selected from the group consisting of a carboxyl group, NHR4 (wherein R4 represents H or a $C_{1-6}$ alkyl group optionally having a substituent(s) and is preferably H) and a hydroxy group. Preferably, R3 is a $C_{1-8}$ alkyl group (preferably a $C_{2-3}$ alkyl group, particularly preferably an ethyl group or a propyl group) substituted by one or two carboxyl groups, a $C_{1-8}$ alkyl group (preferably a $C_{2-3}$ alkyl group, particularly preferably an ethyl group or a propyl group) substituted by one $-SO_2$, or a $C_{1-8}$ alkyl group (preferably a $C_{4-8}$ alkyl group, particularly preferably a n-butyl group or a n-pentyl group) substituted by one carboxyl group and one $NH_2$. Particularly preferably, R3 is a $C_{1-8}$ alkyl group substituted by two carboxyl groups. When the $C_{1-8}$ alkyl group described above is substituted by two substituents, the substituents are preferably bound on different carbon atoms of the $C_{1-8}$ alkyl group.

Both of n represent an integer of 0 to 2. Preferably, n is 0 or 2.

Both of X represent a bond or NH. Preferably, X is NH.

Both of Y represent **-C(=O)O- or **-OC(=O)-(wherein ** is bonded to the benzene ring substituted by a guanidino group or a carbamimidoyl group). Preferably, both of Y are **-C(=O)O-.

Qa and Qb are the same or different and each represent $-(CH_2)_mO-*$, $-(CH_2)_mNR5-*$, $-(CH_2)_mNR5C(=O)-*$, -C(=O)NR5-*, -S-*, or $-SO_2-*$ (wherein * is bonded to Z). R5 represents H or a $C_{1-6}$ alkyl group optionally having a substituent(s). m represents an integer of 0 to 6. Preferably, each of Qa and Qb is $-(CH_2)_mO-*$, and m is an integer of 0 to 3. More preferably, each of Qa and Qb is O.

Z represents -[(CR6aR6b)$_q$-A]$_r$-(CR7aR7b)$_s$-.
A represents O, S, $SO_2$, NR8, C(=O), OC(=O), C(=O)O, NR8C(=O), or C(=O)NR8.
R6a, R6b, R7a and R7b are the same or different and each represent H, halogen, a hydroxy group, a $C_{1-6}$ alkyl group optionally having a substituent(s), or a $C_{1-6}$ alkoxy group optionally having a substituent(s). Preferably, all of R6a, R6b, R7a and R7b are H.
R8 represents H or a $C_{1-6}$ alkyl group optionally having a substituent(s). The $C_{1-6}$ alkyl group optionally having a substituent(s) is a $C_{1-6}$ alkyl group optionally having 1 to 5 substituents selected from the substituent group A described above.
q represents an integer of 1 to 6 and is preferably an integer of 1 to 3, more preferably an integer of 2 or 3.
r represents an integer of 0 to 50 and is preferably an integer of 0 to 10, more preferably an integer of 0 to 7. Most preferably, r is an integer of 0 to 3.
s represents an integer of 1 to 6 and is preferably an integer of 1 to 3, more preferably an integer of 2 or 3. Particularly preferably, s is the same integer as that of q.
Further specifically, Z represents -[(CR6aR6b)$_q$-O]$_r$-(CR7aR7b)$_s$-, -[(CR6aR6b)$_q$-S]$_r$-(CR7aR7b)$_s$-, -[(CR6aR6b)$_q$-$SO_2$]$_r$-(CR7aR7b)$_s$-, -[(CR6aR6b)$_q$-NR7]$_r$-(CR7aR7b)$_s$-,-[(CR6aR6b)$_q$-C(=O)]$_r$-(CR7aR7b)$_s$-, -[(CR6aR6b)$_q$-C(=O)O]$_r$-(CR7aR7b)$_s$-, -[(CR6aR6b)$_q$-NR7C(=O)]$_r$-(CR7$_a$R7b)$_s$-, or-[(CR6aR6b)$_q$-C(=O)NR7]$_r$-(CR7aR7b)$_s$-.
Examples of Qa-Z-Qb specifically include the following structures.

[Table 1]

| Qa | Z | Qb |
|---|---|---|
| -(CH2)mO- | -[(CR6aR6b)q-O]r-(CR7aR7b)s- | -O-(CH2)m, |
| -(CH2)mO- | -[(CR6aR6b)q-O]r-(CR7aR7b)s- | -NR5(CH2)m-, |
| -(CH2)mO- | -[(CR6aR6b)q-O]r-(CR7aR7b)s- | -C(=O)NR5(CH2)m-, |
| -(CH2)mO- | -[(CR6aR6b)q-O]r-(CR7aR7b)s- | -NR5C(=O)-, |
| -(CH2)mO- | -[(CR6aR6b)q-O]r-(CR7aR7b)s- | -S-, |
| -(CH2)mO- | -[(CR6aR6b)q-O]r-(CR7aR7b)s- | -SO2- |
| -(CH2)mO- | -[(CR6aR6b)q-S]r-(CR7aR7b)s- | -O-(CH2)m, |
| -(CH2)mO- | -[(CR6aR6b)q-S]r-(CR7aR7b)s- | -NR5(CH2)m-, |
| -(CH2)mO- | -[(CR6aR6b)q-S]r-(CR7aR7b)s- | -C(=O)NR5(CH2)m-, |
| -(CH2)mO- | -[(CR6aR6b)q-S]r-(CR7aR7b)s- | -NR5C(=O)-, |
| -(CH2)mO- | -[(CR6aR6b)q-S]r-(CR7aR7b)s- | -S-, |
| -(CH2)mO- | -[(CR6aR6b)q-S]r-(CR7aR7b)s- | -SO2- |
| -(CH2)mO- | -[(CR6aR6b)q-SO2]r-(CR7aR7b)s- | -O-(CH2)m, |
| -(CH2)mO- | -[(CR6aR6b)q-SO2]r-(CR7aR7b)s- | -NR5(CH2)m-, |
| -(CH2)mO- | -[(CR6aR6b)q-SO2]r-(CR7aR7b)s- | -C(=O)NR5(CH2)m-, |
| -(CH2)mO- | -[(CR6aR6b)q-SO2]r-(CR7aR7b)s- | -NR5C(=O)-, |
| -(CH2)mO- | -[(CR6aR6b)q-SO2]r-(CR7aR7b)s- | -S-, |
| -(CH2)mO- | -[(CR6aR6b)q-SO2]r-(CR7aR7b)s- | -SO2- |
| -(CH2)mO- | -[(CR6aR6b)q-NR8]r-(CR7aR7b)s- | -O-(CH2)m, |
| -(CH2)mO- | -[(CR6aR6b)q-NR8]r-(CR7aR7b)s- | -NR5(CH2)m-, |
| -(CH2)mO- | -[(CR6aR6b)q-NR8]r-(CR7aR7b)s- | -C(=O)NR5(CH2)m-, |
| -(CH2)mO- | -[(CR6aR6b)q-NR8]r-(CR7aR7b)s- | -NR5C(=O)-, |
| -(CH2)mO- | -[(CR6aR6b)q-NR8]r-(CR7aR7b)s- | -S-, |
| -(CH2)mO- | -[(CR6aR6b)q-NR8]r-(CR7aR7b)s- | -SO2- |
| -(CH2)mO- | -[(CR6aR6b)q-C(=O)]r-(CR7aR7b)s- | -O-(CH2)m, |

(continued)

| Qa | Z | Qb |
|---|---|---|
| -(CH2)mO- | -[(CR6aR6b)q-C(=O)]r-(CR7aR7b)s- | -NR5(CH2)m-, |
| -(CH2)mO- | -[(CR6aR6b)q-C(=O)]r-(CR7aR7b)s- | -C(=O)NR5(CH2)m-, |
| -(CH2)mO- | -[(CR6aR6b)q-C(=O)]r-(CR7aR7b)s- | -NR5C(=O)-, |
| -(CH2)mO- | -[(CR6aR6b)q-C(=O)]r-(CR7aR7b)s- | -S-, |
| -(CH2)mO- | -[(CR6aR6b)q-C(=O)]r-(CR7aR7b)s- | -SO2- |
| -(CH2)mO- | -[(CR6aR6b)q-C(=O)O]r-(CR7aR7b)s- | -O-(CH2)m, |
| -(CH2)mO- | -[(CR6aR6b)q-C(=O)O]r-(CR7aR7b)s- | -NR5(CH2)m-, |
| -(CH2)mO- | -[(CR6aR6b)q-C(=O)O]r-(CR7aR7b)s- | -C(=O)NR5(CH2)m-, |
| -(CH2)mO- | -[(CR6aR6b)q-C(=O)O]r-(CR7aR7b)s- | -NR5C(=O)-, |
| -(CH2)mO- | -[(CR6aR6b)q-C(=O)O]r-(CR7aR7b)s- | -S-, |
| -(CH2)mO- | -[(CR6aR6b)q-C(=O)O]r-(CR7aR7b)s- | -SO2- |
| -(CH2)mO- | -[(CR6aR6b)q-NR8C(=O)]r-(CR7aR7b)s- | -O-(CH2)m, |
| -(CH2)mO- | -[(CR6aR6b)q-NR8C(=O)]r-(CR7aR7b)s- | -NR5(CH2)m-, |
| -(CH2)mO- | -[(CR6aR6b)q-NR8C(=O)]r-(CR7aR7b)s- | -C(=O)NR5(CH2)m-, |
| -(CH2)mO- | -[(CR6aR6b)q-NR8C(=O)]r-(CR7aR7b)s- | -NR5C(=O)-, |
| -(CH2)mO- | -[(CR6aR6b)q-NR8C(=O)]r-(CR7aR7b)s- | -S-, |
| -(CH2)mO- | -[(CR6aR6b)q-NR8C(=O)]r-(CR7aR7b)s- | -SO2- |
| -(CH2)mO- | -[(CR6aR6b)q-C(=O)NR8]r-(CR7aR7b)s- | -O-(CH2)m, |
| -(CH2)mO- | -[(CR6aR6b)q-C(=O)NR8]r-(CR7aR7b)s- | -NR5(CH2)m-, |
| -(CH2)mO- | -[(CR6aR6b)q-C(=O)NR8]r-(CR7aR7b)s- | -C(=O)NR5(CH2)m-, |
| -(CH2)mO- | -[(CR6aR6b)q-C(=O)NR8]r-(CR7aR7b)s- | -NR5C(=O)-, |
| -(CH2)mO- | -[(CR6aR6b)q-C(=O)NR8]r-(CR7aR7b)s- | -S-, |
| -(CH2)mO- | -[(CR6aR6b)q-C(=O)NR8]r-(CR7aR7b)s- | -SO2- |
| -(CH2)mNR5- | -[(CR6aR6b)q-O]r-(CR7aR7b)s- | -O-(CH2)m, |
| -(CH2)mNR5- | -[(CR6aR6b)q-O]r-(CR7aR7b)s- | -NR5(CH2)m-, |
| -(CH2)mNR5- | -[(CR6aR6b)q-O]r-(CR7aR7b)s- | -C(=O)NR5(CH2)m-, |
| -(CH2)mNR5- | -[(CR6aR6b)q-O]r-(CR7aR7b)s- | -NR5C(=O)-, |
| -(CH2)mNR5- | -[(CR6aR6b)q-O]r-(CR7aR7b)s- | -S-, |
| -(CH2)mNR5- | -[(CR6aR6b)q-O]r-(CR7aR7b)s- | -SO2- |
| -(CH2)mNR5- | -[(CR6aR6b)q-S]r-(CR7aR7b)s- | -O-(CH2)m, |
| -(CH2)mNR5- | -[(CR6aR6b)q-S]r-(CR7aR7b)s- | -NR5(CH2)m-, |
| -(CH2)mNR5- | -[(CR6aR6b)q-S]r-(CR7aR7b)s- | -C(=O)NR5(CH2)m-, |
| -(CH2)mNR5- | -[(CR6aR6b)q-S]r-(CR7aR7b)s- | -NR5C(=O)-, |
| -(CH2)mNR5- | -[(CR6aR6b)q-S]r-(CR7aR7b)s- | -S-, |
| -(CH2)mNR5- | -[(CR6aR6b)q-S]r-(CR7aR7b)s- | -SO2- |
| -(CH2)mNR5- | -[(CR6aR6b)q-SO2]r-(CR7aR7b)s- | -O-(CH2)m, |
| -(CH2)mNR5- | -[(CR6aR6b)q-SO2]r-(CR7aR7b)s- | -NR5(CH2)m-, |
| -(CH2)mNR5- | -[(CR6aR6b)q-SO2]r-(CR7aR7b)s- | -C(=O)NR5(CH2)m-, |

(continued)

| Qa | Z | Qb |
|---|---|---|
| -(CH2)mNR5- | -[(CR6aR6b)q-SO2]r-(CR7aR7b)s- | -NR5C(=O)-, |
| -(CH2)mNR5- | -[(CR6aR6b)q-SO2]r-(CR7aR7b)s- | -S-, |
| -(CH2)mNR5- | -[(CR6aR6b)q-SO2]r-(CR7aR7b)s- | -SO2- |
| -(CH2)mNR5- | -[(CR6aR6b)q-NR8]r-(CR7aR7b)s- | -O-(CH2)m, |
| -(CH2)mNR5- | -[(CR6aR6b)q-NR8]r-(CR7aR7b)s- | -NR5(CH2)m-, |
| -(CH2)mNR5- | -[(CR6aR6b)q-NR8]r-(CR7aR7b)s- | -C(=O)NR5(CH2)m-, |
| -(CH2)mNR5- | -[(CR6aR6b)q-NR8]r-(CR7aR7b)s- | -NR5C(=O)-, |
| -(CH2)mNR5- | -[(CR6aR6b)q-NR8]r-(CR7aR7b)s- | -S-, |
| -(CH2)mNR5- | -[(CR6aR6b)q-NR8]r-(CR7aR7b)s- | -SO2- |
| -(CH2)mNR5- | -[(CR6aR6b)q-C(=O)]r-(CR7aR7b)s- | -O-(CH2)m, |
| -(CH2)mNR5- | -[(CR6aR6b)q-C(=O)]r-(CR7aR7b)s- | -NR5(CH2)m-, |
| -(CH2)mNR5- | -[(CR6aR6b)q-C(=O)]r-(CR7aR7b)s- | -C(=O)NR5(CH2)m-, |
| -(CH2)mNR5- | -[(CR6aR6b)q-C(=O)]r-(CR7aR7b)s- | -NR5C(=O)-, |
| -(CH2)mNR5- | -[(CR6aR6b)q-C(=O)]r-(CR7aR7b)s- | -S-, |
| -(CH2)mNR5- | -[(CR6aR6b)q-C(=O)]r-(CR7aR7b)s- | -SO2- |
| -(CH2)mNR5- | -[(CR6aR6b)q-C(=O)O]r-(CR7aR7b)s- | -O-(CH2)m, |
| -(CH2)mNR5- | -[(CR6aR6b)q-C(=O)O]r-(CR7aR7b)s- | -NR5(CH2)m-, |
| -(CH2)mNR5- | -[(CR6aR6b)q-C(=O)O]r-(CR7aR7b)s- | -C(=O)NR5(CH2)m-, |
| -(CH2)mNR5- | -[(CR6aR6b)q-C(=O)O]r-(CR7aR7b)s- | -NR5C(=O)-, |
| -(CH2)mNR5- | -[(CR6aR6b)q-C(=O)O]r-(CR7aR7b)s- | -S-, |
| -(CH2)mNR5- | -[(CR6aR6b)q-C(=O)O]r-(CR7aR7b)s- | -SO2- |
| -(CH2)mNR5- | -[(CR6aR6b)q-NR8C(=O)]r-(CR7aR7b)s- | -O-(CH2)m, |
| -(CH2)mNR5- | -[(CR6aR6b)q-NR8C(=O)]r-(CR7aR7b)s- | -NR5(CH2)m-, |
| -(CH2)mNR5- | -[(CR6aR6b)q-NR8C(=O)]r-(CR7aR7b)s- | -C(=O)NR5(CH2)m-, |
| -(CH2)mNR5- | -[(CR6aR6b)q-NR8C(=O)]r-(CR7aR7b)s- | -NR5C(=O)-, |
| -(CH2)mNR5- | -[(CR6aR6b)q-NR8C(=O)]r-(CR7aR7b)s- | -S-, |
| -(CH2)mNR5- | -[(CR6aR6b)q-NR8C(=O)]r-(CR7aR7b)s- | -SO2- |
| -(CH2)mNR5- | -[(CR6aR6b)q-C(=O)NR8]r-(CR7aR7b)s- | -O-(CH2)m, |
| -(CH2)mNR5- | -[(CR6aR6b)q-C(=O)NR8]r-(CR7aR7b)s- | -NR5(CH2)m-, |
| -(CH2)mNR5- | -[(CR6aR6b)q-C(=O)NR8]r-(CR7aR7b)s- | -C(=O)NR5(CH2)m-, |
| -(CH2)mNR5- | -[(CR6aR6b)q-C(=O)NR8]r-(CR7aR7b)s- | -NR5C(=O)-, |
| -(CH2)mNR5- | -[(CR6aR6b)q-C(=O)NR8]r-(CR7aR7b)s- | -S-, |
| -(CH2)mNR5- | -[(CR6aR6b)q-C(=O)NR8]r-(CR7aR7b)s- | -SO2- |
| -(CH2)mNR5C(=O)- | -[(CR6aR6b)q-O]r-(CR7aR7b)s- | -O-(CH2)m, |
| -(CH2)mNR5C(=O)- | -[(CR6aR6b)q-O]r-(CR7aR7b)s- | -NR5(CH2)m-, |
| -(CH2)mNR5C(=O)- | -[(CR6aR6b)q-O]r-(CR7aR7b)s- | -C(=O)NR5(CH2)m-, |
| -(CH2)mNR5C(=O)- | -[(CR6aR6b)q-O]r-(CR7aR7b)s- | -NR5C(=O)-, |
| -(CH2)mNR5C(=O)- | -[(CR6aR6b)q-O]r-(CR7aR7b)s- | -S-, |

(continued)

| Qa | Z | Qb |
|---|---|---|
| -(CH2)mNR5C(=O)- | -[(CR6aR6b)q-O]r-(CR7aR7b)s- | -SO2- |
| -(CH2)mNR5C(=O)- | -[(CR6aR6b)q-S]r-(CR7aR7b)s- | -O-(CH2)m, |
| -(CH2)mNR5C(=O)- | -[(CR6aR6b)q-S]r-(CR7aR7b)s- | -NR5(CH2)m-, |
| -(CH2)mNR5C(=O)- | -[(CR6aR6b)q-S]r-(CR7aR7b)s- | -C(=O)NR5(CH2)m-, |
| -(CH2)mNR5C(=O)- | -[(CR6aR6b)q-S]r-(CR7aR7b)s- | -NR5C(=O)-, |
| -(CH2)mNR5C(=O)- | -[(CR6aR6b)q-S]r-(CR7aR7b)s- | -S-, |
| -(CH2)mNR5C(=O)- | -[(CR6aR6b)q-S]r-(CR7aR7b)s- | -SO2- |
| -(CH2)mNR5C(=O)- | -[(CR6aR6b)q-SO2]r-(CR7aR7b)s- | -O-(CH2)m, |
| -(CH2)mNR5C(=O)- | -[(CR6aR6b)q-SO2]r-(CR7aR7b)s- | -NR5(CH2)m-, |
| -(CH2)mNR5C(=O)- | -[(CR6aR6b)q-SO2]r-(CR7aR7b)s- | -C(=O)NR5(CH2)m-, |
| -(CH2)mNR5C(=O)- | -[(CR6aR6b)q-SO2]r-(CR7aR7b)s- | -NR5C(=O)-, |
| -(CH2)mNR5C(=O)- | -[(CR6aR6b)q-SO2]r-(CR7aR7b)s- | -S-, |
| -(CH2)mNR5C(=O)- | -[(CR6aR6b)q-SO2]r-(CR7aR7b)s- | -SO2- |
| -(CH2)mNR5C(=O)- | -[(CR6aR6b)q-NR8]r-(CR7aR7b)s- | -O-(CH2)m, |
| -(CH2)mNR5C(=O)- | -[(CR6aR6b)q-NR8]r-(CR7aR7b)s- | -NR5(CH2)m-, |
| -(CH2)mNR5C(=O)- | -[(CR6aR6b)q-NR8]r-(CR7aR7b)s- | -C(=O)NR5(CH2)m-, |
| -(CH2)mNR5C(=O)- | -[(CR6aR6b)q-NR8]r-(CR7aR7b)s- | -NR5C(=O)-, |
| -(CH2)mNR5C(=O)- | -[(CR6aR6b)q-NR8]r-(CR7aR7b)s- | -S-, |
| -(CH2)mNR5C(=O)- | -[(CR6aR6b)q-NR8]r-(CR7aR7b)s- | -SO2- |
| -(CH2)mNR5C(=O)- | -[(CR6aR6b)q-C(=O)]r-(CR7aR7b)s- | -O-(CH2)m, |
| -(CH2)mNR5C(=O)- | -[(CR6aR6b)q-C(=O)]r-(CR7aR7b)s- | -NR5(CH2)m-, |
| -(CH2)mNR5C(=O)- | -[(CR6aR6b)q-C(=O)]r-(CR7aR7b)s- | -C(=O)NR5(CH2)m-, |
| -(CH2)mNR5C(=O)- | -[(CR6aR6b)q-C(=O)]r-(CR7aR7b)s- | -NR5C(=O)-, |
| -(CH2)mNR5C(=O)- | -[(CR6aR6b)q-C(=O)]r-(CR7aR7b)s- | -S-, |
| -(CH2)mNR5C(=O)- | -[(CR6aR6b)q-C(=O)]r-(CR7aR7b)s- | -SO2- |
| -(CH2)mNR5C(=O)- | -[(CR6aR6b)q-C(=O)O]r-(CR7aR7b)s- | -O-(CH2)m, |
| -(CH2)mNR5C(=O)- | -[(CR6aR6b)q-C(=O)O]r-(CR7aR7b)s- | -NR5(CH2)m-, |
| -(CH2)mNR5C(=O)- | -[(CR6aR6b)q-C(=O)O]r-(CR7aR7b)s- | -C(=O)NR5(CH2)m-, |
| -(CH2)mNR5C(=O)- | -[(CR6aR6b)q-C(=O)O]r-(CR7aR7b)s- | -NR5C(=O)-, |
| -(CH2)mNR5C(=O)- | -[(CR6aR6b)q-C(=O)O]r-(CR7aR7b)s- | -S-, |
| -(CH2)mNR5C(=O)- | -[(CR6aR6b)q-C(=O)O]r-(CR7aR7b)s- | -SO2- |
| -(CH2)mNR5C(=O)- | -[(CR6aR6b)q-NR8C(=O)]r-(CR7aR7b)s- | -O-(CH2)m, |
| -(CH2)mNR5C(=O)- | -[(CR6aR6b)q-NR8C(=O)]r-(CR7aR7b)s- | -NR5(CH2)m-, |
| -(CH2)mNR5C(=O)- | -[(CR6aR6b)q-NR8C(=O)]r-(CR7aR7b)s- | -C(=O)NR5(CH2)m-, |
| -(CH2)mNR5C(=O)- | -[(CR6aR6b)q-NR8C(=O)]r-(CR7aR7b)s- | -NR5C(=O)-, |
| -(CH2)mNR5C(=O)- | -[(CR6aR6b)q-NR8C(=O)]r-(CR7aR7b)s- | -S-, |
| -(CH2)mNR5C(=O)- | -[(CR6aR6b)q-NR8C(=O)]r-(CR7aR7b)s- | -SO2- |
| -(CH2)mNR5C(=O)- | -[(CR6aR6b)q-C(=O)NR8]r-(CR7aR7b)s- | -O-(CH2)m, |

(continued)

| Qa | Z | Qb |
|---|---|---|
| -(CH2)mNR5C(=O)- | -[(CR6aR6b)q-C(=O)NR8]r-(CR7aR7b)s- | -NR5(CH2)m-, |
| -(CH2)mNR5C(=O)- | -[(CR6aR6b)q-C(=O)NR8]r-(CR7aR7b)s- | -C(=O)NR5(CH2)m-, |
| -(CH2)mNR5C(=O)- | -[(CR6aR6b)q-C(=O)NR8]r-(CR7aR7b)s- | -NR5C(=O)-, |
| -(CH2)mNR5C(=O)- | -[(CR6aR6b)q-C(=O)NR8]r-(CR7aR7b)s- | -S-, |
| -(CH2)mNR5C(=O)- | -[(CR6aR6b)q-C(=O)NR8]r-(CR7aR7b)s- | -SO2- |
| -C(=O)NR5- | -[(CR6aR6b)q-O]r-(CR7aR7b)s- | -O-(CH2)m, |
| -C(=O)NR5- | -[(CR6aR6b)q-O]r-(CR7aR7b)s- | -NR5(CH2)m-, |
| -C(=O)NR5- | -[(CR6aR6b)q-O]r-(CR7aR7b)s- | -C(=O)NR5(CH2)m-, |
| -C(=O)NR5- | -[(CR6aR6b)q-O]r-(CR7aR7b)s- | -NR5C(=O)-, |
| -C(=O)NR5- | -[(CR6aR6b)q-O]r-(CR7aR7b)s- | -S-, |
| -C(=O)NR5- | -[(CR6aR6b)q-O]r-(CR7aR7b)s- | -SO2- |
| -C(=O)NR5- | -[(CR6aR6b)q-S]r-(CR7aR7b)s- | -O-(CH2)m, |
| -C(=O)NR5- | -[(CR6aR6b)q-S]r-(CR7aR7b)s- | -NR5(CH2)m-, |
| -C(=O)NR5- | -[(CR6aR6b)q-S]r-(CR7aR7b)s- | -C(=O)NR5(CH2)m-, |
| -C(=O)NR5- | -[(CR6aR6b)q-S]r-(CR7aR7b)s- | -NR5C(=O)-, |
| -C(=O)NR5- | -[(CR6aR6b)q-S]r-(CR7aR7b)s- | -S-, |
| -C(=O)NR5- | -[(CR6aR6b)q-S]r-(CR7aR7b)s- | -SO2- |
| -C(=O)NR5- | -[(CR6aR6b)q-SO2]r-(CR7aR7b)s- | -O-(CH2)m, |
| -C(=O)NR5- | -[(CR6aR6b)q-SO2]r-(CR7aR7b)s- | -NR5(CH2)m-, |
| -C(=O)NR5- | -[(CR6aR6b)q-SO2]r-(CR7aR7b)s- | -C(=O)NR5(CH2)m-, |
| -C(=O)NR5- | -[(CR6aR6b)q-SO2]r-(CR7aR7b)s- | -NR5C(=O)-, |
| -C(=O)NR5- | -[(CR6aR6b)q-SO2]r-(CR7aR7b)s- | -S-, |
| -C(=O)NR5- | -[(CR6aR6b)q-SO2]r-(CR7aR7b)s- | -SO2- |
| -C(=O)NR5- | -[(CR6aR6b)q-NR8]r-(CR7aR7b)s- | -O-(CH2)m, |
| -C(=O)NR5- | -[(CR6aR6b)q-NR8]r-(CR7aR7b)s- | -NR5(CH2)m-, |
| -C(=O)NR5- | -[(CR6aR6b)q-NR8]r-(CR7aR7b)s- | -C(=O)NR5(CH2)m-, |
| -C(=O)NR5- | -[(CR6aR6b)q-NR8]r-(CR7aR7b)s- | -NR5C(=O)-, |
| -C(=O)NR5- | -[(CR6aR6b)q-NR8]r-(CR7aR7b)s- | -S-, |
| -C(=O)NR5- | -[(CR6aR6b)q-NR8]r-(CR7aR7b)s- | -SO2- |
| -C(=O)NR5- | -[(CR6aR6b)q-C(=O)]r-(CR7aR7b)s- | -O-(CH2)m, |
| -C(=O)NR5- | -[(CR6aR6b)q-C(=O)]r-(CR7aR7b)s- | -NR5(CH2)m-, |
| -C(=O)NR5- | -[(CR6aR6b)q-C(=O)]r-(CR7aR7b)s- | -C(=O)NR5(CH2)m-, |
| -C(=O)NR5- | -[(CR6aR6b)q-C(=O)]r-(CR7aR7b)s- | -NR5C(=O)-, |
| -C(=O)NR5- | -[(CR6aR6b)q-C(=O)]r-(CR7aR7b)s- | -S-, |
| -C(=O)NR5- | -[(CR6aR6b)q-C(=O)]r-(CR7aR7b)s- | -SO2- |
| -C(=O)NR5- | -[(CR6aR6b)q-C(=O)O]r-(CR7aR7b)s- | -O-(CH2)m, |
| -C(=O)NR5- | -[(CR6aR6b)q-C(=O)O]r-(CR7aR7b)s- | -NR5(CH2)m-, |
| -C(=O)NR5- | -[(CR6aR6b)q-C(=O)O]r-(CR7aR7b)s- | -C(=O)NR5(CH2)m-, |

(continued)

| Qa | Z | Qb |
|---|---|---|
| -C(=O)NR5- | -[(CR6aR6b)q-C(=O)O]r-(CR7aR7b)s- | -NR5C(=O)-, |
| -C(=O)NR5- | -[(CR6aR6b)q-C(=O)O]r-(CR7aR7b)s- | -S-, |
| -C(=O)NR5- | -[(CR6aR6b)q-C(=O)O]r-(CR7aR7b)s- | -SO2- |
| -C(=O)NR5- | -[(CR6aR6b)q-NR8C(=O)]r-(CR7aR7b)s- | -O-(CH2)m, |
| -C(=O)NR5- | -[(CR6aR6b)q-NR8C(=O)]r-(CR7aR7b)s- | -NR5(CH2)m-, |
| -C(=O)NR5- | -[(CR6aR6b)q-NR8C(=O)]r-(CR7aR7b)s- | -C(=O)NR5(CH2)m-, |
| -C(=O)NR5- | -[(CR6aR6b)q-NR8C(=O)]r-(CR7aR7b)s- | -NR5C(=O)-, |
| -C(=O)NR5- | -[(CR6aR6b)q-NR8C(=O)]r-(CR7aR7b)s- | -S-, |
| -C(=O)NR5- | -[(CR6aR6b)q-NR8C(=O)]r-(CR7aR7b)s- | -SO2- |
| -C(=O)NR5- | -[(CR6aR6b)q-C(=O)NR8]r-(CR7aR7b)s- | -O-(CH2)m, |
| -C(=O)NR5- | -[(CR6aR6b)q-C(=O)NR8]r-(CR7aR7b)s- | -NR5(CH2)m-, |
| -C(=O)NR5- | -[(CR6aR6b)q-C(=O)NR8]r-(CR7aR7b)s- | -C(=O)NR5(CH2)m-, |
| -C(=O)NR5- | -[(CR6aR6b)q-C(=O)NR8]r-(CR7aR7b)s- | -NR5C(=O)-, |
| -C(=O)NR5- | -[(CR6aR6b)q-C(=O)NR8]r-(CR7aR7b)s- | -S-, |
| -C(=O)NR5- | -[(CR6aR6b)q-C(=O)NR8]r-(CR7aR7b)s- | -SO2- |
| -S- | -[(CR6aR6b)q-O]r-(CR7aR7b)s- | -O-(CH2)m, |
| -S- | -[(CR6aR6b)q-O]r-(CR7aR7b)s- | -NR5(CH2)m-, |
| -S- | -[(CR6aR6b)q-O]r-(CR7aR7b)s- | -C(=O)NR5(CH2)m-, |
| -S- | -[(CR6aR6b)q-O]r-(CR7aR7b)s- | -NR5C(=O)-, |
| -S- | -[(CR6aR6b)q-O]r-(CR7aR7b)s- | -S-, |
| -S- | -[(CR6aR6b)q-O]r-(CR7aR7b)s- | -SO2- |
| -S- | -[(CR6aR6b)q-S]r-(CR7aR7b)s- | -O-(CH2)m, |
| -S- | -[(CR6aR6b)q-S]r-(CR7aR7b)s- | -NR5(CH2)m-, |
| -S- | -[(CR6aR6b)q-S]r-(CR7aR7b)s- | -C(=O)NR5(CH2)m-, |
| -S- | -[(CR6aR6b)q-S]r-(CR7aR7b)s- | -NR5C(=O)-, |
| -S- | -[(CR6aR6b)q-S]r-(CR7aR7b)s- | -S-, |
| -S- | -[(CR6aR6b)q-S]r-(CR7aR7b)s- | -SO2- |
| -S- | -[(CR6aR6b)q-SO2]r-(CR7aR7b)s- | -O-(CH2)m, |
| -S- | -[(CR6aR6b)q-SO2]r-(CR7aR7b)s- | -NR5(CH2)m-, |
| -S- | -[(CR6aR6b)q-SO2]r-(CR7aR7b)s- | -C(=O)NR5(CH2)m-, |
| -S- | -[(CR6aR6b)q-SO2]r-(CR7aR7b)s- | -NR5C(=O)-, |
| -S- | -[(CR6aR6b)q-SO2]r-(CR7aR7b)s- | -S-, |
| -S- | -[(CR6aR6b)q-SO2]r-(CR7aR7b)s- | -SO2- |
| -S- | -[(CR6aR6b)q-NR8]r-(CR7aR7b)s- | -O-(CH2)m, |
| -S- | -[(CR6aR6b)q-NR8]r-(CR7aR7b)s- | -NR5(CH2)m-, |
| -S- | -[(CR6aR6b)q-NR8]r-(CR7aR7b)s- | -C(=O)NR5(CH2)m-, |
| -S- | -[(CR6aR6b)q-NR8]r-(CR7aR7b)s- | -NR5C(=O)-, |
| -S- | -[(CR6aR6b)q-NR8]r-(CR7aR7b)s- | -S-, |

(continued)

| Qa | Z | Qb |
|---|---|---|
| -S- | -[(CR6aR6b)q-NR8]r-(CR7aR7b)s- | -SO2- |
| -S- | -[(CR6aR6b)q-C(=O)]r-(CR7aR7b)s- | -O-(CH2)m, |
| -S- | -[(CR6aR6b)q-C(=O)]r-(CR7aR7b)s- | -NR5(CH2)m-, |
| -S- | -[(CR6aR6b)q-C(=O)]r-(CR7aR7b)s- | -C(=O)NR5(CH2)m-, |
| -S- | -[(CR6aR6b)q-C(=O)]r-(CR7aR7b)s- | -NR5C(=O)-, |
| -S- | -[(CR6aR6b)q-C(=O)]r-(CR7aR7b)s- | -S-, |
| -S- | -[(CR6aR6b)q-C(=O)]r-(CR7aR7b)s- | -SO2- |
| -S- | -[(CR6aR6b)q-C(=O)O]r-(CR7aR7b)s- | -O-(CH2)m, |
| -S- | -[(CR6aR6b)q-C(=O)O]r-(CR7aR7b)s- | -NR5(CH2)m-, |
| -S- | -[(CR6aR6b)q-C(=O)O]r-(CR7aR7b)s- | -C(=O)NR5(CH2)m-, |
| -S- | -[(CR6aR6b)q-C(=O)O]r-(CR7aR7b)s- | -NR5C(=O)-, |
| -S- | -[(CR6aR6b)q-C(=O)O]r-(CR7aR7b)s- | -S-, |
| -S- | -[(CR6aR6b)q-C(=O)O]r-(CR7aR7b)s- | -SO2- |
| -S- | -[(CR6aR6b)q-NR8C(=O)]r-(CR7aR7b)s- | -O-(CH2)m, |
| -S- | -[(CR6aR6b)q-NR8C(=O)]r-(CR7aR7b)s- | -NR5(CH2)m-, |
| -S- | -[(CR6aR6b)q-NR8C(=O)]r-(CR7aR7b)s- | -C(=O)NR5(CH2)m-, |
| -S- | -[(CR6aR6b)q-NR8C(=O)]r-(CR7aR7b)s- | -NR5C(=O)-, |
| -S- | -[(CR6aR6b)q-NR8C(=O)]r-(CR7aR7b)s- | -S-, |
| -S- | -[(CR6aR6b)q-NR8C(=O)]r-(CR7aR7b)s- | -SO2- |
| -S- | -[(CR6aR6b)q-C(=O)NR8]r-(CR7aR7b)s- | -O-(CH2)m, |
| -S- | -[(CR6aR6b)q-C(=O)NR8]r-(CR7aR7b)s- | -NR5(CH2)m-, |
| -S- | -[(CR6aR6b)q-C(=O)NR8]r-(CR7aR7b)s- | -C(=O)NR5(CH2)m-, |
| -S- | -[(CR6aR6b)q-C(=O)NR8]r-(CR7aR7b)s- | -NR5C(=O)-, |
| -S- | -[(CR6aR6b)q-C(=O)NR8]r-(CR7aR7b)s- | -S-, |
| -S- | -[(CR6aR6b)q-C(=O)NR8]r-(CR7aR7b)s- | -SO2- |
| -SO2- | -[(CR6aR6b)q-O]r-(CR7aR7b)s- | -O-(CH2)m, |
| -SO2- | -[(CR6aR6b)q-O]r-(CR7aR7b)s- | -NR5(CH2)m-, |
| -SO2- | -[(CR6aR6b)q-O]r-(CR7aR7b)s- | -C(=O)NR5(CH2)m-, |
| -SO2- | -[(CR6aR6b)q-O]r-(CR7aR7b)s- | -NR5C(=O)-, |
| -SO2- | -[(CR6aR6b)q-O]r-(CR7aR7b)s- | -S-, |
| -SO2- | -[(CR6aR6b)q-O]r-(CR7aR7b)s- | -SO2- |
| -SO2- | -[(CR6aR6b)q-S]r-(CR7aR7b)s- | -O-(CH2)m, |
| -SO2- | -[(CR6aR6b)q-S]r-(CR7aR7b)s- | -NR5(CH2)m-, |
| -SO2- | -[(CR6aR6b)q-S]r-(CR7aR7b)s- | -C(=O)NR5(CH2)m-, |
| -SO2- | -[(CR6aR6b)q-S]r-(CR7aR7b)s- | -NR5C(=O)-, |
| -SO2- | -[(CR6aR6b)q-S]r-(CR7aR7b)s- | -S-, |
| -SO2- | -[(CR6aR6b)q-S]r-(CR7aR7b)s- | -SO2- |
| -SO2- | -[(CR6aR6b)q-SO2]r-(CR7aR7b)s- | -O-(CH2)m, |

(continued)

| Qa | Z | Qb |
|---|---|---|
| -SO2- | -[(CR6aR6b)q-SO2]r-(CR7aR7b)s- | -NR5(CH2)m-, |
| -SO2- | -[(CR6aR6b)q-SO2]r-(CR7aR7b)s- | -C(=O)NR5(CH2)m-, |
| -SO2- | -[(CR6aR6b)q-SO2]r-(CR7aR7b)s- | -NR5C(=O)-, |
| -SO2- | -[(CR6aR6b)q-SO2]r-(CR7aR7b)s- | -S-, |
| -SO2- | -[(CR6aR6b)q-SO2]r-(CR7aR7b)s- | -SO2- |
| -SO2- | -[(CR6aR6b)q-NR8]r-(CR7aR7b)s- | -O-(CH2)m, |
| -SO2- | -[(CR6aR6b)q-NR8]r-(CR7aR7b)s- | -NR5(CH2)m-, |
| -SO2- | -[(CR6aR6b)q-NR8]r-(CR7aR7b)s- | -C(=O)NR5(CH2)m-, |
| -SO2- | -[(CR6aR6b)q-NR8]r-(CR7aR7b)s- | -NR5C(=O)-, |
| -SO2- | -[(CR6aR6b)q-NR8]r-(CR7aR7b)s- | -S-, |
| -SO2- | -[(CR6aR6b)q-NR8]r-(CR7aR7b)s- | -SO2- |
| -SO2- | -[(CR6aR6b)q-C(=O)]r-(CR7aR7b)s- | -O-(CH2)m, |
| -SO2- | -[(CR6aR6b)q-C(=O)]r-(CR7aR7b)s- | -NR5(CH2)m-, |
| -SO2- | -[(CR6aR6b)q-C(=O)]r-(CR7aR7b)s- | -C(=O)NR5(CH2)m-, |
| -SO2- | -[(CR6aR6b)q-C(=O)]r-(CR7aR7b)s- | -NR5C(=O)-, |
| -SO2- | -[(CR6aR6b)q-C(=O)]r-(CR7aR7b)s- | -S-, |
| -SO2- | -[(CR6aR6b)q-C(=O)]r-(CR7aR7b)s- | -SO2- |
| -SO2- | -[(CR6aR6b)q-C(=O)O]r-(CR7aR7b)s- | -O-(CH2)m, |
| -SO2- | -[(CR6aR6b)q-C(=O)O]r-(CR7aR7b)s- | -NR5(CH2)m-, |
| -SO2- | -[(CR6aR6b)q-C(=O)O]r-(CR7aR7b)s- | -C(=O)NR5(CH2)m-, |
| -SO2- | -[(CR6aR6b)q-C(=O)O]r-(CR7aR7b)s- | -NR5C(=O)-, |
| -SO2- | -[(CR6aR6b)q-C(=O)O]r-(CR7aR7b)s- | -S-, |
| -SO2- | -[(CR6aR6b)q-C(=O)O]r-(CR7aR7b)s- | -SO2- |
| -SO2- | -[(CR6aR6b)q-NR8C(=O)]r-(CR7aR7b)s- | -O-(CH2)m, |
| -SO2- | -[(CR6aR6b)q-NR8C(=O)]r-(CR7aR7b)s- | -NR5(CH2)m-, |
| -SO2- | -[(CR6aR6b)q-NR8C(=O)]r-(CR7aR7b)s- | -C(=O)NR5(CH2)m-, |
| -SO2- | -[(CR6aR6b)q-NR8C(=O)]r-(CR7aR7b)s- | -NR5C(=O)-, |
| -SO2- | -[(CR6aR6b)q-NR8C(=O)]r-(CR7aR7b)s- | -S-, |
| -SO2- | -[(CR6aR6b)q-NR8C(=O)]r-(CR7aR7b)s- | -SO2- |
| -SO2- | -[(CR6aR6b)q-C(=O)NR8]r-(CR7aR7b)s- | -O-(CH2)m, |
| -SO2- | -[(CR6aR6b)q-C(=O)NR8]r-(CR7aR7b)s- | -NR5(CH2)m-, |
| -SO2- | -[(CR6aR6b)q-C(=O)NR8]r-(CR7aR7b)s- | -C(=O)NR5(CH2)m-, |
| -SO2- | -[(CR6aR6b)q-C(=O)NR8]r-(CR7aR7b)s- | -NR5C(=O)-, |
| -SO2- | -[(CR6aR6b)q-C(=O)NR8]r-(CR7aR7b)s- | -S-, |
| -SO2- | -[(CR6aR6b)q-C(=O)NR8]r-(CR7aR7b)s- | -SO2- |

Preferably, each of Qa and Qb is $-(CH_2)_mO-*$, m is an integer of 0 to 3, Z is $-[(CR6aR6b)_q-O]_r-(CR7aR7b)_s-$, each of q and s is an integer of 1 to 6, and r is an integer of 0 to 10. Further preferably, each of q and s is an integer of 1 to 3. More preferably, m is 0, each of q and s is an integer of 2 or 3, and r is an integer of 0 to 3.

Particularly preferably, each of Qa and Qb is O, Z is $-[(CH_2)_2-O]r-(CH_2)_2-$, and r is an integer of 0 to 3.

Examples of the salt of the compound represented by the formula (I) include a metal salt, ammonium salt, a salt with an organic base, a salt with an inorganic acid, a salt with an organic acid, and a salt with a basic or acidic amino acid.

Preferred examples of the metal salt include: alkali metal salts such as sodium salt and potassium salt; alkaline earth metal salts such as calcium salt, magnesium salt, and barium salt; and aluminum salt.

Preferred examples of the salt with an organic base include a salt with trimethylamine, triethylamine, pyridine, picoline, 2,6-lutidine, ethanolamine, diethanolamine, triethanolamine, cyclohexylamine, dicyclohexylamine, or N,N-dibenzylethylenediamine.

Preferred examples of the salt with an inorganic acid include a salt with hydrochloric acid, hydrobromic acid, nitric acid, sulfuric acid, or phosphoric acid.

Preferred examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, or p-toluenesulfonic acid.

Preferred examples of the salt with a basic amino acid include a salt with arginine, lysine, or ornithine. Preferred examples of the salt with an acidic amino acid include a salt with aspartic acid or glutamic acid.

Among these salts, a pharmaceutically acceptable salt is preferred.

A method for producing the compound of the present invention will be described below.

A starting material or a reagent used in each step in the production method given below and the obtained compound may each form a salt. Examples of such a salt include the same as the aforementioned salt of the compound of the present invention.

When the compound obtained in each step is a free compound, this compound can be converted to a salt of interest by a method known per se in the art. On the contrary, when the compound obtained in each step is a salt, this salt can be converted to a free form or another type of salt of interest by a method known per se in the art.

The compound obtained in each step may be used in the next reaction in the form of its reaction solution or after being obtained as a crude product. Alternatively, the compound obtained in each step can be isolated and/or purified from the reaction mixture by a separation approach such as concentration, crystallization, recrystallization, distillation, solvent extraction, fractionation, or chromatography according to a routine method.

If a starting material or a reagent compound for each step is commercially available, the commercially available product can be used directly.

In the reaction of each step, the reaction time may differ depending on the reagent or the solvent used and is usually 1 minute to 48 hours, preferably 10 minutes to 8 hours, unless otherwise specified.

In the reaction of each step, the reaction temperature may differ depending on the reagent or the solvent used and is usually -78°C to 300°C, preferably - 78°C to 150°C, unless otherwise specified.

In the reaction of each step, the pressure may differ depending on the reagent or the solvent used and is usually 1 atm to 20 atm, preferably 1 atm to 3 atm, unless otherwise specified.

In the reaction of each step, a microwave synthesis apparatus, for example, Initiator manufactured by Biotage Japan Ltd., may be used. The reaction temperature may differ depending on the reagent or the solvent used and is usually room temperature to 300°C, preferably 50°C to 250°C, unless otherwise specified. The reaction time may differ depending on the reagent or the solvent used and is usually 1 minute to 48 hours, preferably 1 minute to 8 hours, unless otherwise specified.

In the reaction of each step, the reagent is used at 0.5 equivalents to 20 equivalents, preferably 0.8 equivalents to 5 equivalents, with respect to the substrate, unless otherwise specified. In the case of using the reagent as a catalyst, the reagent is used at 0.001 equivalents to 1 equivalent, preferably 0.01 equivalents to 0.2 equivalents, with respect to the substrate. When the reagent also serves as a reaction solvent, the reagent is used in the amount of the solvent.

In the reaction of each step, this reaction is carried out without a solvent or by dissolution or suspension in an appropriate solvent, unless otherwise specified. Specific examples of the solvent include solvents described in Examples and the following:

alcohols: methanol, ethanol, tert-butyl alcohol, 2-methoxyethanol, and the like;
ethers: diethyl ether, diphenyl ether, tetrahydrofuran, 1,2-dimethoxyethane, cyclopentyl methyl ether and the like;
aromatic hydrocarbons: chlorobenzene, toluene, xylene, and the like;
saturated hydrocarbons: cyclohexane, hexane, and the like;
amides: N,N-dimethylformamide,N-methyl-2-pyrrolidone, and the like;
halogenated hydrocarbons: dichloromethane, carbon tetrachloride, and the like;
nitriles: acetonitrile and the like;
sulfoxides: dimethyl sulfoxide and the like;
aromatic organic bases: pyridine and the like;

acid anhydrides: acetic anhydride and the like;
organic acids: formic acid, acetic acid, trifluoroacetic acid, and the like;
inorganic acids: hydrochloric acid, sulfuric acid, and the like;
esters: ethyl acetate and the like;
ketones: acetone, methyl ethyl ketone, and the like; and water.

Two or more of these solvents may be used as a mixture at an appropriate ratio.

In the case of using a base in the reaction of each step, for example, the following base or a base described in Examples is used:

inorganic bases: sodium hydroxide, magnesium hydroxide, sodium carbonate, calcium carbonate, sodium bicarbonate, and the like;
organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine, and the like;
metal alkoxides: sodium ethoxide, potassium tert-butoxide, and the like;
alkali metal hydrides: sodium hydride, and the like;
metal amides: sodium amide, lithium diisopropylamide, lithium hexamethyldisilazide, and the like; and
organic lithiums: n-butyllithium and the like.

In the case of using an acid or an acidic catalyst in the reaction of each step, for example, the following acid or acidic catalyst or an acid or an acidic catalyst described in Examples is used:

inorganic acids: hydrochloric acid, sulfuric acid, nitric acid, hydrobromic acid, phosphoric acid, and the like;
organic acids: acetic acid, trifluoroacetic acid, citric acid, p-toluenesulfonic acid, 10-camphorsulfonic acid, and the like; and
Lewis acids: boron trifluoride-diethyl ether complex, zinc iodide, anhydrous aluminum chloride, anhydrous zinc chloride, anhydrous iron chloride, and the like.

The reaction of each step is carried out according to a method known per se in the art, for example, a method described in The Fifth Series of Experimental Chemistry, Vol. 13 to Vol. 19 (edited by The Chemical Society of Japan); Shin Jikken Kagaku Koza (New Experimental Chemistry in English), Vol. 14 to Vol. 15 (edited by The Chemical Society of Japan); Reactions and Syntheses: In the Organic Chemistry Laboratory, Revised, 2nd Ed. (L. F. Tietze, Th. Eicher, Nankodo Co., Ltd.); Organic Name Reactions; The Reaction Mechanism and Essence, Revised (Hideo Tougo, Kodansha Ltd.); Organic Syntheses Collective Volume I to VII (John Wiley & Sons, Inc.); Modern Organic Synthesis in the Laboratory: A Collection of Standard Experimental Procedures (Jie Jack Li, Oxford University Press); Comprehensive Heterocyclic Chemistry III, Vol. 1 to Vol. 14 (Elsevier Japan KK); Strategic Applications of Named Reactions in Organic Synthesis (translated by Kiyoshi Tomioka, published by Kagaku-Dojin Publishing Company, Inc.); Comprehensive Organic Transformations (VCH Publishers, Inc.) (1989), etc., or a method described in Examples, unless otherwise specified.

In each step, the protection or deprotection reaction of a functional group is carried out according to a method known per se in the art, for example, a method described in "Protective Groups in Organic Synthesis, 4th Ed." (Theodora W. Greene, Peter G. M. Wuts), Wiley-Interscience (2007); "Protecting Groups, 3rd Ed." (P.J. Kocienski), Thieme Medical Publishers (2004), etc., or a method described in Examples.

Examples of a protective group for a hydroxy group such as a hydroxy group or a phenolic hydroxy group in an alcohol or the like include: ether-type protective groups such as methoxy methyl ether, benzyl ether, tert-butyl dimethyl silyl ether, and tetrahydropyranyl ether; carboxylic acid ester-type protective groups such as acetic acid ester; sulfonic acid ester-type protective groups such as methanesulfonic acid ester; and carbonic acid ester-type protective groups such as tert-butyl carbonate.

Examples of a protective group for a carbonyl group in an aldehyde include: acetal-type protective groups such as dimethylacetal; and cyclic acetal-type protective groups such as 1,3-dioxane.

Examples of a protective group for a carbonyl group in a ketone include: ketal-type protective groups such as dimethylketal; cyclic ketal-type protective groups such as 1,3-dioxane; oxime-type protective groups such as O-methyloxime; and hydrazone-type protective groups such as N,N-dimethylhydrazone.

Examples of a protective group for a carboxyl group include: ester-type protective groups such as methyl ester, ethyl ester, tert-butyl ester, and benzyl ester; and amide-type protective groups such as N,N-dimethylamide.

Examples of a protective group for a thiol include: ether-type protective groups such as benzyl thioether; and ester-type protective groups such as thioacetic acid ester, thiocarbonate, and thiocarbamate.

Examples of a protective group for an amine such as an amino group or an aromatic heterocyclic ring such as imidazole, pyrrole, or indole include: carbamate-type protective groups such as benzyl carbamate and tert-butyl carbamate; amide-type protective groups such as acetamide; alkylamine-type protective groups such as N-triphenylmethylamine; and sulfonamide-type protective groups such as methanesulfonamide.

These protective groups can be removed by use of a method known per se in the art, for example, a method using an acid, a base, ultraviolet light, hydrazine, phenylhydrazine, sodium N-methyldithiocarbamate, tetrabutylammonium fluoride, palladium acetate, or trialkylsilyl halide (e.g., trimethylsilyl iodide and trimethylsilyl bromide), or a reduction method.

In the case of carrying out reduction reaction in each step, examples of the reducing agent used include: metal hydrides such as lithium aluminum hydride, sodium triacetoxyborohydride, sodium cyanoborohydride, diisobutyl aluminum hydride (DIBAL-H), sodium borohydride, and tetramethylammonium triacetoxyborohydride, lithium tri-sec-butyl borohydride; boranes such as a borane-tetrahydrofuran complex; Raney nickel; Raney cobalt; hydrogen; and formic acid; triethylsilane. In the case of reducing a carbon-carbon double bond or triple bond, a method using a catalyst such as palladium-carbon or a Lindlar's catalyst can be used.

In the case of carrying out oxidation reaction in each step, examples of the oxidizing agent used include: peracids such as m-chloroperbenzoic acid (mCPBA), hydrogen peroxide, and tert-butyl hydroperoxide; perchlorates such as tetrabutylammonium perchlorate; chlorates such as sodium chlorate; chlorites such as sodium chlorite; periodates such as sodium periodate; high-valent iodine reagents such as iodosylbenzene; reagents having manganese, such as manganese dioxide and potassium permanganate; leads such as lead tetraacetate; reagents having chromium, such as pyridinium chlorochromate (PCC), pyridinium dichromate (PDC), and Jones reagents; halogen compounds such as N-bromosuccinimide (NBS); oxygen; ozone; a sulfur trioxidepyridine complex; osmium tetroxide; selenium dioxide; and 2,3-dichloro-5,6-dicyano-1,4-benzoquinone (DDQ).

In the case of carrying out radical cyclization reaction in each step, examples of the radical initiator used include: azo compounds such as azobisisobutyronitrile (AIBN); water-soluble radical initiators such as 4,4'-azobis-4-cyanopentanoic acid (ACPA); triethylboron in the presence of air or oxygen; and benzoyl peroxide. Examples of the radical reaction agent used include tributylstannane, tristrimethylsilylsilane, 1,1,2,2-tetraphenyldisilane, diphenylsilane, and samarium iodide.

In the case of carrying out Wittig reaction in each step, examples of the Wittig reagent used include alkylidenephosphoranes. The alkylidenephosphoranes can be prepared by a method known per se in the art, for example, the reaction between a phosphonium salt and a strong base.

In the case of carrying out Horner-Emmons reaction in each step, examples of the reagent used include: phosphonoacetic acid esters such as methyl dimethylphosphonoacetate and ethyl diethylphosphonoacetate; and bases such as alkali metal hydrides and organic lithiums.

In the case of carrying out Friedel-Crafts reaction in each step, examples of the reagent used include a combination of a Lewis acid and an acid chloride and a combination of a Lewis acid and an alkylating agent (e.g., alkyl halides, alcohols, and olefins). Alternatively, an organic acid or an inorganic acid may be used instead of the Lewis acid, and an acid anhydride such as acetic anhydride may be used instead of the acid chloride.

In the case of carrying out aromatic nucleophilic substitution reaction in each step, a nucleophile (e.g., amines and imidazole) and a base (e.g., organic bases) are used as reagents.

In the case of carrying out nucleophilic addition reaction using a carbanion, nucleophilic 1,4-addition reaction (Michael addition reaction) using a carbanion, or nucleophilic substitution reaction using a carbanion in each step, examples of the base used for generating the carbanion include organic lithiums, metal alkoxides, inorganic bases, and organic bases.

In the case of carrying out Grignard reaction in each step, examples of the Grignard reagent include: aryl magnesium halides such as phenyl magnesium bromide; and alkyl magnesium halides such as methyl magnesium bromide. The Grignard reagent can be prepared by a method known per se in the art, for example, the reaction between alkyl halide or aryl halide and metal magnesium with ether or tetrahydrofuran as a solvent.

In the case of carrying out Knoevenagel condensation reaction in each step, an active methylene compound flanked by two electron-attracting groups (e.g., malonic acid, diethyl malonate, and malononitrile) and a base (e.g., organic bases, metal alkoxides, and inorganic bases) are used as reagents.

In the case of carrying out Vilsmeier-Haack reaction in each step, phosphoryl chloride and an amide derivative (e.g., N,N-dimethylformamide) are used as reagents.

In the case of carrying out azidation reaction of alcohols, alkyl halides, or sulfonic acid esters in each step, examples of the azidating agent used include diphenylphosphorylazide (DPPA), trimethylsilylazide, and sodium azide. In the case of azidating, for example, alcohols, a method using diphenylphosphorylazide and 1,8-diazabicyclo[5.4.0]undec-7-ene (DBU), a method using trimethylsilylazide and a Lewis acid, or the like can be used.

In the case of carrying out reductive amination reaction in each step, examples of the reducing agent used include sodium triacetoxyborohydride, sodium cyanoborohydride, hydrogen, and formic acid. When the substrate is an amine compound, examples of the carbonyl compound used include p-formaldehyde as well as aldehydes such as acetaldehyde, and ketones such as cyclohexanone. When the substrate is a carbonyl compound, examples of the

amines used include: primary amine such as ammonia and methylamine; and secondary amine such as dimethylamine.

In the case of carrying out Mitsunobu reaction in each step, azodicarboxylic acid esters (e.g., diethyl azodicarboxylate (DEAD) and diisopropyl azodicarboxylate (DIAD), bis(2-methoxyethyl) azodicarboxylate) and triphenylphosphine, cyanomethylenetributylphosphorane (CMBP) are used as reagents.

In the case of carrying out esterification reaction, amidation reaction, or ureation reaction in each step, examples of the reagent used include: an acyl halide form of acid chloride, acid bromide, and the like; and activated carboxylic acids such as an acid anhydride, an active ester form, and a sulfuric acid ester form. Examples of the activator for carboxylic acid include: carbodiimide condensing agents such as 1-ethyl-3-(3-dimethylaminopropyl)carbodiimide hydrochloride (WSCD); triazine condensing agents such as 4-(4,6-dimethoxy-1,3,5-triazin-2-yl)-4-methylmorpholinium chloride-n-hydrate (DMT-MM); carbonic acid ester condensing agents such as 1,1-carbonyldiimidazole (CDI); diphenylphosphorylazide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformate such as ethyl chloroformate; O-(7-azabenzotriazol-1-yl)-N,N,N',N'-tetramethyluronium hexafluorophosphate (HATU); sulfuric acid; propylphosphonic anhydride (T3P) and combinations thereof. In the case of using a carbodiimide condensing agent, an additive such as 1-hydroxybenzotriazole (HOBt), N-hydroxysuccinimide (HOSu), or dimethylaminopyridine (DMAP), ethyl cyano(hydroxyimino)acetate (Oxymapure) may be further added for the reaction.

In the case of carrying out coupling reaction in each step, examples of the metal catalyst used include: palladium compounds such as palladium(II) acetate, tetrakis(triphenylphosphine)palladium(0), dichlorobis(triphenylphosphine)palladium(II), dichlorobis(triethylphosphine)palladium(II), tris(dibenzylideneacetone)dipalladium(0), and 1,1'-bis(diphenylphosphino)ferrocene palladium(II) chloride; nickel compounds such as tetrakis(triphenylphosphine)nickel(0); rhodium compounds such as tris(triphenylphosphine)rhodium(III) chloride; cobalt compounds; copper compounds such as copper oxide and copper(I) iodide; and platinum compounds. A base may be further added for the reaction. Examples of such a base include inorganic bases.

In the case of carrying out thiocarbonylation reaction in each step, diphosphorus pentasulfide is typically used as a thiocarbonylating agent. A reagent having a 1,3,2,4-dithiadiphosphetane-2,4-disulfide structure such as 2,4-bis(4-methoxyphenyl)-1,3,2,4-dithiadiphosphetane-2,4-disulfide (Lawesson's reagent) may be used instead of diphosphorus pentasulfide.

In the case of carrying out Wohl-Ziegler reaction in each step, examples of the halogenating agent used include N-iodosuccinimide, N-bromosuccinimide (NBS), N-chlorosuccinimide (NCS), bromine, and sulfuryl chloride. The reaction can be accelerated by the further addition of a radical initiator such as heat, light, benzoyl peroxide, or azobisisobutyronitrile for the reaction.

In the case of carrying out halogenation reaction of a hydroxy group in each step, examples of the halogenating agent used include a hydrohalic acid and an acid halide of an inorganic acid, specifically, hydrochloric acid, thionyl chloride, and phosphorus oxychloride for chlorination, and 48% hydrobromic acid for bromination. Also, a method for obtaining an alkyl halide form from an alcohol by the action of triphenylphosphine and carbon tetrachloride or carbon tetrabromide or the like may be used. Alternatively, a method for synthesizing an alkyl halide form through 2-stage reactions involving the conversion of an alcohol to sulfonic acid ester and the subsequent reaction with lithium bromide, lithium chloride, or sodium iodide may be used.

In the case of carrying out Arbuzov reaction in each step, examples of the reagent used include: alkyl halides such as ethyl bromoacetate; and phosphites such as triethyl phosphite and tri(isopropyl) phosphite.

In the case of carrying out sulfonic acid-esterification reaction in each step, examples of the sulfonylating agent used include methanesulfonyl chloride, p-toluenesulfonyl chloride, methanesulfonic anhydride, and p-toluenesulfonic anhydride.

In the case of carrying out hydrolysis reaction in each step, an acid or a base is used as a reagent. In the case of carrying out acid hydrolysis reaction of tert-butyl ester, formic acid, triethylsilane, or the like may be added in order to reductively trap a byproduct tert-butyl cation.

In the case of carrying out dehydration reaction in each step, examples of the dehydrating agent used include sulfuric acid, diphosphorus pentoxide, phosphorus oxychloride, N,N'-dicyclohexylcarbodiimide, alumina, and polyphosphoric acid.

<Production Method 1>

Among the compounds (I), compound (Ia) can be produced by a method shown below from compound (1).

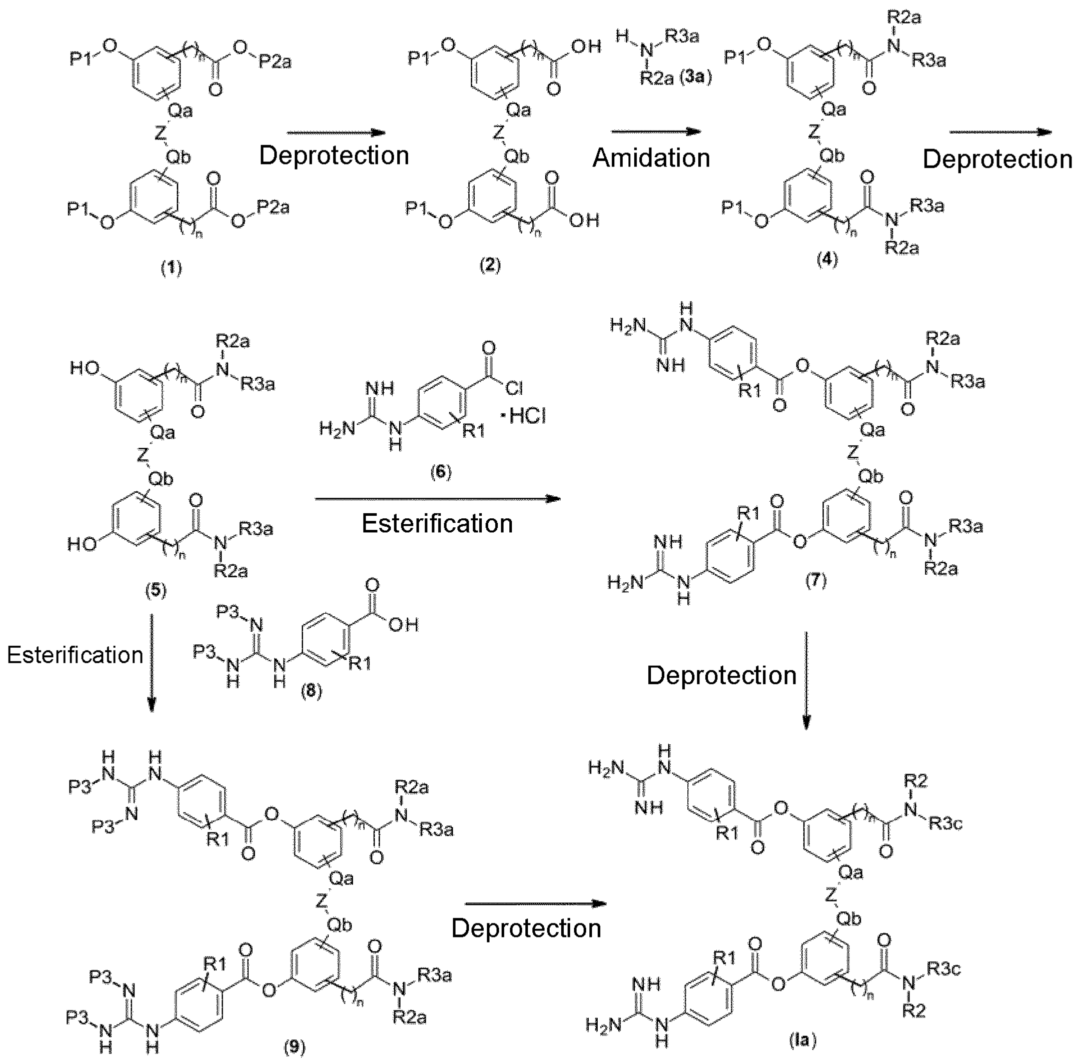


In the formula, R2a represents H or a $C_{1-6}$ alkyl group optionally substituted by one C(=O)OP2a, R3a represents a linear or branched $C_{1-8}$ alkyl group optionally substituted by one or two C(=O)OP2a, R3c represents a linear or branched $C_{1-8}$ alkyl group optionally substituted by one or two carboxyl groups, P1 represents a protective group for a hydroxy group, P2a represents a protective group for a carboxyl group, P3 represents a protective group for an amine, and the other symbols are as defined above.

Compounds (3a), (6) and (8) can each be produced by a method known per se in the art or in accordance with the method.

<Production Method 2>

Among the compounds (I), compound (Ib) can be produced by a method shown below from compound (1).

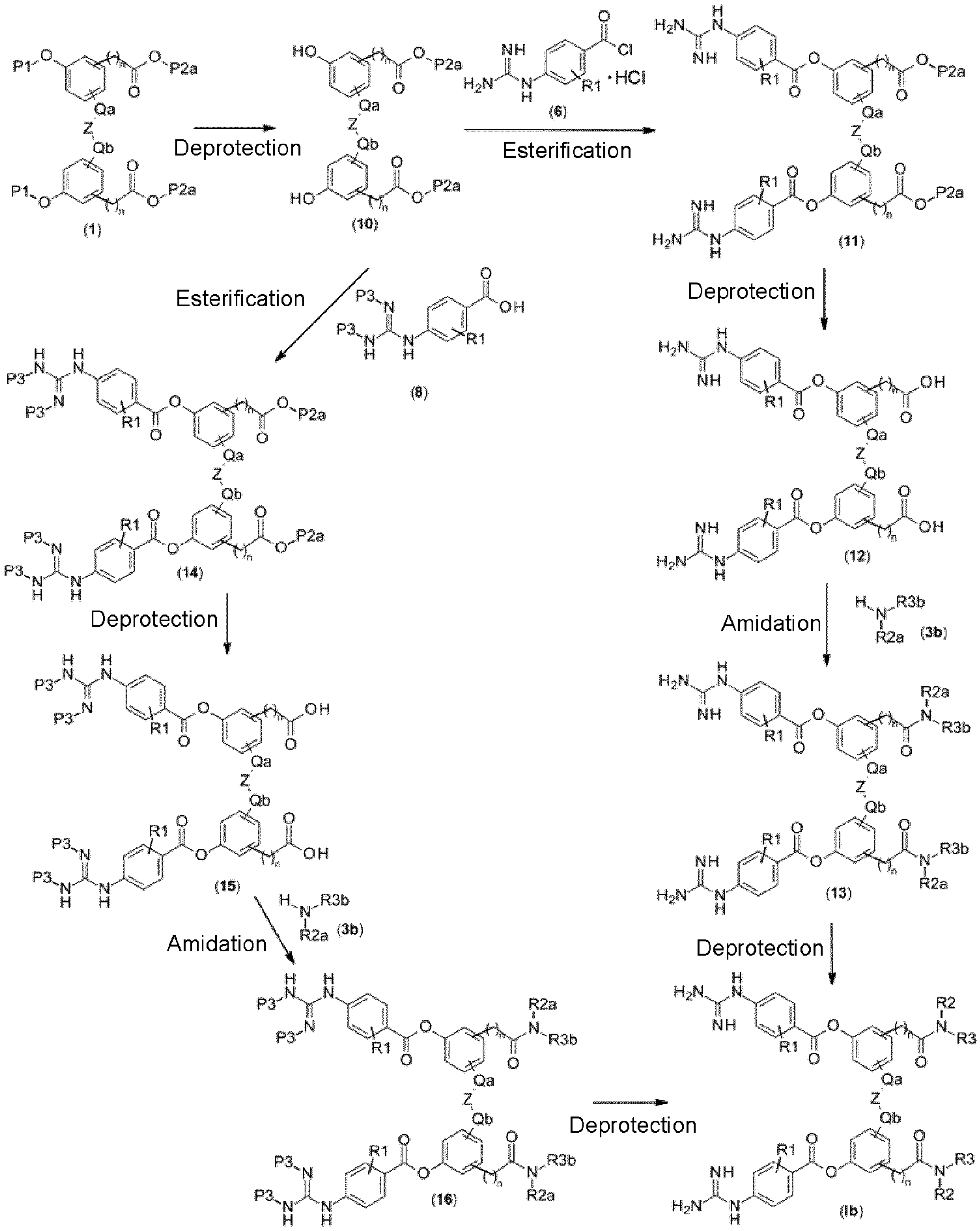


In the formula, R3b represents a linear or branched $C_{1-8}$ alkyl group substituted by one substituent selected from C(=O)OP2b, a sulfonic acid group, and a phosphoric acid group and optionally further substituted by one substituent selected from C(=O)OP2b, NR4P3, and a hydroxy group, P2b represents a protective group for a carboxyl group, and the other symbols are as defined above.

Compound (3b) can be produced by a method known per se in the art or in accordance with the method.

<Production Method 3>

Among the compounds (I), compound (Ic) can be produced by a method shown below from compound (17).

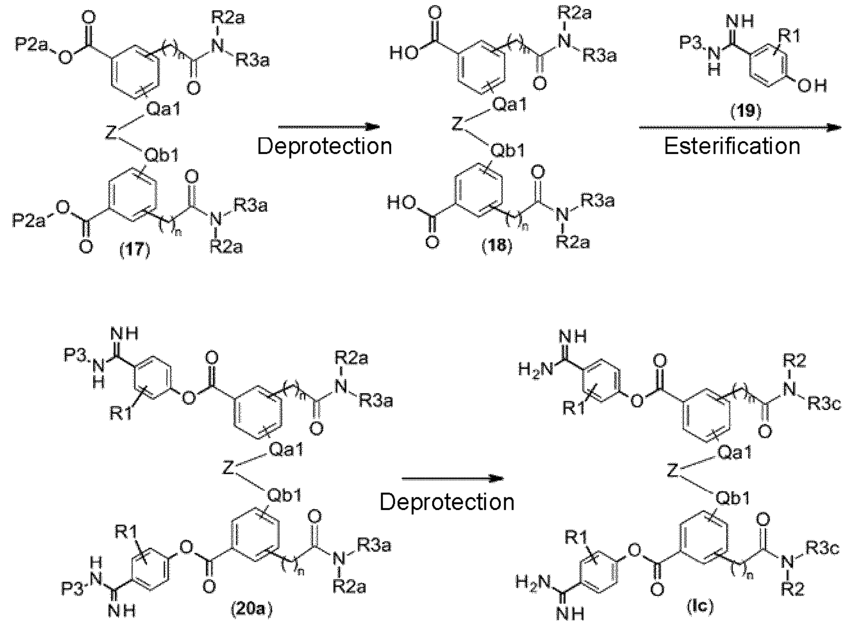

(17) (18) (20a) (Ic)

In the formula, Qa1 and Qb1 each represent $-(CH_2)_mO-*$, m represents an integer of 0 to 6, and the other symbols are as defined above.

Compound (19) can be produced by a method known per se in the art or in accordance with the method.

<Production Method 4>

Among the compounds (I), compound (Id) can be produced by a method shown below from compound (21).

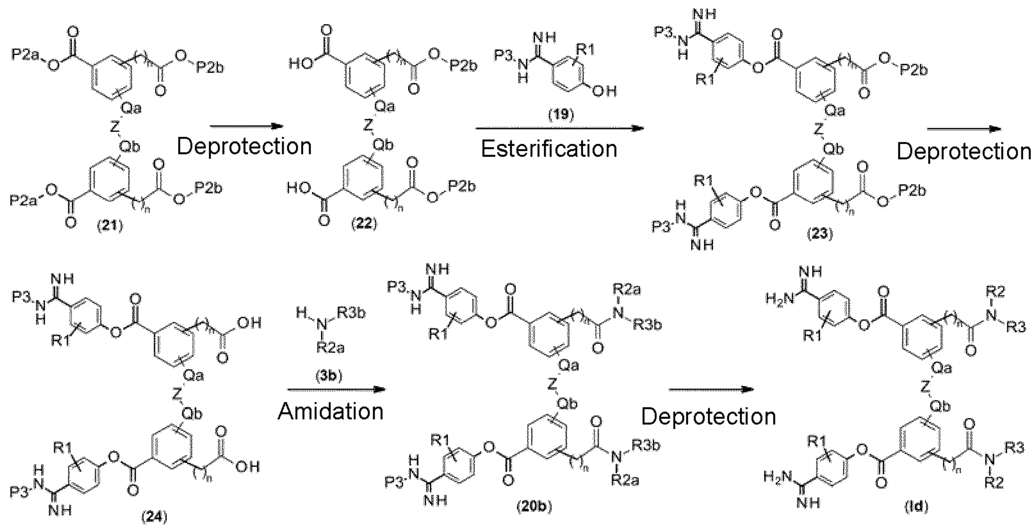

(21) (22) (23) (24) (20b) (Id)

In the formula, the symbols are as defined above.

<Production Method 5>

Among the compounds (1), compound (1a) can be produced by a method shown below from compounds (25a and 25b) .

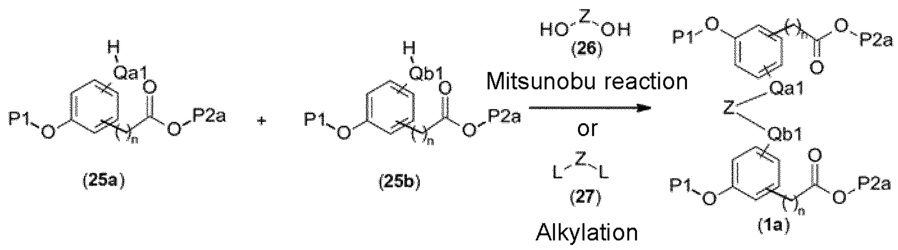


In the formula, L represents a leaving group (e.g., a methanesulfonyloxy group, a p-toluenesulfonyloxy group, and a halogen atom (e.g., chlorine, bromine, and iodine)), and the other symbols are as defined above.

Compounds (25a and 25b), (26) and (27) can each be produced by a method known per se in the art or in accordance with the method.

<Production Method 6>

Among the compounds (1), compound (1b) can be produced by a method shown below from compounds (25c and 25d) .

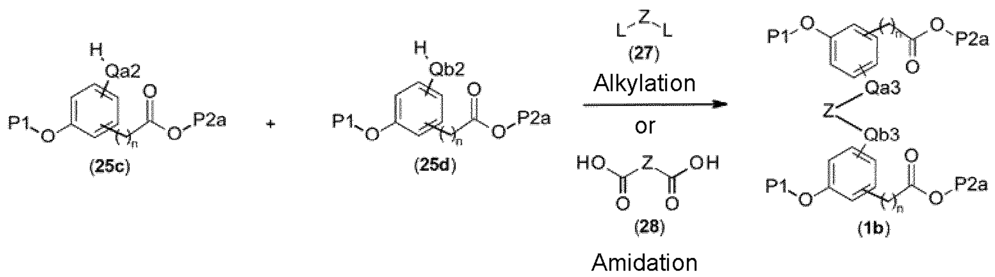


In the formula, Qa2 and Qb2 each represent - $(CH_2)_m$NR4-***, Qa3 and Qb3 each represent -$(CH_2)_m$NR4-*** or -$(CH_2)_m$NR4C(=O)-***, and the other symbols are as defined above. *** is bonded to H.

Compounds (25c and 25d) and (28) can each be produced by a method known per se in the art or in accordance with the method.

<Production Method 7>

Among the compounds (1), compound (1c) can be produced by a method shown below from compound (29).

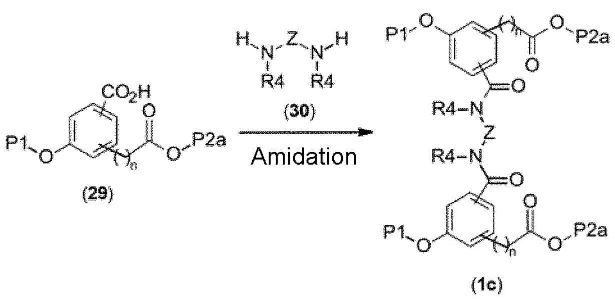


In the formula, the symbols are as defined above.

Compounds (29) and (30) can each be produced by a method known per se in the art or in accordance with the method.

<Production Method 8>

Among the compounds (1), compounds (1d) and (1e) can be produced by a method shown below from compound (31).

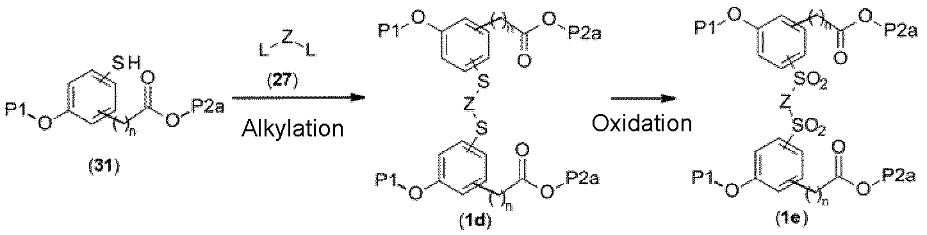


In the formula, the symbols are as defined above.

Compound (31) can be produced by a method known per se in the art or in accordance with the method.

<Production Method 9>

Among the compounds (4), compound (4a) can also be produced by a method shown below from compounds (32a and 32b) .

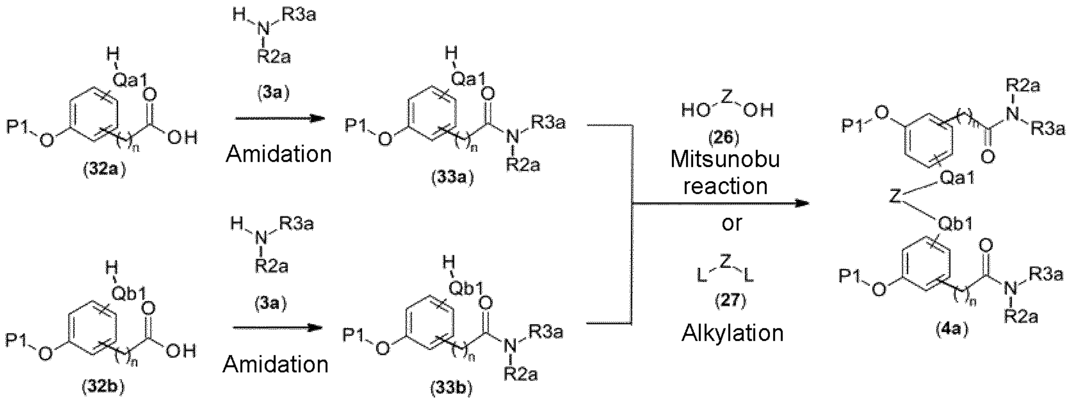


In the formula, the symbols are as defined above.

Compounds (32a and 32b) can each be produced by a method known per se in the art or in accordance with the method.

<Production Method 10>

Among the compounds (5), compound (5a) can also be produced by a method shown below from compounds (34a and 34b) .

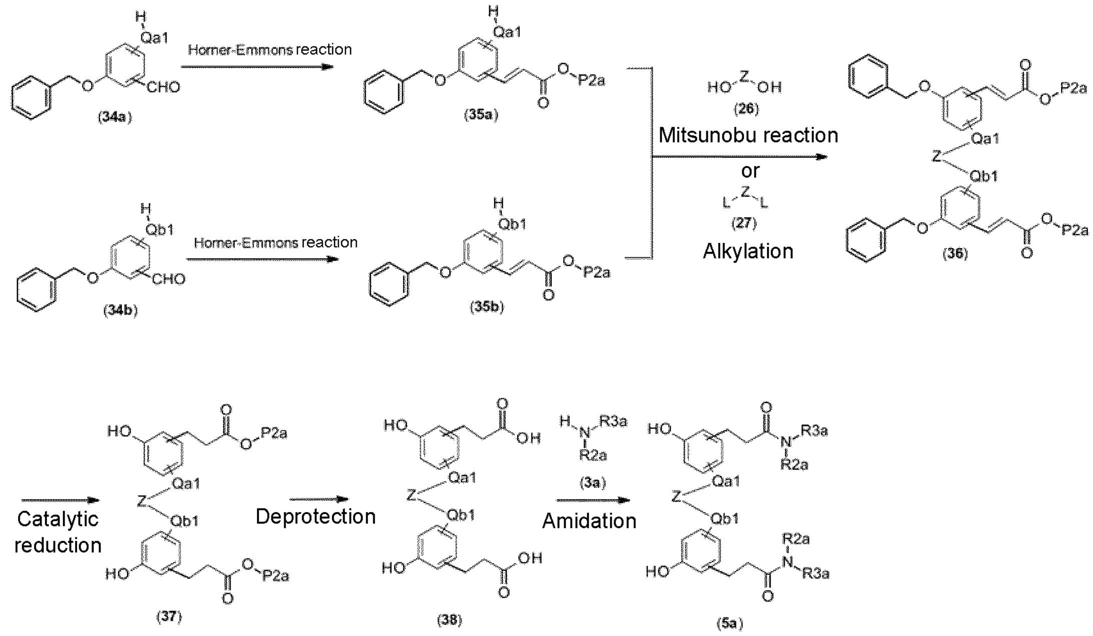


In the formula, the symbols are as defined above.

Compounds (34a and 34b) can each be produced by a method known per se in the art or in accordance with the method.

<Production Method 11>

Compound (17) can be produced by a method shown below from compounds (39a and 39b).

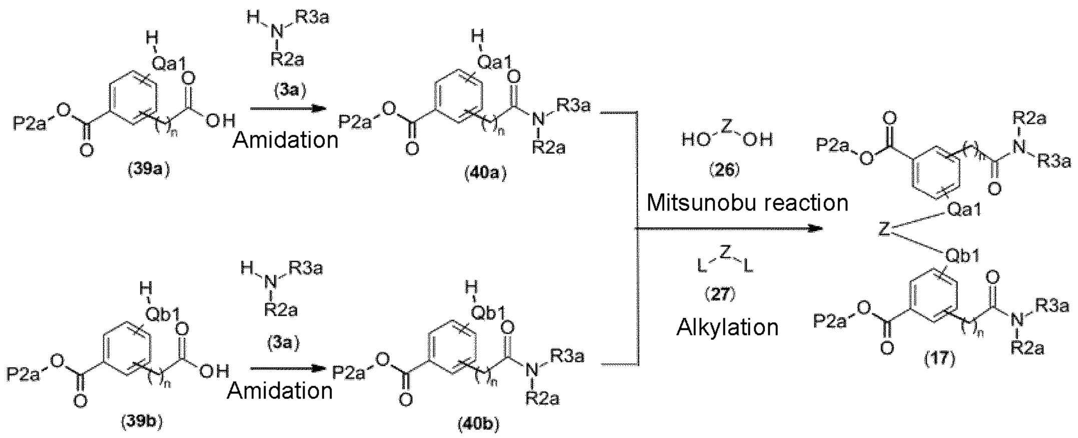


In the formula, the symbols are as defined above.

Compounds (39a and 39b) can each be produced by a method known per se in the art or in accordance with the method.

<Production Method 12>

Among the compounds (21), compound (21a) can be produced by a method shown below from compounds (41a and 41b) .

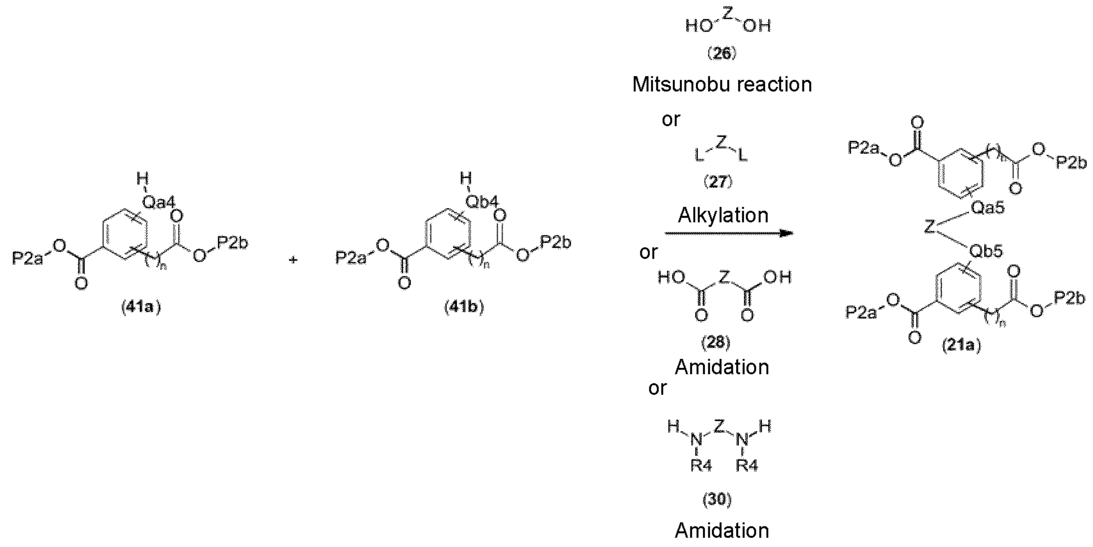


In the formula, Qa4 and Qb4 each represent $-(CH_2)_m-$***, $(CH_2)_mNR4-$***, or -C(=O)O-***, Qa5 and Qb5 each represent $(CH_2)_mO-$***, $-(CH_2)_mNR4-$***, $-(CH_2)_mNR4C(=O)-$***, or -C(=O)NR4-***, and the other symbols are as defined above. *** is bonded to H.

Compounds (41a and 41b) can each be produced by a method known per se in the art or in accordance with the method.

<Production Method 13>

Among the compounds (21), compounds (21b) and (21c) can be produced by a method shown below from compound (42) .

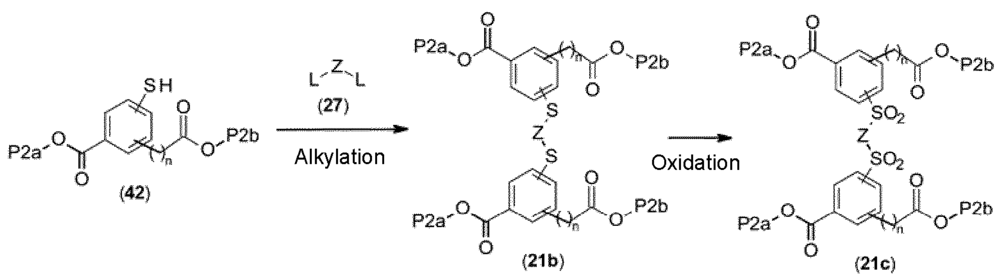


In the formula, the symbols are as defined above.

Compound (42) can be produced by a method known per se in the art or in accordance with the method.

Compounds (1, 17, and 21) are not limited by the production methods listed herein and each can also be produced by a method known per se in the art or in accordance with the method.

Compound (I) may have isomers such as optical isomers, stereoisomers, positional isomers, and rotational isomers. In such a case, one of the isomers and an isomeric mixture thereof are also included in compound (I). For example, when compound (I) has optical isomers, optical isomers resolved from a racemate are also included in compound (I). These isomers can each be obtained as a single compound by a synthesis approach, a separation approach (e.g., concentration, solvent extraction, column chromatography, and recrystallization), an optical resolution approach (e.g., fractional recrystallization method, chiral column method, and diastereomer method,), and the like known per se in the art.

Compound (I) may be amorphous or may be crystals. Single crystal forms and polymorphic mixtures are both included in compound (I). The crystals can be produced by crystallizing compound (I) by the application of a crystallization method known per se in the art.

In addition, compound (I) may be a pharmaceutically acceptable cocrystal or cocrystal salt. In this context, the cocrystal or the cocrystal salt means a crystalline substance constituted by two or more unique substances that are solids at room temperature and differ in physical properties (e.g., structure, melting point, heat of melting, hygroscopicity, solubility, and stability). The cocrystal and the cocrystal salt can be produced according to a cocrystallization method

known per se in the art.

In the present specification, a melting point means a melting point that is measured using, for example, a micro melting point apparatus (Yanaco model MP-500D or Buchi model B-545) or a DSC (differential scanning calorimetry) apparatus (SEIKO EXSTAR6000).

In general, melting points may vary depending on a measurement apparatus, measurement conditions, etc. In the present specification, the crystals may be crystals that exhibit a value different from the melting points described in the present specification as long as the value falls within a usual margin of error.

The crystals of the present invention are excellent in physicochemical properties (e.g., melting point, solubility, and stability) and biological properties (e.g., disposition (absorbability, distribution, metabolism, and excretion), and manifestation of efficacy) and are very useful as a medicament.

Compound (I) may be a solvate (e.g., a hydrate) or may be a non-solvate (e.g., a non-hydrate). All of them are included in compound (I).

A compound labeled with an isotope (e.g., $^{3}H$, $^{13}C$, $^{14}C$, $^{18}F$, $^{35}S$, and $^{125}I$) or the like is also included in compound (I).

A deuterium conversion form wherein $^{1}H$ is converted to $^{2}H(D)$ is also included in compound (I).

Compound (I) labeled or substituted with an isotope can be used as, for example, a tracer (PET tracer) for use in positron emission tomography (PET), and is useful in the fields of medical diagnosis and the like.

Compound (I) or a salt thereof (hereinafter, collectively referred to as the compound of the present invention) has an excellent enteropeptidase inhibitory effect, particularly, *in vivo,* and is useful as an enteropeptidase inhibitor.

The compound of the present invention has low toxicity (e.g., acute toxicity, chronic toxicity, genetic toxicity, reproductive toxicity, cardiac toxicity, and carcinogenicity). Thus, the compound of the present invention can be prepared into a pharmaceutical composition alone or as a mixture with a pharmacologically acceptable carrier or the like and thereby administered safely to a mammal (e.g., a mouse, a rat, a hamster, a rabbit, a cat, a dog, cattle, sheep, a monkey, and a human).

The compound of the present invention is useful as an agent for preventing or treating conditions or diseases caused by enteropeptidase.

Also, the compound of the present invention is very low absorbable orally because of a structural feature having physicochemical properties (molecular weight, polar surface area, etc.) that resist oral absorption, and is therefore excellent in safety and useful as a medicament selectively inhibiting enteropeptidase secreted from duodenal epithelial cells.

Furthermore, the solubility or the degree of crystallization of the compound of the present invention can be adjusted by adjusting the length and structure of the linker (Qa-Z-Qb) moiety.

Specifically, the compound of the present invention can be used as an agent for preventing or treating obesity based on symptomatic obesity or simple obesity, conditions or diseases associated with obesity, eating disorder, diabetes mellitus (e.g., type 1 diabetes mellitus, type 2 diabetes mellitus, gestational diabetes mellitus, and obese diabetes mellitus), hyperlipidemia (e.g., hypertriglyceridemia, hypercholesterolemia, high LDL-cholesterolemia, low HDL-cholesterolemia, and postprandial hyperlipidemia), hypertension, cardiac failure, diabetic complications [e.g., neuropathy, nephropathy, retinopathy, diabetic cardiomyopathy, cataract, macroangiopathy, osteopenia, hyperosmolar diabetic coma, infectious disease (e.g., respiratory infection, urinary tract infection, gastrointestinal infection, dermal soft tissue infection, and inferior limb infection), diabetic gangrene, xerostomia, hypacusis, cerebrovascular disorder, and peripheral blood circulation disorder], metabolic syndrome (conditions having 3 or more selected from hypertriglycerid(TG)emia, low HDL cholesterol(HDL-C)emia, hypertension, abdominal obesity, and impaired glucose tolerance), sarcopenia, reflux esophagitis, and the like.

The compound of the present invention is particularly useful as an agent for preventing or treating obesity or an agent for preventing or treating diabetes mellitus on the basis of its enteropeptidase inhibitory effect.

Examples of the symptomatic obesity include endocrine obesity (e.g., Cushing syndrome, hypothyroidism, insulinoma, obese type II diabetes mellitus, pseudohypoparathyroidism, and hypogonadism), central obesity (e.g., hypothalamic obesity, frontal lobe syndrome, and Kleine-Levin syndrome), genetic obesity (e.g., Prader-Willi syndrome and Laurence-Moon-Biedl syndrome), and drug-induced obesity (e.g., obesity caused by steroids, phenothiazines, insulins, sulfonylurea (SU) agents, and β-blockers).

Examples of the conditions or the diseases associated with obesity include impaired glucose tolerance, diabetes mellitus (particularly, type 2 diabetes mellitus and obese diabetes mellitus), abnormal lipid metabolism (which has the same meaning as that of the hyperlipidemia mentioned above), hypertension, cardiac failure, hyperuricemia or gout, fatty liver (including non-alcoholic steato-hepatitis), coronary diseases (myocardial infarction and angina pectoris), cerebral infarction (cerebral thrombosis and transient ischemic attack), bone or joint diseases (knee osteoarthritis, hip osteoarthritis, spondylosis deformans, and lumbago), sleep apnea syndrome or Pickwick syndrome, menstruation disorder (disorder of menstrual cycle, abnormality of the amount of blood lost at menstrual period and menstrual cycle, amenorrhea, and abnormality of menstruation-related symptoms), and metabolic syndrome.

The Japan Diabetes Society reported the diagnostic criteria of diabetes mellitus in 1999.

According to this report, diabetes mellitus refers to a state that meets any of a fasting blood glucose level (glucose concentration in venous plasma) of 126 mg/dl or more, a 2-hr value (glucose concentration in venous plasma) of 200 mg/dl or more in the 75 g oral glucose tolerance test (75 g OGTT), and a casual blood glucose level (glucose concentration in venous plasma) of 200 mg/dl or more. Also, a state that does not apply to the diabetes mellitus described above, and is not a state exhibiting "a fasting blood glucose level (glucose concentration in venous plasma) of less than 110 mg/dl or a 2-hr value (glucose concentration in venous plasma) of less than 140 mg/dl in the 75 g oral glucose tolerance test (75 g OGTT)" (normal type) is called "borderline type".

Also, the diagnostic criteria of diabetes mellitus were reported in 1997 by ADA (American Diabetes Association) and in 1998 by WHO (World Health Organization).

According to these reports, diabetes mellitus refers to a state that exhibits a fasting blood glucose level (glucose concentration in venous plasma) of 126 mg/dl or more and a 2-hr value (glucose concentration in venous plasma) of 200 mg/dl or more in the 75 g oral glucose tolerance test

According to the reports of ADA and WHO, impaired glucose tolerance (IGT) refers to a state that exhibits a fasting blood glucose level (glucose concentration in venous plasma) of less than 126 mg/dl and a 2-hr value (glucose concentration in venous plasma) of 140 mg/dl or more and less than 200 mg/dl in the 75 g oral glucose tolerance test. According to the report of ADA, a state exhibiting a fasting blood glucose level (glucose concentration in venous plasma) of 110 mg/dl or more and less than 126 mg/dl is called IFG (impaired fasting glucose). On the other hand, according to the report of WHO, an IFG (impaired fasting glucose) state exhibiting a 2-hr value (glucose concentration in venous plasma) less than 140 mg/dl in the 75 g oral glucose tolerance test is called IFG (impaired fasting glycemia).

The compound of the present invention is also used as an agent for preventing or treating diabetes mellitus, borderline type, impaired glucose tolerance, IFG (impaired fasting glucose), and IFG (impaired fasting glycemia) determined according to the diagnostic criteria described above. In addition, the compound of the present invention can also prevent the progression of borderline type, impaired glucose tolerance, IFG (impaired fasting glucose), or IFG (impaired fasting glycemia) into diabetes mellitus.

The compound of the present invention has an effect of suppressing body weight gain and as such, can be used as an agent suppressing body weight gain in a mammal. The mammal to which the compound of the present invention is to be applied can be any mammal desired to avoid body weight gain and may be a mammal genetically having a risk of body weight gain or may be a mammal affected by a lifestyle-related disease such as diabetes mellitus, hypertension, and/or hyperlipidemia, etc. The body weight gain may be caused by excessive dietary intake or nutritionally unbalanced diet or may be derived from concomitant drugs (e.g., insulin sensitizers having a PPAR-gamma agonist-like effect, such as troglitazone, rosiglitazone, englitazone, ciglitazone, and pioglitazone). Also, the body weight gain may be body weight gain before reaching obesity or may be body weight gain in an obesity patient. In this context, the obesity is defined as having BMI (body mass index: Body weight (kg) / [Height (m)]$^2$) of 25 or more (according to the criteria of the Japan Society for the Study of Obesity (JASSO)) for Japanese or having BMI of 30 or more (according to the criteria of WHO) for Westerners.

The compound of the present invention is also useful as an agent for preventing or treating metabolic syndrome. The incidence of cardiovascular disease is significantly high in metabolic syndrome patients, compared with patients with a single lifestyle-related disease. Therefore, the prevention or treatment of metabolic syndrome is exceedingly important for preventing cardiovascular disease.

The diagnostic criteria of metabolic syndrome were announced by WHO in 1999 and by NCEP in 2001. According to the diagnostic criteria of WHO, an individual having hyperinsulinemia or abnormal glucose tolerance as a requirement and two or more of visceral obesity, dyslipidemia (high TG or low HDL), and hypertension is diagnosed as having metabolic syndrome (World Health Organization: Definition, Diagnosis and Classification of Diabetes Mellitus and Its Complications. Part I: Diagnosis and Classification of Diabetes Mellitus, World Health Organization, Geneva, 1999). According to the diagnostic criteria of the Adult Treatment Panel III of the National Cholesterol Education Program (guideline of ischemic heart disease) in USA, an individual having three or more of visceral obesity, hypertriglyceridemia, low HDL-cholesterolemia, hypertension, and abnormal glucose tolerance is diagnosed as having metabolic syndrome (National Cholesterol Education Program: Executive Summary of the Third Report of National Cholesterol Education Program (NCEP) Expert Panel on Detection, Evaluation, and Treatment of High Blood Cholesterol in Adults (Adults Treatment Panel III). The Journal of the American Medical Association, Vol. 285, 2486-2497, 2001).

The compound of the present invention can also be used as an agent for preventing or treating, for example, osteoporosis, cachexia (e.g., cancerous cachexia, tuberculous cachexia, diabetic cachexia, cachexia associated with blood disease, cachexia associated with endocrine disease, cachexia associated with infectious disease, or cachexia caused by acquired immunodeficiency syndrome), fatty liver, polycystic ovary syndrome, renal disease (e.g., diabetic nephropathy, glomerulonephritis, glomerulosclerosis, nephrotic syndrome, hypertensive nephrosclerosis, and end-stage renal disease), muscular dystrophy, myocardial infarction, angina pectoris, cerebrovascular disorder (e.g., cerebral infarction and stroke), Alzheimer's disease, Parkinson's disease, anxiety disorder, dementia, insulin resistant syndrome,

syndrome X, hyperinsulinemia, paresthesia caused by hyperinsulinemia, acute or chronic diarrhea, inflammatory disease (e.g., chronic rheumatoid arthritis, spondylitis deformans, arthritis deformans, lumbago, gout, post-operational or post-traumatic inflammation, bloating, neuralgia, laryngopharyngitis, cystitis, hepatitis (including non-alcoholic steatohepatitis), pneumonia, pancreatitis, enteritis, inflammatory bowel disease (including inflammatory large bowel disease), colitis ulcerosa, and gastric mucosal injury (including gastric mucosal injury caused by aspirin)), small intestinal mucosal injury, malabsorption, testicular dysfunction, visceral obesity syndrome, and sarcopenia.

The compound of the present invention can also be used as an agent for preventing or treating various cancers (particularly, breast cancer (e.g., invasive ductal breast cancer, noninvasive ductal breast cancer, and inflammatory breast cancer), prostate cancer (e.g., hormone-dependent prostate cancer and hormone-independent prostate cancer), pancreatic cancer (e.g., ductal pancreatic cancer), gastric cancer (e.g., papillary adenocarcinoma, mucous adenocarcinoma, and adenosquamous carcinoma), lung cancer (e.g., non-small cell lung cancer, small-cell lung cancer, and malignant mesothelioma), colon cancer (e.g., gastrointestinal stromal tumor), rectal cancer (e.g., gastrointestinal stromal tumor), colorectal cancer (e.g., familial colorectal cancer, hereditary non-polyposis colorectal cancer, and gastrointestinal stromal tumor), small intestinal cancer (e.g., non-Hodgkin's lymphoma and gastrointestinal stromal tumor), esophageal cancer, duodenal cancer, tongue cancer, pharyngeal cancer (e.g., nasopharyngeal cancer, oropharynx cancer, and hypopharyngeal cancer), salivary gland cancer, brain tumor (e.g., pineal astrocytoma, pilocytic astrocytoma, diffuse astrocytoma, and anaplastic astrocytoma), neurilemmoma, liver cancer (e.g., primary liver cancer and extrahepatic bile duct cancer), renal cancer (e.g., renal cell cancer and transitional cell cancer of the renal pelvis and ureter), bile duct cancer, endometrial cancer, uterine cervical cancer, ovarian cancer (e.g., epithelial ovarian cancer, extragonadal germ cell tumor, ovarian germ cell tumor, and ovarian tumor of low malignant potential), bladder cancer, urethral cancer, skin cancer (e.g., intraocular (ocular) melanoma and Merkel cell carcinoma), hemangioma, malignant lymphoma, malignant melanoma, thyroid cancer (e.g., medullary thyroid cancer), parathyroid cancer, nasal cavity cancer, sinus cancer, bone tumor (e.g., osteosarcoma, Ewing tumor, uterine sarcoma, and soft tissue sarcoma), angiofibroma, sarcoma of the retina, penis cancer, testicular tumor, pediatric solid tumor (e.g., Wilms' tumor and childhood kidney tumor), Kaposi's sarcoma, Kaposi's sarcoma caused by AIDS, tumor of maxillary sinus, fibrous histiocytoma, leiomyosarcoma, rhabdomyosarcoma, leukemia (e.g., acute myeloid leukemia and acute lymphoblastic leukemia), etc.).

The compound of the present invention can also be used for the secondary prevention or suppression of progression of various diseases described above (e.g., cardiovascular events such as myocardial infarction).

A medicament comprising the compound of the present invention can be obtained using the compound of the present invention alone or as a mixture with a pharmacologically acceptable carrier according to a method known per se in the art (e.g., a method described in the Japanese Pharmacopoeia) as a method for producing pharmaceutical preparations, and safely administered orally or parenterally (e.g., administered intravenously, intramuscularly, subcutaneously, into an organ, into a nasal cavity, intracutaneously, through ocular instillation, intracerebrally, rectally, vaginally, intraperitoneally, to the inside of tumor, or to the proximity of tumor, and administered directly to a lesion) to a mammal as, for example, tablets (including sugar-coated tablets, film-coated tablets, sublingual tablets, orally disintegrating tablets, buccal tablets, and the like), pills, powders, granules, capsules (including soft capsules and microcapsules), troches, syrups, solutions, emulsions, suspensions, controlled release preparations (e.g., rapid release preparations, sustained-release preparations, and sustained-release microcapsules), aerosols, films, (e.g., orally disintegrating films, and patch films for application to the oral mucosa), injections (e.g., subcutaneous injections, intravenous injections, intramuscular injections, and intraperitoneal injections), transfusions, dermal preparations, ointments, lotions, patches, suppositories (e.g., rectal suppositories and vaginal suppositories), pellets, nasal preparations, pulmonary preparations (inhalants), or eye drops.

For the production of an oral preparation, the preparation may be coated, if necessary, for the purpose of taste masking, enteric properties, or durability.

Examples of the coating base for use in coating include sugar coating bases, aqueous film coating bases, enteric film coating bases, and sustained-release film coating bases.

Saccharose is used as the sugar coating base. Alternatively, one sugar coating base or two or more sugar coating bases in combination selected from talc, precipitated calcium carbonate, gelatin, gum arabic, pullulan, carnauba wax, and the like may be used.

Examples of the aqueous film coating base include: cellulose polymers such as hydroxypropylcellulose, hydroxypropylmethylcellulose, hydroxyethylcellulose, and methylhydroxyethylcellulose; synthetic polymers such as polyvinylacetal diethylaminoacetate, aminoalkyl methacrylate copolymer E [Eudragit E (trade name)], and polyvinylpyrrolidone; and polysaccharides such as pullulan.

Examples of the enteric film coating base include: cellulose polymers such as hydroxypropylmethylcellulose phthalate, hydroxypropylmethylcellulose acetate succinate, carboxymethylethylcellulose, and cellulose acetate phthalate; acrylic acid polymers such as methacrylic acid copolymer L [Eudragit L (trade name)], methacrylic acid copolymer LD [Eudragit L-30D55 (trade name)], and methacrylic acid copolymer S [Eudragit S (trade name)]; and naturally occurring substances such as shellac.

Examples of the sustained-release film coating base include: cellulose polymers such as ethyl cellulose; and acrylic acid polymers such as aminoalkyl methacrylate copolymer RS [Eudragit RS (trade name)] and an ethyl acrylate-methyl methacrylate copolymer suspension [Eudragit NE (trade name)].

The coating bases described above may be used as a mixture of two or more thereof at an appropriate ratio. For coating, for example, a light shielding agent such as titanium oxide or red ferric oxide may be used.

The content of the compound of the present invention in the pharmaceutical preparation is approximately 0.01 to approximately 100% by weight of the whole preparation. The dose differs depending on a recipient, an administration route, a disease, symptoms, etc. For example, when the compound of the present invention is orally administered to a diabetes mellitus patient (body weight: approximately 60 kg), the daily dose is approximately 0.01 to approximately 30 mg/kg body weight, preferably approximately 0.1 to approximately 20 mg/kg body weight, more preferably approximately 1 to approximately 20 mg/kg body weight, of the active ingredient [compound of the present invention]. This dose can be administered once a day or in several divided portions per day (e.g., in one to three potions per day).

Examples of the pharmacologically acceptable carrier described above include various organic or inorganic carrier materials routinely used as preparation materials. Examples thereof include: excipients, lubricants, binding agents, and disintegrants for solid preparations; and solvents, solubilizing agents, suspending agents, tonicity agents, buffering agents, and soothing agents for liquid preparations. If necessary, ordinary additives such as a preservative, an antioxidant, a colorant, a sweetening agent, an adsorbent, and a wetting agent can also be further used.

Examples of the excipient include lactose, saccharose, D-mannitol, starch, corn starch, crystalline cellulose, and light anhydrous silicic acid.

Examples of the lubricant include magnesium stearate, calcium stearate, talc, and colloidal silica.

Examples of the binding agent include crystalline cellulose, saccharose, D-mannitol, dextrin, hydroxypropylcellulose, hydroxypropylmethylcellulose, polyvinylpyrrolidone, starch, sucrose, gelatin, methylcellulose, and carboxymethylcellulose sodium.

Examples of the disintegrant include starch, carboxymethylcellulose, carboxymethylcellulose calcium, carboxymethyl starch sodium, and L-hydroxypropylcellulose.

Examples of the solvent include injectable water, alcohol, propylene glycol, Macrogol, sesame oil, corn oil, and olive oil.

Examples of the solubilizing agent include polyethylene glycol, propylene glycol, D-mannitol, benzyl benzoate, ethanol, trisaminomethane, cholesterol, triethanolamine, sodium carbonate, and sodium citrate.

Examples of the suspending agent include: surfactants such as stearyl triethanolamine, sodium lauryl sulfate, laurylaminopropionic acid, lecithin, benzalkonium chloride, benzethonium chloride, and glycerin monostearate; and hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose.

Examples of the tonicity agent include glucose, D-sorbitol, sodium chloride, glycerin, and D-mannitol.

Examples of the buffering agent include buffer solutions of phosphate, acetate, carbonate, citrate, and the like.

Examples of the soothing agent include benzyl alcohol.

Examples of the preservative include p-hydroxybenzoic acid esters, chlorobutanol, benzyl alcohol, phenethyl alcohol, dehydroacetic acid, and sorbic acid.

Examples of the antioxidant include sulfites, ascorbic acid, and $\alpha$-tocopherol.

Examples of the colorant include water-soluble food tar dyes (e.g., food dyes such as Food Red No. 2 and No. 3, Food Yellow No. 4 and No. 5, and Food Blue No. 1 and No. 2), water-insoluble lake dyes (e.g., aluminum salts of the water-soluble food tar dyes described above), and natural dyes (e.g., beta-carotene, chlorophyll, and ferric oxide red).

Examples of the sweetening agent include saccharin sodium, dipotassium glycyrrhizinate, aspartame, and stevia.

The compound of the present invention can be used in combination with a drug other than the compound of the present invention.

Examples of the drug (hereinafter, also referred to as a concomitant drug) that may be used in combination with the compound of the present invention include anti-obesity agents, agents for treating diabetes mellitus, agents for treating diabetic complications, agents for treating hyperlipidemia, antihypertensive agents, diuretics, chemotherapeutic agents, immunotherapeutic agents, anti-inflammatory drugs, antithrombotic agents, agents for treating osteoporosis, vitamins, antidementia drugs, drugs for the amelioration of erectile dysfunction, drugs for treating pollakiuria or urinary incontinence, and for treating difficulty of urination. Specific examples thereof include the following.

Examples of the anti-obesity agent include monoamine uptake inhibitors (e.g., phentermine, sibutramine, mazindol, fluoxetine, and tesofensine), serotonin 2C receptor agonists (e.g., lorcaserin), serotonin 6 receptor antagonists, histamine H3 receptor modulators, GABA modulators (e.g., topiramate), neuropeptide Y antagonists (e.g., velneperit), cannabinoid receptor antagonists (e.g., rimonabant and taranabant), ghrelin antagonists, ghrelin receptor antagonists, ghrelinacylation enzyme inhibitors, opioid receptor antagonists (e.g., GSK-1521498), orexin receptor antagonists, melanocortin 4 receptor agonists, 11$\beta$-hydroxysteroid dehydrogenase inhibitors (e.g., AZD-4017), pancreatic lipase

inhibitors (e.g., orlistat and cetilistat), β3 agonists (e.g., N-5984), diacylglycerol acyltransferase 1 (DGAT1) inhibitors, acetyl-CoA carboxylase (ACC) inhibitors, stearoyl-CoA desaturated enzyme inhibitors, microsomal triglyceride transfer protein inhibitors (e.g., R-256918), Na-glucose cotransporter inhibitors (e.g., JNJ-28431754 and remogliflozin), $NF_{\kappa}$ inhibitors (e.g., HE-3286), PPAR agonists (e.g., GFT-505 and DRF-11605), phosphotyrosine phosphatase inhibitors (e.g., sodium vanadate and trodusquemine), GPR119 agonists (e.g., PSN821, MBX-2982, and APD597), glucokinase activators (e.g., AZD-1656), leptin, leptin derivatives (e.g., metreleptin), CNTF (ciliary neurotrophic factor), BDNF (brain-derived neurotrophic factor), cholecystokinin agonists, glucagonlike peptide-1 (GLP-1) preparations (e.g., animal GLP-1 preparations extracted from the bovine or swine pancreas; human GLP-1 preparations genetically synthesized by using *Escherichia. coli* or yeast; fragments or derivatives of GLP-1 (e.g., exenatide and liraglutide)), amylin preparations (e.g., pramlintide and AC-2307), neuropeptide Y agonists (e.g., PYY3-36, derivatives of PYY3-36, obineptide, TM-30339, and TM-30335), oxyntomodulin preparations: FGF21 preparations (e.g., animal FGF21 preparations extracted from the bovine or swine pancreas; human FGF21 preparations genetically synthesized using *Escherichia coli* or yeast; and fragments or derivatives of FGF21), and anorexigenic agents (e.g., P-57).

Examples of the agent for treating diabetes mellitus include insulin preparations (e.g., animal insulin preparations extracted from the bovine or swine pancreas; human insulin preparations genetically synthesized using *Escherichia coli* or yeast; zinc insulin; protamine zinc insulin; fragments or derivatives of insulin (e.g., INS-1), and oral insulin preparations), insulin sensitizers (e.g., pioglitazone or a salt thereof (preferably, hydrochloride), rosiglitazone or a salt thereof (preferably, maleate), metaglidasen, AMG-131, balaglitazone, MBX-2044, rivoglitazone, aleglitazar, chiglitazar, lobeglitazone, PLX-204, PN-2034, GFT-505, THR-0921, and compounds described in WO2007/013694, WO2007/018314, WO2008/093639, or WO2008/099794), α-glucosidase inhibitors (e.g., voglibose, acarbose, miglitol, and emiglitate), biguanides (e.g., metformin, buformin, and their salts (e.g., hydrochloride, fumarate, and succinate)), insulin secretagogues (e.g., sulfonylurea (e.g., tolbutamide, glibenclamide, gliclazide, chlorpropamide, tolazamide, acetohexamide, glyclopyramide, glimepiride, glipizide, and glybuzole), repaglinide, nateglinide, mitiglinide, or calcium salt hydrate thereof), dipeptidyl peptidase IV inhibitors (e.g., alogliptin or a salt thereof (preferably, benzoate), trelagliptin or a salt thereof (preferably, succinate), Vildagliptin, Sitagliptin, saxagliptin, BI1356, GRC8200, MP-513, PF-00734200, PHX1149, SK-0403, ALS2-0426, TA-6666, TS-021, KRP-104, β3 agonists (e.g., N-5984), GPR40 agonists (e.g., fasiglifam and compounds described in WO2004/041266, WO2004/106276, WO2005/063729, WO2005/063725, WO2005/087710, WO2005/095338, WO2007/013689, or WO2008/001931), GLP-1 receptor agonists (e.g., GLP-1, GLP-1 MR preparations, liraglutide, exenatide, AVE-0010, BIM-51077, Aib(8,35)hGLP-1(7,37)$NH_2$, CJC-1131, and albiglutide), amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists, and FBPase inhibitors), SGLT2 (sodium-glucose cotransporter 2) inhibitors (e.g., dapagliflozin, AVE2268, TS-033, YM543, TA-7284, remogliflozin, and ASP1941), SGLT1 inhibitors, 11β-hydroxysteroid dehydrogenase inhibitors (e.g., BVT-3498 and INCB-13739), adiponectin or agonists thereof, IKK inhibitors (e.g., AS-2868), leptin resistance improving drugs, somatostatin receptor agonists, glucokinase activators (e.g., piragliatin, AZD1656, AZD6370, TTP-355, and compounds described in WO2006/112549, WO2007/028135, WO2008/047821, WO2008/050821, WO2008/136428, or WO2008/156757), GIP (glucose-dependent insulinotropic peptide), GPR119 agonists (e.g. PSN821, MBX-2982, and APD597), FGF21, and FGF analogs.

Examples of the agent for treating diabetic complications include aldose reductase inhibitors (e.g., tolrestat, epalrestat, zopolrestat, fidarestat, CT-112, ranirestat (AS-3201), and lidorestat), neurotrophic factor and increasing agents thereof (e.g., NGF, NT-3, BDNF, neurotrophic production or secretion promoting agents described in WO01/14372 (e.g., 4-(4-chlorophenyl)-2-(2-methyl-1-imidazolyl)-5-[3-(2-methylphenoxy)propyl]oxazole), and compounds described in WO2004/039365), PKC inhibitors (e.g., ruboxistaurin mesylate), AGE inhibitors (e.g., ALT946, N-phenacylthiazolium bromide (ALT766), EXO-226, Pyridorin, and pyridoxamine), GABA receptor agonists (e.g., gabapentin and pregabalin), serotonin and noradrenalin reuptake inhibitors (e.g., duloxetine), sodium channel inhibitors (e.g., lacosamide), active oxygen scavengers (e.g., thioctic acid), cerebral vasodilators (e.g., tiapuride and mexiletine), somatostatin receptor agonists (e.g., BIM23190), and apoptosis signal regulating kinase-1 (ASK-1) inhibitors.

Examples of the agent for treating hyperlipidemia include HMG-CoA reductase inhibitors (e.g., pravastatin, simvastatin, lovastatin, atorvastatin, fluvastatin, rosuvastatin, pitavastatin, and their salts (e.g., sodium salt and calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidin-4-acetic acid), fibrate compounds (e.g., bezafibrate, clofibrate, simfibrate, and clinofibrate), anion exchange resin (e.g., colestyramine), probucol, nicotinic acid drugs (e.g., nicomol, niceritrol, and Niaspan), ethyl icosapentate, phytosterol (e.g., soysterol and γ-oryzanol)), cholesterol absorption inhibitors (e.g., zechia), CETP inhibitors (e.g., dalcetrapib and anacetrapib), and ω-3 fatty acid preparations (e.g., ω-3-fatty acid ethyl esters 90).

Examples of the antihypertensive agent include angiotensin converting enzyme inhibitors (e.g., captopril, enalapril, and delapril), angiotensin II antagonists (e.g., candesartan cilexetil, candesartan, losartan, losartan potassium, eprosartan, valsartan, telmisartan, irbesartan, tasosartan, olmesartan, olmesartan medoxomil, azilsartan, and azilsartan

medoxomil), calcium antagonists (e.g., manidipine, nifedipine, amlodipine, efonidipine, nicardipine, amlodipine, and cilnidipine), β blockers (e.g., metoprolol, atenolol, propranolol, carvedilol, and pindolol), and clonidine.

Examples of the diuretic include xanthine derivatives (e.g., theobromine sodium salicylate, and theobromine calcium salicylate), thiazide preparations (e.g., ethiazide, cyclopenthiazide, trichloromethiazide, hydrochlorothiazide, hydroflumethiazide, benzylhydrochlorothiazide, penflutiazide, poly 5 thiazide, and methyclothiazide), antialdosterone preparations (e.g., spironolactone and triamterene), carbonic anhydrase inhibitors (e.g., acetazolamide), chlorobenzenesulfonamide agents (e.g., chlortalidone, mefruside, and indapamide), azosemide, isosorbide, ethacrynic acid, piretanide, bumetanide, and furosemide.

Examples of the chemotherapeutic agent include alkylating agents (e.g., cyclophosphamide and ifosfamide), antimetabolites (e.g., methotrexate and 5-fluorouracil), anticancer antibiotics (e.g., mitomycin and adriamycin), plant-derived anticancer agents (e.g., vincristine, vindesine, and Taxol), cisplatin, carboplatin, and etoposide. Among others, a 5-fluorouracil derivative furtulon or neofurtulon is preferred.

Examples of the immunotherapeutic agent include microbial or bacterial components (e.g., muramyl dipeptide derivatives and Picibanil), polysaccharides having immunoenhancing activity (e.g., lentinan, sizofiran, and Krestin), cytokines obtained by genetic engineering approaches (e.g., interferon and interleukin (IL)), and colony-stimulating factors (e.g., granulocyte colony-stimulating factor, and erythropoietin). Among others, interleukins such as IL-1, IL-2, and IL-12 are preferred.

Examples of the anti-inflammatory drug include nonsteroidal anti-inflammatory drugs such as aspirin, acetaminophen, and indomethacin.

Examples of the antithrombotic agent include heparin (e.g., heparin sodium, heparin calcium, enoxaparin sodium, and dalteparin sodium), warfarin (e.g., warfarin potassium), anti-thrombin drugs (e.g., argatroban and dabigatran), FXa inhibitors (e.g., rivaroxaban, apixaban, edoxaban, YM150, and compounds described in WO02/06234, WO2004/048363, WO2005/030740, WO2005/058823, or WO2005/113504), thrombolytic agents (e.g., urokinase, tisokinase, alteplase, nateplase, monteplase, and pamiteplase), and platelet aggregation inhibitors (e.g., ticlopidine hydrochloride, clopidogrel, prasugrel, E5555, SHC530348, cilostazol, ethyl icosapentate, beraprost sodium, and sarpogrelate hydrochloride).

Examples of the agent for treating osteoporosis include alfacalcidol, calcitriol, elcatonin, calcitonin salmon, estriol, ipriflavone, pamidronate disodium, alendronate sodium hydrate, incadronate disodium, and risedronate disodium.

Examples of the vitamin include vitamin $B_1$ and vitamin $B_{12}$.

Examples of the antidementia drug include tacrine, donepezil, rivastigmine, and galanthamine.

Examples of the drug for the amelioration of erectile dysfunction include apomorphine and sildenafil citrate.

Examples of the drug for treating pollakiuria or urinary incontinence include flavoxate hydrochloride, oxybutynin hydrochloride, and propiverine hydrochloride.

Examples of the agent for treating difficulty of urination include acetylcholine esterase inhibitors (e.g., distigmine).

A drug confirmed to have a cachexia-ameliorating effect either in animal models or clinically, i.e., a cyclooxygenase inhibitor (e.g., indomethacin), a progesterone derivative (e.g., megestrol acetate), glucocorticoid (e.g., dexamethasone), a metoclopramide drug, a tetrahydrocannabinol drug, an agent improving fat metabolism (e.g., eicosapentaenoic acid), growth hormone, IGF-1, or an antibody against a cachexia-inducing factor TNF-α, LIF, IL-6 or oncostatin M, or the like can also be used in combination with the compound of the present invention.

Alternatively, a glycation inhibitor (e.g., ALT-711), a nerve regeneration-promoting drug (e.g., Y-128, VX853, and prosaptide), an antidepressant (e.g., desipramine, amitriptyline, and imipramine), an antiepileptic drug (e.g., lamotrigine, Trileptal, Keppra, Zonegran, Pregabalin, Harkoseride, and carbamazepine), an antiarrhythmic drug (e.g., mexiletine), an acetylcholine receptor ligand (e.g., ABT-594), an endothelin receptor antagonist (e.g., ABT-627), a monoamine uptake inhibitor (e.g., tramadol), a narcotic analgesic (e.g., morphine), a GABA receptor agonist (e.g., gabapentin and MR preparations of gabapentin), an α2 receptor agonist (e.g., clonidine), a local analgesic (e.g., capsaicin), an antianxiety drug (e.g., benzothiazepine), a phosphodiesterase inhibitor (e.g., sildenafil), a dopamine receptor agonist (e.g., apomorphine), midazolam, ketoconazole, or the like can also be used in combination with the compound of the present invention.

In the case of using the compound of the present invention and a concomitant drug in combination, the respective amounts of the drugs can be reduced within safe ranges in consideration of the adverse reactions of the drugs. In addition, the dosage of the concomitant drug can be reduced. As a result, adverse reactions that might be caused by the concomitant drug can be effectively prevented.

The compound of the present invention combined with a concomitant drug can produce excellent effects in such a way that:

(1) the dose of the compound of the present invention or a concomitant drug can be reduced as compared with single administration of the compound of the present invention or a concomitant drug;

(2) the period of treatment can be set longer by selecting a concomitant drug having a different mechanism of action

from that of the compound of the present invention;

(3) sustained therapeutic effects can be achieved by selecting a concomitant drug having a different mechanism of action from that of the compound of the present invention; and

(4) synergistic effects can be obtained by a combined use of the compound of the present invention and a concomitant drug.

In the case of using the compound of the present invention and a concomitant drug in combination, the times of administration of the compound of the present invention and the concomitant drug are not limited, and the compound of the present invention and the concomitant drug may be administered simultaneously or in a staggered manner to a recipient. The dose of the concomitant drug can conform to doses clinically used and can be appropriately selected depending on a recipient, an administration route, a disease, a combination, etc.

Examples of the administration mode of the compound of the present invention and the concomitant drug include (1) administration of a single preparation obtained by simultaneously processing the compound of the present invention and the concomitant drug, (2) simultaneous administration of two preparations separately obtained from the compound of the present invention and the concomitant drug, through the same administration route, (3) administration of two preparations separately obtained from the compound of the present invention and the concomitant drug, through the same administration route in a staggered manner, (4) simultaneous administration of two preparations separately obtained from the compound of the present invention and the concomitant drug, through different administration routes, and (5) administration of two preparations separately obtained from the compound of the present invention and the concomitant drug, through different administration routes in a staggered manner (e.g., administration in the order of the compound of the present invention and the concomitant drug, or in the reverse order).

Examples

The present invention will be further described in detail by the following examples, test examples, and preparation examples, which are not intended to limit the present invention and may be modified within the scope of the present invention.

Throughout the following examples, the term "room temperature" generally refers to a temperature of about 10°C to about 35°C; the "ratio" shown in a solvent mixture is a volume ratio, unless otherwise specified; and the term "%" refers to % by weight, unless otherwise specified.

The elution in column chromatography in Examples was carried out under observation by thin layer chromatography (TLC), unless otherwise specified. In the TLC observation, 60 $F_{254}$ manufactured by Merck was used as a TLC plate, and a solvent used as an elution solvent in column chromatography was used as a development solvent. Also, a UV detector was adopted in detection. The term "NH" in silica gel column chromatography indicates that aminopropyl silane-bonded silica gel was used. The term "Diol" indicates that 3-(2,3-dihydroxypropoxy)propylsilane-bonded silica gel was used. The term "C18" in preparative high-performance liquid chromatography (HPLC) indicates that octadecyl-bonded silica gel was used. The ratio of elution solvent is a volume ratio, unless otherwise specified.

$^1$H NMR analysis was performed with, for example, ACD/SpecManager (trade name) software. Very gentle peaks of protons of, for example, hydroxyl groups, amino groups, and carboxylic acid, will not be described.

MS was measured by LC/MS. As an ionization method, an ESI method or an APCI method was used. Measured values (found) are shown as the data. In general, a molecular ion peak is observed. However, the peak observed may be of a fragment ion. In a salt, generally, the peak observed is of a free molecular ion or a fragment ion.

In the following examples, the following abbreviations are used.

mp: melting point
MS: mass spectrum
M: molar concentration
N: normality
$CDCl_3$: deuterated chloroform
DMSO-$d_6$: deuterated dimethyl sulfoxide
$CD_3OD$: Deuterated methanol
$^1$H NMR: proton nuclear magnetic resonance
LC/MS: liquid chromatograph mass spectrometer
ESI: electrospray ionization
APCI: atomospheric pressure chemical ionization
TFA: trifluoroacetic acid
HATU: 1-[bis(dimethylamino)methylene]-1H-1,2,3-triazolo[4,5-b]pyridinium 3-oxide hexafluorophosphate
DMAP: N,N-dimethyl-4-aminopyridine

Example 1

(2S)-2-[[3-[2-[2-[2-[3-[[(1S)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid ditrifluoroacetate

A) Di-tert-butyl (2S)-2-[(3,5-dihydroxybenzoyl)amino]butanedioate

A mixture of 3,5-dihydroxybenzoic acid (4.50 g), di-tert-butyl (2S)-2-aminosuccinate hydrochloride (9.05 g), 1-ethyl-3-(3-dimethylaminopropyl)carbodiimide hydrochloride (6.72 g), 1-hydroxybenzotriazole monohydrate (5.37 g), N,N-diisopropylethylamine (3.77 g), and N,N-dimethylformamide (45 ml) was stirred overnight at room temperature, and the reaction mixture was then diluted with ethyl acetate. The mixture was washed with water, then dried over anhydrous magnesium sulfate, and concentrated under reduced pressure. The residue was purified by silica gel column chromatography (ethyl acetate/hexane) to obtain the title compound (8.50 g).
MS: $[M+Na]^+$ 404.2.

B) Di-tert-butyl (2S)-2-[(3-benzyloxy-5-hydroxybenzoyl)amino]butanedioate

Cyanomethylenetributylphosphorane (10.5 g) was added to a mixture of di-tert-butyl (2S)-2-[(3,5-dihydroxybenzoyl)amino]butanedioate (8.30 g), benzyl alcohol (2.35 g), and toluene (50 ml) at room temperature, followed by stirring at 100°C for 2 hours and subsequent concentration under reduced pressure. The residue was purified by silica gel column chromatography (ethyl acetate/hexane) to obtain the title compound (2.75 g) .
MS: $[M+H]^+$ 472.3.

C) Di-tert-butyl (2S)-2-[[3-benzyloxy-5-[2-[2-[2-[3-benzyloxy-5-[[(1S)-3-tert-butoxy-1-tert-butoxycarbonyl-3-oxopropyl]carbamoyl]phenoxy]ethoxy]ethoxy]ethoxy]benzoyl] amino]butanedioate

Cyanomethylenetributylphosphorane (1.00 g) was added to a mixture of di-tert-butyl (2S)-2-[(3-benzyloxy-5-hydroxybenzoyl)amino]butanedioate (0.98 g), triethylene glycol (0.156 g), and toluene (20 ml) at room temperature, followed by stirring at 100°C for 2 hours and subsequent concentration under reduced pressure. The residue was purified by silica gel column chromatography (ethyl acetate/hexane) to obtain the title compound (0.90 g).
MS: $[M+H]^+$ 1057.4.

D) Di-tert-butyl (2S)-2-[[3-[2-[2-[2-[3-[[(1S)-3-tert-butoxy-1-tert-butoxycarbonyl-3-oxopropyl]carbamoyl]-5-hydroxyphenoxy]ethoxy]ethoxy]ethoxy]-5-hydroxybenzoyl]amino]butanedioate

A mixture of di-tert-butyl (2S)-2-[[3-benzyloxy-5-[2-[2-[2-[3-benzyloxy-5-[[(1S)-3-tert-butoxy-1-tert-butoxycarbonyl-3-oxopropyl]carbamoyl]phenoxy]ethoxy]ethoxy]ethoxy]benzoyl] amino]butanedioate (0.90 g), 10% palladium on carbon (water content: about 55%, 0.20 g), and ethyl acetate (20 ml) was stirred overnight under a hydrogen atmosphere at room temperature, and the catalyst was then removed by filtration, followed by concentration under reduced pressure. The residue was purified by silica gel column chromatography (ethyl acetate/hexane) to obtain the title compound (0.62 g).
MS: $[M+H]^+$ 877.3.

E) Di-tert-butyl (2S)-2-[[3-[2-[2-[2-[3-[[(1S)-3-tert-butoxy-1-tert-butoxycarbonyl-3-oxopropyl]carbamoyl]-5-(4-guanidinobenzoyl)oxyphenoxy]ethoxy]ethoxy]ethoxy]-5-(4-guanidinobenzoyl) oxybenzoyl] amino]butanedioate

4-Guanidinobenzoyl chloride hydrochloride (0.107 g) was added in small portions to a mixture of di-tert-butyl (2S)-2-[[3-[2-[2-[2-[3-[[(1S)-3-tert-butoxy-1-tert-butoxycarbonyl-3-oxopropyl]carbamoyl]-5-hydroxyphenoxy]ethoxy]ethoxy]ethoxy]-5-hydroxybenzoyl]amino]butanedioate (0.10 g), pyridine (0.4 mL), and N-methyl-2-pyrrolidone (0.4 mL) at 50°C. After the completion of reaction, the solvent was removed under reduced pressure, followed by purification by preparative HPLC (C18, mobile phase: water/acetonitrile (system containing 0.1% TFA)) to obtain the title compound (0.099 g).
MS: $[M+H]^+$ 1199.7.

F) (2S)-2-[[3-[2-[2-[2-[3-[[(1S)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid ditrifluoroacetate

A solution of di-tert-butyl (2S)-2-[[3-[2-[2-[2-[3-[[(1S)-3-tert-butoxy-1-tert-butoxycarbonyl-3-oxopropyl]carbamoyl]-5-(4-guanidinobenzoyl)oxyphenoxy]ethoxy]ethoxy]ethoxy]-5-(4-guanidinobenzoyl)oxybenzoyl]amino]butanedioate (99 mg) in trifluoroacetic acid (2.00 ml) was stirred at room temperature for 2 hours and then concentrated under reduced pressure. Ethyl acetate and diethyl ether were added to the residue, and the resulting solid was then collected by filtration to obtain the title compound (80 mg) .
$^1$H NMR (300 MHz, DMSO-$d_6$) δ 2.63-2.75 (2H, m), 2.77-2.92 (2H, m), 3.64 (4H, s), 3.72-3.84 (4H, m), 4.19 (4H, br s), 4.66-4.82 (2H, m), 7.12 (2H, s), 7.33 (2H, s), 7.43 (6H, br d, J = 8.8 Hz), 7.82 (8H, s), 8.16 (4H, d, J = 8.4 Hz), 8.82 (2H, br d, J = 7.6 Hz), 10.10-10.43 (2H, m), 12.35-12.93 (4H, m).

Example 4

(2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioic acid ditrifluoroacetate

A) 4-Benzyloxy-2-hydroxybenzaldehyde

Benzyl bromide (8.60 ml) was added to a mixture of 2,4-dihydroxybenzaldehyde (10.0 g), sodium hydrogen carbonate (7.30 g), and acetonitrile (200 ml) at room temperature, followed by stirring at 80°C for 1 day. 1 M hydrochloric acid was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (11.3 g).
MS: $[M+H]^+$ 229.1.

B) (E)-3-(4-Benzyloxy-2-hydroxyphenyl)prop-2-enoic acid

[(Ethoxycarbonyl)methylene]triphenylphosphorane (6.05 g) was added to a solution of 4-benzyloxy-2-hydroxybenzaldehyde (3.44 g) in toluene (92 ml) at room temperature, followed by stirring overnight and subsequent concentration under reduced pressure. 2 M sodium hydroxide aqueous solution (30.1 ml) was added to a mixture of the residue, methanol (20 ml), and tetrahydrofuran (20 ml), followed by stirring overnight at room temperature and subsequent concentration under reduced pressure. The residue was washed with diethyl ether, and 2 M hydrochloric acid (20 ml) was then added to the residue, followed by extraction with ethyl acetate. The organic layer was washed with a saturated saline solution, then dried over anhydrous magnesium sulfate, and concentrated under reduced pressure to obtain the title compound (3.65 g).
MS: [M-H]- 268.9.

C) Di-tert-butyl (2S)-2-[[(E)-3-(4-benzyloxy-2-hydroxyphenyl)prop-2-enoyl]amino]butanedioate

A mixture of (E)-3-(4-benzyloxy-2-hydroxyphenyl)prop-2-enoic acid (3.15 g), di-tert-butyl (2S)-2-aminosuccinate hydrochloride (3.94 g), 1-ethyl-3-(3-dimethylaminopropyl)carbodiimide hydrochloride (2.46 g), 1-hydroxybenzotriazole monohydrate (1.96 g), N,N-diisopropylethylamine (2.19 ml), and N,N-dimethylformamide (30 ml) was stirred overnight at room temperature and then diluted with ethyl acetate. The diluted mixture was washed with water, then dried over anhydrous magnesium sulfate, and concentrated under reduced pressure. The residue was purified by silica gel column chromatography (ethyl acetate/hexane) to obtain the title compound (5.30 g).
MS: $[M+H]^+$ 498.2.

D) Di-tert-butyl (2S)-2-[[(E)-3-[4-benzyloxy-2-[2-[2-[2-[5-benzyloxy-2-[(E)-3-[[(1S)-3-tert-butoxy-1-tert-butoxycarbonyl-3-oxopropyl]amino]-3-oxoprop-1-enyl]phenoxy]ethoxy]ethoxy]ethoxy]phenyl]prop-2-enoyl]amino]butanedioate

Cyanomethylenetributylphosphorane (3.86 g) was added to a mixture of di-tert-butyl (2S)-2-[[(E)-3-(4-benzyloxy-2-hydroxyphenyl)prop-2-enoyl]amino]butanedioate (5.30 g), triethylene glycol (800 mg), and toluene (53 ml) at room temperature, followed by stirring at 100°C for 1 hour and subsequent concentration under reduced pressure. The residue was purified by silica gel column chromatography (ethyl acetate/hexane) to obtain the title compound (5.36 g).
MS: $[M+H]^+$ 1109.5.

E) Di-tert-butyl (2S)-2-[3-[2-[2-[2-[2-[2-[3-[[(1S)-3-tert-butoxy-1-tert-butoxycarbonyl-3-oxopropyl]amino]-3-oxopropyl]-5-hydroxyphenoxy]ethoxy]ethoxy]ethoxy]-4-hydroxyphenyl]propanoylamino]butanedioate

A mixture of di-tert-butyl (2S)-2-[[(E)-3-[4-benzyloxy-2-[2-[2-[2-[5-benzyloxy-2-[(E)-3-[[(1S)-3-tert-butoxy-1-tert-butoxycarbonyl-3-oxopropyl]amino]-3-oxoprop-1-enyl]phenoxy]ethoxy]ethoxy]ethoxy]phenyl]prop-2-enoyl]amino]butanedioate (5.36 g), 10% palladium on carbon (water content: about 55%, 0.540 g), and ethyl acetate (20 ml) was stirred

overnight under a hydrogen atmosphere at room temperature, and the catalyst was then removed by filtration, followed by concentration under reduced pressure. The residue was purified by silica gel column chromatography (ethyl acetate/hexane) to obtain the title compound (4.30 g).
MS: $[M+H]^+$ 933.4.

F) Di-tert-butyl (2S)-2-[3-[2-[2-[2-[2-[2-[3-[[(1S)-3-tert-butoxy-1-tert-butoxycarbonyl-3-oxopropyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioate

4-Guanidinobenzoyl chloride hydrochloride (0.100 g) was added in small portions to a mixture of di-tert-butyl (2S)-2-[3-[2-[2-[2-[2-[2-[3-[[(1S)-3-tert-butoxy-1-tert-butoxycarbonyl-3-oxopropyl]amino]-3-oxopropyl]-5-hydroxyphenoxy]ethoxy]ethoxy]ethoxy]-4-hydroxyphenyl]propanoylamino]butanedioate (0.100 g), pyridine (0.4 mL), and N-methyl-2-pyrrolidone (0.4 mL) at 50°C. After the completion of reaction, the solvent was removed under reduced pressure, followed by purification by preparative HPLC (C18, mobile phase: water/acetonitrile (system containing 0.1% TFA)) to obtain the title compound (0.099 g).
MS: $[M+H]^+$ 1255.7.

G) (2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioic acid ditrifluoroacetate

A solution of di-tert-butyl (2S)-2-[3-[2-[2-[2-[2-[2-[3-[[(1S)-3-tert-butoxy-1-tert-butoxycarbonyl-3-oxopropyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioate (96 mg) in trifluoroacetic acid (2.00 ml) was stirred at room temperature for 2 hours and then concentrated under reduced pressure. Ethyl acetate and diethyl ether were added to the residue, and the resulting solid was then collected by filtration to obtain the title compound (70 mg) .
$^1H$ NMR (300 MHz, DMSO-$d_6$) $\delta$ 2.36-2.45 (4H, m), 2.54-2.87 (8H, m), 3.64 (4H, s), 3.73-3.84 (4H, m), 4.03-4.18 (4H, m), 4.45-4.63 (2H, m), 6.74 (2H, d, J = 8.2 Hz), 6.90 (2H, s), 7.19 (2H, d, J = 8.3 Hz), 7.42 (4H, d, J = 8.4 Hz), 7.80 (8H, s), 8.14 (6H, br d, J = 8.4 Hz), 10.21 (2H, s), 12.41-12.67 (3H, m) .

Example 5

2-[[3-[2-[2-[2-[3-[Bis(carboxymethyl)carbamoyl]-5-(4-guanidinobenzoyl)oxyphenoxy]ethoxy]ethoxy]ethoxy]-5-(4-guanidinobenzoyl)oxybenzoyl]-(carboxymethyl)amino]acetic acid ditrifluoroacetate

A) Ethyl 3-benzyloxy-5-[2-[2-[2-(3-benzyloxy-5-ethoxycarbonylphenoxy)ethoxy]ethoxy]ethoxy]benzoate

Cyanomethylenetributylphosphorane (6.12 g) was added to a mixture of ethyl 3-benzyloxy-5-hydroxybenzoate (4.60 g), triethylene glycol (1.27 g), and toluene (50 ml) at room temperature, followed by stirring at 100°C for 2 hours and subsequent concentration under reduced pressure. The residue was purified by silica gel column chromatography (ethyl acetate/hexane) to obtain the title compound (4.80 g).
MS: $[M+Na]^+$ 681.2.

B) 3-Benzyloxy-5-[2-[2-[2-(3-benzyloxy-5-carboxyphenoxy)ethoxy]ethoxy]ethoxy]benzoic acid

2 M sodium hydroxide aqueous solution (18.2 ml) was added to a mixture of ethyl 3-benzyloxy-5-[2-[2-[2-(3-benzyloxy-5-ethoxycarbonylphenoxy)ethoxy]ethoxy]ethoxy]benzoate (4.80 g) and ethanol (50 ml), followed by stirring overnight at room temperature and subsequent concentration under reduced pressure. The residue was added to 2 M hydrochloric acid (18.2 ml), followed by extraction with ethyl acetate. The organic layer was washed with a saturated saline solution, then dried over anhydrous magnesium sulfate, and concentrated under reduced pressure to obtain the title compound (4.39 g).
MS: $[M+Na]^+$ 625.1.

C) 3-[2-[2-[2-(3-Carboxy-5-hydroxyphenoxy)ethoxy]ethoxy]ethoxy]-5-hydroxybenzoic acid

A mixture of 3-benzyloxy-5-[2-[2-[2-(3-benzyloxy-5-carboxyphenoxy)ethoxy]ethoxy]ethoxy]benzoic acid (4.39 g), 10% palladium on carbon (water content: about 55%, 0.4 g), and ethyl acetate (50 ml) was stirred overnight under a hydrogen atmosphere at room temperature, and the catalyst was then removed by filtration, followed by concentration under reduced pressure. The residue was purified by silica gel column chromatography (ethyl acetate/hexane) to obtain

the title compound (3.08 g).
MS: $[M+Na]^+$ 445.0.

D) Benzyl 3-[2-[2-[2-(3-benzyloxycarbonyl-5-hydroxyphenoxy)ethoxy]ethoxy]ethoxy]-5-hydroxybenzoate

Benzyl bromide (1.82 ml) was added to a mixture of 3-[2-[2-[2-(3-carboxy-5-hydroxyphenoxy)ethoxy]ethoxy]ethoxy]-5-hydroxybenzoic acid (3.08 g), N,N-diisopropylethylamine (4.12 ml), and N,N-dimethylformamide (60 ml) at room temperature, followed by stirring overnight at room temperature. 1 M hydrochloric acid was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (3.60 g).
MS: $[M+Na]^+$ 625.1.

E) Benzyl 3-[2-[2-[2-[3-benzyloxycarbonyl-5-(4-guanidinobenzoyl)oxyphenoxy]ethoxy]ethoxy]ethoxy]-5-(4-guanidinobenzoyl)oxybenzoate

4-Guanidinobenzoyl chloride hydrochloride (1.21 g) was added to a mixture of benzyl 3-[2-[2-[2-(3-benzyloxycarbonyl-5-hydroxyphenoxy)ethoxy]ethoxy]ethoxy]-5-hydroxybenzoate (0.78 g), pyridine (0.4 mL), and N-methyl-2-pyrrolidone (0.4 mL) at 50°C. After stirring overnight at 50°C, the solvent was removed under reduced pressure, followed by purification by preparative HPLC (C18, mobile phase: water/acetonitrile (system containing 0.1% TFA)) to obtain the title compound (0.733 g).
MS: $[M+H]^+$ 925.3.

F) 3-[2-[2-[2-[3-Carboxy-5-(4-guanidinobenzoyl)oxyphenoxy]ethoxy]ethoxy]ethoxy]-5-(4-guanidinobenzoyl)oxybenzoic acid

A mixture of benzyl 3-[2-[2-[2-[3-benzyloxycarbonyl-5-(4-guanidinobenzoyl)oxyphenoxy]ethoxy]ethoxy]ethoxy]-5-(4-guanidinobenzoyl)oxybenzoate (0.73 g), 10% palladium on carbon (water content: about 55%, 0.4 g), and ethyl acetate (20 ml) was stirred overnight under a hydrogen atmosphere at room temperature, and the catalyst was then removed by filtration, followed by concentration under reduced pressure to obtain the title compound (0.58 g).
MS: $[M+H]^+$ 745.2.

G) [3-[Bis(2-tert-butoxy-2-oxoethyl)carbamoyl]-5-[2-[2-[2-[3-[bis(2-tert-butoxy-2-oxoethyl)carbamoyl]-5-(4-guanidinobenzoyl)oxyphenoxy]ethoxy]ethoxy]ethoxy]phenyl]4 -guanidinobenzoate

A mixture of 3-[2-[2-[2-[3-carboxy-5-(4-guanidinobenzoyl)oxyphenoxy]ethoxy]ethoxy]ethoxy]-5-(4-guanidinobenzoyl)oxybenzoic acid (0.100 g), tert-butyl 2-[(2-tert-butoxy-2-oxo-ethyl)amino]acetate (0.0988 g), 1-ethyl-3-(3-dimethylaminopropyl)carbodiimide hydrochloride (0.0772 g), ethyl cyano(hydroxyimino)acetate (0.0572 g), N,N-diisopropylethylamine (0.0572 g), and N,N-dimethylformamide (2 ml) was stirred overnight at room temperature, and the solvent was then removed under reduced pressure, followed by purification by preparative HPLC (C18, mobile phase: water/acetonitrile (system containing 0.1% TFA)) to obtain the title compound (0.099 g).
MS: $[M+H]^+$ 1199.7.

H) 2-[[3-[2-[2-[2-[3-[Bis(carboxymethyl)carbamoyl]-5-(4-guanidinobenzoyl)oxyphenoxy]ethoxy]ethoxy]ethoxy]-5-(4-guanidinobenzoyl)oxybenzoyl]-(carboxymethyl)amino]acetic acid ditrifluoroacetate

A solution of [3-[bis(2-tert-butoxy-2-oxoethyl)carbamoyl]-5-[2-[2-[2-[3-[bis(2-tert-butoxy-2-oxoethyl)carbamoyl]-5-(4-guanidinobenzoyl)oxyphenoxy]ethoxy]ethoxy]ethoxy]phenyl]4 -guanidinobenzoate (99 mg) in trifluoroacetic acid (2.00 ml) was stirred at room temperature for 2 hours and then concentrated under reduced pressure. Ethyl acetate and diethyl ether were added to the residue, and the resulting solid was then collected by filtration to obtain the title compound (45 mg).
$^1H$ NMR (300 MHz, DMSO-$d_6$) $\delta$ 3.61 (4H, s), 3.74 (4H, br s), 3.96 (4H, br s), 4.03-4.18 (8H, m), 6.80 (4H, s), 7.04 (2H, s), 7.42 (4H, d, J = 8.4 Hz), 7.73-7.89 (8H, m), 8.15 (4H, d, J = 8.3 Hz), 10.17-10.39 (2H, m).

Example 6

(2R)-2-[[3-[2-[2-[2-[3-[[(1R)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid ditrifluoroacetate

A) Di-tert-butyl (2R)-2-[[3-[2-[2-[2-[3-[[(1R)-3-tert-butoxy-1-tert-butoxycarbonyl-3-oxopropyl]carbamoyl]-5-(4-guanidinobenzoyl)oxyphenoxy]ethoxy]ethoxy]ethoxy]-5-(4-guanidinobenzoyl)oxybenzoyl]amino]butanedioate

A mixture of 3-[2-[2-[2-[3-carboxy-5-(4-guanidinobenzoyl)oxyphenoxy]ethoxy]ethoxy]ethoxy]-5-(4-guanidinobenzoyl)oxybenzoic acid (0.100 g) synthesized in F) of Example 5, di-tert-butyl (2R)-2-aminosuccinate hydrochloride (0.114 g), 1-ethyl-3-(3-dimethylaminopropyl)carbodiimide hydrochloride (0.0772 g), ethyl cyano(hydroxyimino)acetate (0.0572 g), N,N-diisopropylethylamine (0.0521 g), and N,N-dimethylformamide (2 ml) was stirred overnight at room temperature, and the solvent was then removed under reduced pressure, followed by purification by preparative HPLC (C18, mobile phase: water/acetonitrile (system containing 0.1% TFA)) to obtain the title compound (0.075 g).
MS: $[M+H]^+$ 1199.7.

B) (2R)-2-[[3-[2-[2-[2-[3-[[(1R)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid ditrifluoroacetate

A solution of di-tert-butyl (2R)-2-[[3-[2-[2-[2-[3-[[(1R)-3-tert-butoxy-1-tert-butoxycarbonyl-3-oxopropyl]carbamoyl]-5-(4-guanidinobenzoyl)oxyphenoxy]ethoxy]ethoxy]ethoxy]-5-(4-guanidinobenzoyl)oxybenzoyl]amino]butanedioate (75 mg) in trifluoroacetic acid (2.00 ml) was stirred at room temperature for 2 hours and then concentrated under reduced pressure. Ethyl acetate and diethyl ether were added to the residue, and the resulting solid was then collected by filtration to obtain the title compound (55 mg) .
$^1H$ NMR (300 MHz, DMSO-$d_6$) $\delta$ 2.66 (2H, dd, J = 16.3, 7.7 Hz), 2.82 (2H, dd, J = 16.1, 6.1 Hz), 3.63 (4H, s), 3.78 (4H, br s), 4.19 (4H, br s), 4.71 (2H, q, J = 7.5 Hz), 7.12 (2H, s), 7.33 (2H, s), 7.43 (6H, br d, J = 8.9 Hz), 7.83 (8H, br s), 8.16 (4H, d, J = 8.3 Hz), 8.78 (2H, br d, J = 7.6 Hz) .

Example 7

(2S)-2-[[3-(4-Carbamimidoylphenoxy)carbonyl-5-[2-[2-[2-[3-(4-carbamimidoylphenoxy)carbonyl-5-[[(1S)-1,2-dicarboxyethyl]carbamoyl]phenoxy]ethoxy]ethoxy]ethoxy]ben zoyl]amino]butanedioic acid ditrifluoroacetate

A) 3-Benzyloxycarbonyl-5-hydroxybenzoic acid

Benzyl bromide (3.91 ml) was added to 5-hydroxybenzene-1,3-dicarboxylic acid (5.00 g), potassium carbonate (4.55 g), and N,N-dimethylformamide (100 ml), followed by stirring at room temperature for 3 hours. 2 M hydrochloric acid was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (5.57 g) as a 1:1 mixture with dibenzyl 5-hydroxybenzene-1,3-dicarboxylate.
MS: $[M-H]^-$ 271.1.

B) Di-tert-butyl (2S)-2-[(3-benzyloxycarbonyl-5-hydroxybenzoyl)amino]butanedioate

N,N-Diisopropylethylamine (5.66 ml) was added to a mixture of 3-benzyloxycarbonyl-5-hydroxybenzoic acid (3.00 g), di-tert-butyl (2S)-2-aminosuccinate hydrochloride (3.10 g), 1-ethyl-3-(3-dimethylaminopropyl)carbodiimide hydrochloride (2.53 g), 1-hydroxybenzotriazole monohydrate (2.02 g) and N,N-dimethylformamide (60 ml), followed by stirring overnight at room temperature. Water was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (1.23 g). MS: [M-H]- 498.1.

C) Di-tert-butyl (2S)-2-[[3-benzyloxycarbonyl-5-[2-[2-[2-[3-benzyloxycarbonyl-5-[[(1S)-3-tert-butoxy-1-tert-butoxycarbonyl-3-oxopropyl]carbamoyl]phenoxy]ethoxy]ethoxy]ethoxy]benzoyl] amino]butanedioate

Cyanomethylenetributylphosphorane (1.29 ml) was added to a mixture of di-tert-butyl (2S)-2-[(3-benzyloxycarbonyl-5-hydroxybenzoyl)amino]butanedioate (1.23 g), triethylene glycol (0.164 ml), and toluene (25 ml) at room temperature, followed by stirring overnight at 100°C and subsequent concentration under reduced pressure. The residue was purified by silica gel column chromatography (ethyl acetate/hexane) to obtain the title compound (1.21 g).
MS: $[M+H]^+$ 1113.5.

D) 3-[[(1S)-3-tert-Butoxy-1-tert-butoxycarbonyl-3-oxopropyl]carbamoyl]-5-[2-[2-[2-[3-[[(1S)-3-tert-butoxy-1-tert-butoxycarbonyl-3-oxopropyl]carbamoyl]-5-carboxyphenoxy]ethoxy]ethoxy]ethoxy]benzoic acid

A mixture of di-tert-butyl (2S)-2-[[3-benzyloxycarbonyl-5-[2-[2-[2-[3-benzyloxycarbonyl-5-[[(1S)-3-tert-butoxy-1-tert-butoxycarbonyl-3-oxopropyl]carbamoyl]phenoxy]ethoxy]ethoxy]ethoxy]benzoyl] amino]butanedioate (1.21 g,), 10% palladium on carbon (water content: about 55%, 0.400 g), and ethanol (25 ml) was stirred under a hydrogen atmosphere at room temperature for 3 hours, and the catalyst was then removed by filtration, followed by concentration under reduced pressure. The residue was purified by silica gel column chromatography (ethyl acetate/hexane) to obtain the title compound (0.496 g).
MS: $[M+H]^+$ 933.3.

E) Di-tert-butyl (2S)-2-[[3-[2-[2-[2-[3-[[(1S)-3-tert-butoxy-1-tert-butoxycarbonyl-3-oxopropyl]carbamoyl]-5-[4-(N-tert-butoxycarbonylcarbamimidoyl)phenoxy]carbonylphenoxy]ethox y]ethoxy]ethoxy]-5-[4-(N-tert-butoxycarbonylcarbamimidoyl)phenoxy]carbonylbenzoyl]amino ]butanedioate

N,N-Diisopropylethylamine (0.165 ml) was added to a mixture of 3-[[(1S)-3-tert-butoxy-1-tert-butoxycarbonyl-3-oxopropyl]carbamoyl]-5-[2-[2-[2-[3-[[(1S)-3-tert-butoxy-l-tert-butoxycarbonyl-3-oxopropyl]carbamoyl]-5-carboxyphenoxy]ethoxy]ethoxy]ethoxy]benzoic acid (150 mg), tert-butyl N-(4-hydroxybenzenecarboximidoyl)carbamate (114 mg), HATU (183 mg), DMAP (3.9 mg), and N,N-dimethylformamide (1 ml), followed by stirring at room temperature for 2 hours. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (153 mg).
MS: $[M+H]^+$ 1369.6.

F) (2S)-2-[[3-(4-Carbamimidoylphenoxy)carbonyl-5-[2-[2-[2-[3-(4-carbamimidoylphenoxy)carbonyl-5-[[(1S)-1,2-dicarboxyethyl]carbamoyl]phenoxy]ethoxy]ethoxy]ethoxy]ben zoyl]amino]butanedioic acid ditrifluoroacetate

4 M hydrochloric acid in ethyl acetate (10 ml) was added to a solution of di-tert-butyl (2S)-2-[[3-[2-[2-[2-[3-[[(1S)-3-tert-butoxy-1-tert-butoxycarbonyl-3-oxopropyl]carbamoyl]-5-[4-(N-tert-butoxycarbonylcarbamimidoyl)phenoxy]carbonylphenoxy]ethox y]ethoxy]ethoxy]-5-[4-(N-tert-butoxycarbonylcarbamimidoyl)phenoxy]carbonylbenzoyl]amino ]butanedioate (153 mg) in ethyl acetate (1 ml), followed by stirring at room temperature for 1 hour and subsequent concentration under reduced pressure. Trifluoroacetic acid (10 ml) was added to the residue, followed by stirring at room temperature for 1 hour and subsequent concentration under reduced pressure. The residue was purified by preparative HPLC (C18, mobile phase: water/acetonitrile (system containing 0.1% TFA)) to obtain the title compound (73.8 mg).
$^1H$ NMR (300 MHz, DMSO-$d_6$) $\delta$ 2.55-2.69 (2H, m), 2.76-2.91 (2H, m), 3.63-3.69 (4H, m), 3.78-3.85 (4H, m), 4.23-4.32 (4H, m), 4.63-4.76 (2H, m), 7.61 (4H, d, J = 8.4 Hz), 7.78 (2H, s), 7.81 (2H, s), 7.92 (4H, d, J = 8.3 Hz), 8.23 (2H, s), 8.91-8.99 (2H, m), 9.02 (4H, br s), 9.37 (4H, br s).

Example 8

(2S)-2-[[3-[2-[2-[3-[[(1S)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid ditrifluoroacetate

A) Di-tert-butyl (2S)-2-[[3-benzyloxy-5-[2-[2-[3-benzyloxy-5-[[(1S)-3-tert-butoxy-1-tert-butoxycarbonyl-3-oxopropyl]carbamoyl]phenoxy]ethoxy]ethoxy]benzoyl]amino]b utanedioate

Cyanomethylenetributylphosphorane (1.45 g) was added to a mixture of di-tert-butyl (2S)-2-[(3-benzyloxy-5-hydroxybenzoyl)amino]butanedioate (2.07 g) synthesized in B) of Example 1, 2-(2-hydroxyethoxy)ethanol (0.21 g), and toluene (40 ml), followed by stirring at 90°C for 16 hours and subsequent concentration under reduced pressure. The residue was purified by silica gel column chromatography (ethyl acetate/hexane) to obtain the title compound.
MS: $[M+H]^+$ 1013.4.

B) Di-tert-butyl (2S)-2-[[3-[2-[2-[3-[[(1S)-3-tert-butoxy-l-tert-butoxycarbonyl-3-oxopropyl]carbamoyl]-5-hydroxyphenoxy]ethoxy]ethoxy]-5-hydroxybenzoyl]amino]butanedioate

A mixture of di-tert-butyl (2S)-2-[[3-benzyloxy-5-[2-[2-[3-benzyloxy-5-[[(1S)-3-tert-butoxy-1-tert-butoxycarbonyl-3-oxopropyl]carbamoyl]phenoxy]ethoxy]ethoxy]benzoyl]amino]b utanedioate (1.09 g), 10% palladium on carbon (water content: about 55%, 0.229 g), and methanol (20 ml) was stirred under a hydrogen atmosphere at room temperature for 16 hours. The catalyst was removed by filtration, followed by concentration under reduced pressure to obtain the title compound (0.890 g).
MS: $[M+H]^+$ 833.3.

C) Di-tert-butyl (2S)-2-[[3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-[[(1S)-3-tert-butoxy-1-tert-butoxycarbonyl-3-oxopropyl]carbamoyl]phenoxy]ethoxy]ethoxy]benzoyl]amino]b utanedioate

4-[[N,N'-Bis(tert-butoxycarbonyl)carbamimidoyl]amino]benzoic acid (0.465 g), HATU (0.466 g), and N,N-diisopropylethylamine (0.317 g) were added to a mixture of di-tert-butyl (2S)-2-[[3-[2-[2-[3-[[(1S)-3-tert-butoxy-1-tert-butoxycarbonyl-3-oxopropyl]carbamoyl]-5-hydroxyphenoxy]ethoxy]ethoxy]-5-hydroxybenzoyl]amino]butanedioate (0.340 g) and N,N-dimethylformamide (3.4 ml), followed by stirring overnight at room temperature. Water was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (0.410 g).
MS: $[M+H]^+$ 1556.0.

D) (2S)-2-[[3-[2-[2-[3-[[(1S)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid ditrifluoroacetate

A solution of di-tert-butyl (2S)-2-[[3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-[[(1S)-3-tert-butoxy-1-tert-butoxycarbonyl-3-oxopropyl]carbamoyl]phenoxy]ethoxy]ethoxy]benzoyl]amino]buta nedioate (99.0 mg) in trifluoroacetic acid (2.00 ml) was stirred at room temperature for 2 hours and then concentrated under reduced pressure. Ethyl acetate and diethyl ether were added to the residue, and the resulting solid was then collected by filtration to obtain the title compound (70.0 mg).
$^1H$ NMR (300 MHz, DMSO-$d_6$) $\delta$ 2.61-2.89 (4H, m), 3.81-3.93 (4H, m), 4.23 (4H, br s), 4.72 (2H, br d, J = 6.3 Hz), 7.11-7.15 (2H, m), 7.33 (2H, s), 7.43 (6H, d, J = 8.7 Hz), 7.84 (8H, br s), 8.16 (4H, d, J = 8.7 Hz), 8.76-8.84 (2H, m), 10.02-10.64 (2H, m), 12.36-13.22 (3H, m).

Example 12

(2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioic acid dihydrochloride

A) Di-tert-butyl (2S)-2-[3-[4-[4-[[N,N'-bis(tert-butoxycarbonyl)carbamimidoyl]amino]benzoyl]oxy-2-[2-[2-[2-[5-[4-[[N,N'-bis(tert-butoxycarbonyl)carbamimidoyl]amino]benzoyl]oxy-2-[3-[[(1S)-3-tert-butoxy-1-tert-butoxycarbonyl-3-oxopropyl]amino]-3-oxopropyl]phenoxy]ethoxy]ethoxy]ethoxy]phenyl]propanoylam ino]butanedioate

4-[[N,N'-Bis(tert-butoxycarbonyl)carbamimidoyl]amino]benzoic acid (3.05 g), 1-ethyl-3-(3-dimethylaminopropyl)carbodiimide hydrochloride (1.85 g), ethyl cyano(hydroxyimino)acetate (1.37 g), and sodium hydrogen carbonate (2.70 g) were added to a mixture of di-tert-butyl (2S)-2-[3-[2-[2-[2-[2-[2-[3-[[(1S)-3-tert-butoxy-1-tert-butoxycarbonyl-3-oxopropyl]amino]-3-oxopropyl]-5-hydroxyphenoxy]ethoxy]ethoxy]ethoxy]-4-hydroxyphenyl]propanoylamino]butanedioate (3.00 g) synthesized in E) of Example 4, and acetonitrile-water (10:1) (30 ml), followed by stirring overnight at room temperature. Water was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.70 g).
$^1H$ NMR (300 MHz, DMSO-$d_6$) $\delta$ 1.35-1.47 (55H, m), 1.51 (16H, br s), 2.37-2.46 (4H, m), 2.71-2.84 (4H, m), 3.64 (4H, s), 3.77 (4H, br s), 4.08 (4H, br s), 4.48 (2H, br d, J = 7.2 Hz), 6.70-6.77 (2H, m, J = 8.2 Hz), 6.90 (2H, s), 7.13-7.20 (2H, m, J = 8.3 Hz), 7.80 (4H, br d, J = 8.3 Hz), 8.00-8.17 (6H, m), 10.25 (2H, br s), 11.24 (2H, br s) .

B) (2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl)oxyphenyl]propanoylamino]butanedioic acid dihydrochloride

A solution of di-tert-butyl (2S)-2-[3-[4-[4-[[N,N'-bis(tert-butoxycarbonyl)carbamimidoyl]amino]benzoyl]oxy-2-[2-[2-[2-[5-[4-[[N,N'-bis(tert-butoxycarbonyl)carbamimidoyl]amino]benzoyl]oxy-2-[3-[[(1S)-3-tert-butoxy-1-tert-butoxycarbonyl-3-oxopropyl]amino]-3-oxopropyl]phenoxy]ethoxy]ethoxy]ethoxy]phenyl]propanoylam ino]butanedioate (200 mg) in trifluoroacetic acid (2.00 ml) was stirred at room temperature for 2 hours and then concentrated under reduced pressure. A solution of 4 M hydrochloric acid in ethyl acetate (10 ml) was added to the residue, and the resulting solid was then collected by filtration to obtain the title compound (120 mg).
$^1H$ NMR (300 MHz, DMSO-$d_6$) $\delta$ 2.37-2.47 (4H, m), 2.54-2.72 (4H, m), 2.73-2.83 (4H, m), 3.64 (4H, s), 3.77 (4H, br s), 4.10 (4H, br s), 4.54 (2H, br d, J = 7.2 Hz), 6.71-6.77 (2H, m, J = 8.3 Hz), 6.90 (2H, s), 7.15-7.22 (2H, m, J = 8.3 Hz), 7.42 (4H, d, J = 8.5 Hz), 7.81 (8H, br s), 8.11-8.19 (6H, m), 10.37 (2H, br s).

Example 15

(2R)-2-[[3-[2-[2-[3-[[(1R)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid dihydrochloride

A) Ethyl 3-benzyloxy-5-hydroxybenzoate

Benzyl bromide (30.2 ml) was added to ethyl 3,5-dihydroxybenzoate (46.4 g), potassium carbonate (70.3 g), and N,N-dimethylformamide (500 ml), followed by stirring at room temperature for 6 hours. 2 M hydrochloric acid was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (24.1 g).
MS: $[M+H]^+$ 273.1.

B) Ethyl 3-benzyloxy-5-[2-[2-(3-benzyloxy-5-ethoxycarbonylphenoxy)ethoxy]ethoxy]benzoate

Potassium carbonate (15.2 g) was added to a mixture of ethyl 3-benzyloxy-5-hydroxybenzoate (10.0 g), diethylene glycol bis(p-toluenesulfonate) (7.61 g), and N,N-dimethylformamide (100 ml) at room temperature, followed by stirring at 50°C for 6 hours. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (7.85 g).
MS: $[M+Na]^+$ 637.3.

C) 3-Benzyloxy-5-[2-[2-(3-benzyloxy-5-carboxyphenoxy)ethoxy]ethoxy]benzoic acid

2 M sodium hydroxide aqueous solution (75 ml) was added to a mixture of ethyl 3-benzyloxy-5-[2-[2-(3-benzyloxy-5-ethoxycarbonylphenoxy)ethoxy]ethoxy]benzoate (7.85 g), water (75 ml), and ethanol (75 ml), followed by stirring overnight at room temperature and at 50°C for 1 hour and subsequent concentration under reduced pressure. The residue was neutralized with 1 M hydrochloric acid, followed by extraction with ethyl acetate. The organic layer was washed with a saturated saline solution, then dried over anhydrous magnesium sulfate, and concentrated under reduced pressure. The residue was washed with diisopropyl ether to obtain the title compound (6.50 g).
MS: $[M+Na]^+$ 581.3.

D) Di-tert-butyl (2R)-2-[[3-benzyloxy-5-[2-[2-[3-benzyloxy-5-[[(1R)-3-tert-butoxy-1-tert-butoxycarbonyl-3-oxopropyl]carbamoyl]phenoxy]ethoxy]ethoxy]benzoyl]amino]b utanedioate

N,N-Diisopropylethylamine (2.59 ml) was added to a mixture of 3-benzyloxy-5-[2-[2-(3-benzyloxy-5-carboxyphenoxy)ethoxy]ethoxy]benzoic acid (1.66 g), di-tert-butyl (2R)-2-aminosuccinate hydrochloride (1.84 g), HATU (2.49 g), and N,N-dimethylformamide (15 ml), followed by stirring at room temperature for 2.5 hours. Water was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.77 g).
MS: $[M+H]^+$ 1013.6.

E) Di-tert-butyl (2R)-2-[[3-[2-[2-[3-[[(1R)-3-tert-butoxy-1-tert-butoxycarbonyl-3-oxopropyl]carbamoyl]-5-hydroxyphenoxy]ethoxy]ethoxy]-5-hydroxybenzoyl]amino]butanedioate

A mixture of di-tert-butyl (2R)-2-[[3-benzyloxy-5-[2-[2-[3-benzyloxy-5-[[(1R)-3-tert-butoxy-1-tert-butoxycarbonyl-3-oxopropyl]carbamoyl]phenoxy]ethoxy]ethoxy]benzoyl]amino]b utanedioate (2.77 g), 10% palladium on carbon (water content: about 55%, 1.00 g), and ethanol (30 ml) was stirred under a hydrogen atmosphere at room temperature for 3 hours, and the catalyst was then removed by filtration, followed by concentration under reduced pressure. The residue was purified by silica gel column chromatography (ethyl acetate/hexane and then methanol/ethyl acetate) to obtain the title compound (2.23 g).
MS: $[M+H]^+$ 833.4.

F) 4-[[N,N'-Bis(tert-butoxycarbonyl)carbamimidoyl]amino]benzoic acid

4-Aminobenzoic acid (4.42 g) and triethylamine (13.5 ml) were added to a solution of N,N'-bis(tert-butoxycarbonyl)-1H-pyrazole-1-carboxamidine (5.00 g) in N-methyl-2-pyrrolidone (30 ml), followed by stirring at room temperature for 5 days. The reaction mixture was rendered acidic with 1 M hydrochloric acid, followed by extraction with ethyl acetate. The organic layer was washed with 0.1 M hydrochloric acid and water, then dried over anhydrous magnesium sulfate, and concentrated under reduced pressure. The residue was suspended in ethyl acetate/hexane, and the precipitates were then collected by filtration and washed with hexane to obtain the title compound (4.90 g).
MS: $[M+H]^+$ 380.3.

G) Di-tert-butyl (2R)-2-[[3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-[[(1R)-3-tert-butoxy-1-tert-butoxycarbonyl-3-oxopropyl]carbamoyl]phenoxy]ethoxy]ethoxy]benzoyl]amino]b utanedioate

N,N-Diisopropylethylamine (3.61 ml) was added to a mixture of di-tert-butyl (2R)-2-[[3-[2-[2-[3-[[(1R)-3-tert-butoxy-1-tert-butoxycarbonyl-3-oxopropyl]carbamoyl]-5-hydroxyphenoxy]ethoxy]ethoxy]-5-hydroxybenzoyl]amino]butanedioate (3.51 g), 4-[[N,N'-bis(tert-butoxycarbonyl)carbamimidoyl]amino]benzoic acid (3.84 g), HATU (3.85 g), DMAP (0.0257 g), and N,N-dimethylformamide (35 ml), followed by stirring at room temperature for 72 hours. Water was added to the reaction mixture, and the resulting precipitates were then collected by filtration and washed with water to obtain the title compound (6.91 g).
$^1$H NMR (300 MHz, DMSO-$d_6$) $\delta$ 1.37 (18H, s), 1.39 (18H, s), 1.43 (18H, s), 1.52 (18H, s), 2.58-2.69 (2H, m), 2.74-2.85 (2H, m), 3.82-3.90 (4H, m), 4.17-4.26 (4H, m), 4.64-4.75 (2H, m), 7.14 (2H, s), 7.32 (2H, s), 7.37 (2H, s), 7.82 (4H, br d, J = 8.8 Hz), 8.09 (4H, br d, J = 8.0 Hz), 8.81 (2H, br d, J = 8.3 Hz), 10.25 (2H, s), 11.21 (2H, s).

H) (2R)-2-[[3-[2-[2-[3-[[(1R)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid dihydrochloride

Di-tert-butyl (2R)-2-[[3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-[[(1R)-3-tert-butoxy-1-tert-butoxycarbonyl-3-oxopropyl]carbamoyl]phenoxy]ethoxy]ethoxy]benzoyl]amino]b utanedioate (6.53 g) was dissolved in trifluoroacetic acid (40 mL) and then stirred at room temperature for 2 hours. The reaction mixture was poured into 4 M hydrochloric acid in ethyl acetate (100 ml), and the resulting precipitates were then collected by filtration and washed with ethyl acetate to obtain a solid (4.05 g). The obtained solid (503 mg) was dissolved in a solvent mixture of acetone (1.5 ml) and 2 M hydrochloric acid (1.5 ml), and acetone (12 ml) was then added thereto, followed by stirring at room temperature for 45 hours. The resulting precipitates were collected by filtration, washed with acetone, and then dried in air to obtain the title compound (397 mg).
$^1$H NMR (300 MHz, DMSO-d6) $\delta$ 2.67 (2H, dd, J = 16.4, 7.9 Hz), 2.83 (2H, dd, J = 16.4, 6.1 Hz), 3.86 (4H, br s), 4.16-4.29 (4H, m), 4.67-4.76 (2H, m), 7.13(2H, t, J = 2.1 Hz), 7.34 (2H, s), 7.43 (6H, d, J = 8.6 Hz), 7.86 (8H, br s), 8.16 (4H, d, J = 8.7 Hz), 8.81 (2H, br d, J = 7.8 Hz), 9.90-11.04 (2H, m), 12.42-13.27 (3H, m).

Example 16

(2R)-2-[[3-[2-[3-[[(1R)-1,2-Dicarboxyethyl]carbamoyl]-5-(4-Guanidinobenzoyl)oxyphenoxy]ethoxy]-5-(4-guanidinobenzoyl)oxybenzoyl]amino]butanedioic acid dihydrochloride

A) Di-tert-butyl (2R)-2-[(3-benzyloxy-5-hydroxybenzoyl)amino]butanedioate

1-Hydroxybenzotriazole monohydrate (3.26 g) was added to a mixture of 3-benzyloxy-5-hydroxybenzoic acid (4.33 g), di-tert-butyl (2R)-2-aminosuccinate hydrochloride (5.00 g), 1-ethyl-3-(3-dimethylaminopropyl)carbodiimide hydrochloride (4.08 g), triethylamine (1.80 g), and N,N-dimethylformamide (80 ml), followed by stirring at room temperature for 16 hours. Ethyl acetate and water were added to the reaction mixture, followed by extraction with ethyl acetate. The organic layer was washed with water, a saturated sodium hydrogen carbonate aqueous solution, and a saturated saline solution, then dried over anhydrous magnesium sulfate, and concentrated under reduced pressure. The residue was purified by silica gel column chromatography (ethyl acetate/hexane) to obtain the title compound (7.60 g).
$^1$H NMR (300 MHz, $CDCl_3$) $\delta$ 1.42-1.46 (9H, m), 1.48 (9H, s), 2.78-3.02 (2H, m), 4.79-4.89 (1H, m), 5.02-5.07 (2H, m), 6.48-6.52 (1H, m), 6.60-6.65 (1H, m), 6.95-6.98 (1H, m), 7.04 (1H, dd, J = 2.2, 1.4 Hz), 7.13-7.25 (2H, m), 7.31-7.47 (5H, m).

B) Di-tert-butyl (2R)-2-[[3-benzyloxy-5-[2-[3-benzyloxy-5-[[(1R)-3-tert-butoxy-1-tert-butoxycarbonyl-3-oxopropyl]carbamoyl]phenoxy]ethoxy]benzoyl]amino]butanedi oate

Cyanomethylenetributylphosphorane (2.30 g) was added to a mixture of di-tert-butyl (2R)-2-[(3-benzyloxy-5-hydroxybenzoyl)amino]butanedioate (3.00 g), ethylene glycol (0.197 g), and toluene (20 ml) at room temperature, followed by stirring at 100°C for 2 hours and subsequent concentration under reduced pressure. The residue was purified by silica gel column chromatography (ethyl acetate/hexane) to obtain the title compound (0.510 g).
MS: $[M+Na]^+$ 991.5.

C) Di-tert-butyl (2R)-2-[[3-[2-[3-[[(1R)-3-tert-butoxy-1-tert-butoxycarbonyl-3-oxo-propyl]carbamoyl]-5-hydroxyphenoxy]ethoxy]-5-hydroxybenzoyl]amino]butanedioate

A mixture of di-tert-butyl (2R)-2-[[3-benzyloxy-5-[2-[3-benzyloxy-5-[[(1R)-3-tert-butoxy-1-tert-butoxycarbonyl-3-oxopropyl]carbamoyl]phenoxy]ethoxy]benzoyl]amino]butanedi oate (0.510 g), 10% palladium on carbon (water content: about 55%, 0.200 g), and ethyl acetate (20 ml) was stirred overnight under a hydrogen atmosphere at room temperature, and the catalyst was then removed by filtration, followed by concentration under reduced pressure. The residue was purified by silica gel column chromatography (ethyl acetate/hexane) to obtain the title compound (0.420 g).
MS: $[M+H]^+$ 789.5.

D) Di-tert-butyl (2R)-2-[[3-[4-[[N,N'-bis(tert-butoxycarbonyl)carbamimidoyl]amino]benzoyl]oxy-5-[2-[3-[4-[[N,N'-bis(tert-butoxycarbonyl)carbamimidoyl]amino]benzoyl]oxy-5-[[(1R)-3-tert-butoxy-1-tert-butoxycarbonyl-3-oxopropyl]carbamoyl]phenoxy]ethoxy]benzoyl]amino]butanedi oate

4-[[N,N'-Bis(tert-butoxycarbonyl)carbamimidoyl]amino]benzoic acid (0.606 g), HATU (0.607 g), and N,N-diisopropylethylamine (0.413 g) were added to a mixture of di-tert-butyl (2R)-2-[[3-[2-[3-[[(1R)-3-tert-butoxy-1-tert-butoxycarbonyl-3-oxopropyl]carbamoyl]-5-hydroxyphenoxy]ethoxy]-5-hydroxybenzoyl]amino]butanedioate (0.420 g) and N,N-dimethylformamide (4.20 ml), followed by stirring overnight at room temperature. Water was added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (0.300 g).
MS: $[M+H]^+$ 1512.0.

E) (2R)-2-[[3-[2-[3-[[(1R)-1,2-Dicarboxyethyl]carbamoyl]-5-(4-guanidinobenzoyl)oxyphenoxy]ethoxy]-5-(4-guanidinobenzoyl)oxybenzoyl]amino]butanedioic acid dihydrochloride

A solution of di-tert-butyl (2R)-2-[[3-[4-[[N,N'-bis(tert-butoxycarbonyl)carbamimidoyl]amino]benzoyl]oxy-5-[2-[3-[4-[[N,N'-bis(tert-butoxycarbonyl)carbamimidoyl]amino]benzoyl]oxy-5-[[(1R)-3-tert-butoxy-1-tert-butoxycarbonyl-3-oxopropyl]carbamoyl]phenoxy]ethoxy]benzoyl]amino]butanedi oate (300 mg) in trifluoroacetic acid (2.00 ml) was stirred at room temperature for 2 hours and then concentrated under reduced pressure. A solution of 4 M hydrochloric acid in ethyl acetate (10 ml) was added to the residue, and the resulting solid was then collected by filtration to obtain

the title compound (190 mg).
$^1H$ NMR (300 MHz, DMSO-$d_6$) δ 2.70 (2H, br dd, J = 16.5, 8.4 Hz), 2.85 (2H, dd, J = 16.5, 5.5 Hz), 4.45 (4H, br s), 4.70-4.80 (2H, m), 7.20 (2H, s), 7.36-7.50 (8H, m), 7.83 (8H, br s), 8.17 (4H, d, J = 8.4 Hz), 8.87 (2H, br d, J = 7.8 Hz), 10.33-10.47 (2H, m), 12.60 (3H, br d, J = 4.4 Hz).

Example 17

(2S)-2-[[3-[2-[2-[3-[[(1S)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid dihydrochloride

A solution of di-tert-butyl (2S)-2-[[3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-[[(1S)-3-tert-butoxy-1-tert-butoxycarbonyl-3-oxopropyl]carbamoyl]phenoxy]ethoxy]ethoxy]benzoyl]amino]b utanedioate (0.410 g) synthesized in C) of Example 8 in trifluoroacetic acid (2.00 ml) was stirred at room temperature for 2 hours and then concentrated under reduced pressure. A solution of 4 M hydrochloric acid in ethyl acetate (10 ml) was added to the residue, and the resulting solid was then collected by filtration to obtain the title compound (0.240 g).
$^1H$ NMR (300 MHz, DMSO-$d_6$) δ 2.65-2.90 (4H, m), 3.87 (4H, br s), 4.23 (4H, br s), 4.69-4.80 (2H, m), 7.14 (2H, s), 7.35 (2H, s), 7.39-7.49 (6H, m), 7.87 (8H, br s), 8.16 (4H, d, J = 8.4 Hz), 8.87 (2H, br d, J = 7.8 Hz), 10.53 (2H, s), 12.41-12.78 (3H, m).

Example 20

2-[[3-(4-Guanidinobenzoyl)oxy-5-[2-[2-[3-(4-guanidinobenzoyl)oxy-5-(2-sulfoethylcarbamoyl)phenoxy]ethoxy]ethoxy]benzoyl]amino]e thanesulfonic acid ditrifluoroacetate

A) 3-[2-[2-(3-Carboxy-5-hydroxyphenoxy)ethoxy]ethoxy]-5-hydroxybenzoic acid

A mixture of 3-benzyloxy-5-[2-[2-(3-benzyloxy-5-carboxyphenoxy)ethoxy]ethoxy]benzoic acid (7.10 g) synthesized in C) of Example 15, 10% palladium on carbon (water content: about 55%, 0.7 g), and ethyl acetate (70 ml) was stirred overnight under a hydrogen atmosphere at room temperature. The catalyst was removed by filtration, followed by concentration under reduced pressure to obtain the title compound (4.50 g).
MS: $[M+H]^+$ 379.2.

B) Benzyl 3-[2-[2-(3-benzyloxycarbonyl-5-hydroxyphenoxy)ethoxy]ethoxy]-5-hydroxybenzoate

Benzyl bromide (2.51 ml) was added to a mixture of 3-[2-[2-(3-carboxy-5-hydroxyphenoxy)ethoxy]ethoxy]-5-hydroxybenzoic acid (4.00 g), diisopropylethylamine (4.10 g), and acetonitrile (40 ml), followed by stirring overnight at room temperature. The reaction mixture was concentrated under reduced pressure, and the residue was purified by silica gel column chromatography (ethyl acetate/hexane) to obtain the title compound (4.32 g).
MS: $[M+Na]^+$ 581.3.

C) Benzyl 3-[2-[2-[3-benzyloxycarbonyl-5-[4-[[N,N'-bis(tert-butoxycarbonyl)carbamimidoyl]amino]benzoyl]oxyphenoxy]eth oxy]ethoxy]-5-[4-[[N,N'-bis(tert-butoxycarbonyl)carbamimidoyl]amino]benzoyl]oxybenzoate

4-[[N,N'-Bis(tert-butoxycarbonyl)carbamimidoyl]amino]benzoic acid (3.26 g), HATU (3.276 g), DMAP (0.0219 g), and N,N-diisopropylethylamine (2.31 g) were added to a mixture of benzyl 3-[2-[2-(3-benzyloxycarbonyl-5-hydroxyphenoxy)ethoxy]ethoxy]-5-hydroxybenzoate (2.00 g) and N,N-dimethylformamide (20 ml), followed by stirring overnight at room temperature. Water was added to the reaction mixture, and the resulting solid was collected by filtration to obtain the title compound (3.59 g).
MS: $[M+H]^+$ 1281.6.

D) 3-[4-[[(N,N'-Bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-carboxyphenoxy]ethoxy]ethoxy]benzoic acid

A mixture of benzyl 3-[2-[2-[3-benzyloxycarbonyl-5-[4-[[N,N'-bis(tert-butoxycarbonyl)carbamimidoyl]amino]benzoyl]oxyphenoxy]eth oxy]ethoxy]-5-[4-[[N,N'-bis(tert-butoxycarbonyl)carbamimidoyl]amino]benzoyl]oxybenzoate (3.59 g), 10% palladium on carbon (water content: about 55%, 0.55 g), and ethyl acetate (50 ml) was stirred overnight under a hydrogen atmosphere at room temperature, and the catalyst was then removed by filtration, followed

by concentration under reduced pressure. The residue was purified by silica gel column chromatography (ethyl acetate/hexane) to obtain the title compound (3.09 g).
MS: $[M+H]^+$ 1101.4.

E) 2-[[3-(4-Guanidinobenzoyl)oxy-5-[2-[2-[3-(4-guanidinobenzoyl)oxy-5-(2-sulfoethylcarbamoyl)phenoxy]ethoxy]ethoxy]benzoyl]amino]e thanesulfonic acid ditrifluoroacetate

Taurine (0.0341 g), N,N-diisopropylethylamine (0.0704 g), and a solution of 50% propylphosphonic anhydride in ethyl acetate (0.16 mL) were added to a mixture of 3-[4-[[N,N'-bis(tert-butoxycarbonyl)carbamimidoyl]amino]benzoyl]oxy-5-[2-[2-[3-[4-[[N,N'-bis(tert-butoxycarbonyl)carbamimidoyl]amino]benzoyl]oxy-5-carboxyphenoxy]ethoxy]ethoxy]benzoic acid (0.100 g) and N,N-dimethylformamide (0.5 ml), followed by stirring overnight at room temperature. Taurine (0.0341 g), N,N-diisopropylethylamine (0.0704 g), and a solution of 50% propylphosphonic anhydride in ethyl acetate (0.16 mL) were added to the reaction mixture, followed by stirring overnight at room temperature. Trifluoroacetic acid (2.00 ml) was added to the reaction mixture, followed by stirring at room temperature for 2 hours and subsequent concentration under reduced pressure. The residue was purified by preparative HPLC (C18, mobile phase: water/acetonitrile (system containing 0.1% TFA)) to obtain the title compound (11.8 mg).
$^1H$ NMR (300 MHz, DMSO-$d_6$) $\delta$ 2.70 (4H, br t, J = 6.9 Hz), 3.49-3.55 (6H, m), 3.84 (4H, br s), 4.20 (4H, br s), 7.07 (2H, s), 7.22 (2H, s), 7.32 (2H, s), 7.41 (4H, br d, J = 8.3 Hz), 7.75 (8H, br s), 8.13 (4H, br d, J = 8.3 Hz), 8.55 (2H, br s), 10.08 (2H, s).

Example 21

(2R)-2-[[2-[2-[2-[2-[[(1R)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid dihydrochloride

A) Methyl 4-benzyloxy-2-hydroxybenzoate

Potassium carbonate (4.52 g) and benzyl bromide (5.59 g) were added to a mixture of methyl 2,4-dihydroxybenzoate (5.00 g) and acetone (125 ml), followed by stirring overnight at room temperature. Then, insoluble matter was removed by filtration. The filtrate was concentrated, and the residue was pulverized in water-methanol. Then, the solid was collected by filtration to obtain the title compound (6.80 g).
$^1H$ NMR (300 MHz, DMSO-$d_6$) $\delta$ 3.83-3.91 (3H, m), 5.17 (2H, s), 6.58-6.64 (2H, m), 7.31-7.51 (5H, m), 7.68-7.77 (1H, m), 10.77 (1H, s).

B) Methyl 4-benzyloxy-2-[2-[2-(5-benzyloxy-2-methoxycarbonylphenoxy)ethoxy]ethoxy]benzoate

Potassium carbonate (3.21 g) was added to a mixture of methyl 4-benzyloxy-2-hydroxybenzoate (2.00 g), diethylene glycol bis(p-toluenesulfonate) (1.60 g), and N,N-dimethylformamide (10 ml) at room temperature, followed by stirring at 50°C for 6 hours. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.20 g).
MS: $[M+Na]^+$ 609.3.

C) 4-Benzyloxy-2-[2-[2-(5-benzyloxy-2-carboxyphenoxy)ethoxy]ethoxy]benzoic acid

2 M sodium hydroxide aqueous solution (15 ml) was added to a mixture of methyl 4-benzyloxy-2-[2-[2-(5-benzyloxy-2-methoxycarbonylphenoxy)ethoxy]ethoxy]benzoate (2.20 g) and water-tetrahydrofuran (v/v = 1/1, 40 ml), followed by stirring overnight at room temperature and subsequent concentration under reduced pressure. The residue was neutralized with 1 M hydrochloric acid, followed by extraction with ethyl acetate. The organic layer was washed with a saturated saline solution, then dried over anhydrous magnesium sulfate, and concentrated under reduced pressure. The residue was crystallized from tetrahydrofuran-ethyl acetate-hexane, and the precipitated compound was washed with ethyl acetate-hexane (v/v = 1/1) to obtain the title compound (1.70 g).
MS: $[M+Na]^+$ 581.3.

D) Di-tert-butyl (2R)-2-[[4-benzyloxy-2-[2-[2-[5-benzyloxy-2-[[(1R)-3-tert-butoxy-1-tert-butoxycarbonyl-3-oxopropyl]carbamoyl]phenoxy]ethoxy]ethoxy]benzoyl]amino]b utanedioate

N,N-Diisopropylethylamine (2.04 ml) was added to a mixture of 4-benzyloxy-2-[2-[2-(5-benzyloxy-2-carboxyphenoxy)ethoxy]ethoxy]benzoic acid (1.31 g), di-tert-butyl (2R)-2-aminosuccinate hydrochloride (1.45 g), HATU (1.96 g), and N,N-dimethylformamide (15 ml), followed by stirring overnight at room temperature. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.46 g). MS: $[M+H]^+$ 1013.5.

E) Di-tert-butyl (2R)-2-[[4-[4-[[N,N'-bis(tert-butoxycarbonyl)carbamimidoyl]amino]benzoyl]oxy-2-[2-[2-[5-[4-[[N,N'-bis(tert-butoxycarbonyl)carbamimidoyl]amino]benzoyl]oxy-2-[[(1R)-3-tert-butoxy-1-tert-butoxycarbonyl-3-oxopropyl]carbamoyl]phenoxy]ethoxy]ethoxy]benzoyl]amino]b utanedioate

A mixture of di-tert-butyl (2R)-2-[[4-benzyloxy-2-[2-[2-[5-benzyloxy-2-[[(1R)-3-tert-butoxy-1-tert-butoxycarbonyl-3-oxopropyl]carbamoyl]phenoxy]ethoxy]ethoxy]benzoyl]amino]b utanedioate (1.31 g), 10% palladium on carbon (water content: about 55%, 0.430 g), ethanol (15 ml), and tetrahydrofuran (15 ml) was stirred under a hydrogen atmosphere at room temperature for 3.5 hours. Then, the mixture was filtered, and the residue was washed with ethyl acetate to obtain a solid. The filtrate was concentrated under reduced pressure, and the residue was then washed with diisopropyl ether to obtain a solid. N,N-Diisopropylethylamine (1.10 ml) was added to a mixture of the obtained solid, 4-[[N,N'-bis(tert-butoxycarbonyl)carbamimidoyl]amino]benzoic acid (1.05 g) produced in F) of Example 15, HATU (1.18 g), DMAP (7.9 mg), and N,N-dimethylformamide (20 ml), followed by stirring overnight at room temperature. 4-[[N,N'-Bis(tert-butoxycarbonyl)carbamimidoyl]amino]benzoic acid (0.489 g), HATU (0.736 g), and N,N-diisopropylethylamine (0.442 ml) were added to the reaction mixture, followed by stirring overnight at room temperature. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.18 g).
$^1$H NMR (300 MHz, DMSO-$d_6$) $\delta$ 1.35 (18H, s), 1.38 (18H, s), 1.43 (18H, s), 1.52 (18H, s), 2.71-2.80 (4H, m), 3.94-4.03 (4H, m), 4.35 (4H, br s), 4.69-4.80 (2H, m), 7.02 (2H, dd, J = 8.6, 1.8 Hz), 7.16 (2H, d, J = 1.7 Hz), 7.82 (4H, d, J = 8.8 Hz), 7.98 (2H, d, J = 8.6 Hz), 8.08 (4H, d, J = 8.7 Hz), 8.65 (2H, d, J = 7.6 Hz), 10.26 (2H, s), 11.21 (2H, s).

F) (2R)-2-[[2-[2-[2-[2-[[(1R)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid dihydrochloride

A mixture of di-tert-butyl (2R)-2-[[4-[4-[[N,N'-bis(tert-butoxycarbonyl)carbamimidoyl]amino]benzoyl]oxy-2-[2-[2-[5-[4-[[N,N'-bis(tert-butoxycarbonyl)carbamimidoyl]amino]benzoyl]oxy-2-[[(1R)-3-tert-butoxy-1-tert-butoxycarbonyl-3-oxopropyl]carbamoyl]phenoxy]ethoxy]ethoxy]benzoyl]amino]b utanedioate (2.20 g) and trifluoroacetic acid (50 mL) was stirred overnight at room temperature, and the reaction mixture was then concentrated under reduced pressure. 4 M hydrochloric acid in ethyl acetate (50 ml) was added to the residue, followed by stirring at room temperature for 5 hours. Then, the resulting precipitates were collected by filtration and washed with ethyl acetate to obtain a solid (1.55 g). The obtained solid (103.4 mg) was dissolved in a solvent mixture of 2 M hydrochloric acid (0.2 ml) and acetone (1.6 ml). Since the clear solution turned to a white suspension, additional two drops of 2 M hydrochloric acid were added thereto. The reaction mixture was stirred at room temperature for 3 hours and then left for 2 weeks. The resulting precipitates were collected by filtration, washed with acetone, and then dried in air to obtain the title compound (59.8 mg).
$^1$H NMR (300 MHz, DMSO-d6) $\delta$ 2.82 (4H, br d, J = 4.1 Hz), 3.97 (4H, br s), 4.30 (4H, br d, J = 4.1 Hz), 4.72-4.87 (2H, m), 7.00 (2H, dd, J = 8.6, 2.0 Hz), 7.16 (2H, d, J = 1.9 Hz), 7.43 (4H, d, J = 8.7 Hz), 7.77 (8H, s), 8.00 (2H, d, J = 8.5 Hz), 8.15 (4H, d, J = 8.7 Hz), 8.72 (2H, d, J = 7.6 Hz), 10.19 (2H, s), 12.60-12.93 (3H, m).; Anal. Calcd for $C_{42}H_{44}N_8O_{17}Cl_2 \cdot 2.8H_2O$, C; 47.85, H; 4.74, N; 10.63. Found. C; 48.12, H; 4.99, N; 10.74.

Example 22

(2R)-2-[[2-[2-[2-[[(1R)-1,2-Dicarboxyethyl]carbamoyl]-5-(4-guanidinobenzoyl)oxyphenoxy]ethoxy]-4-(4-guanidinobenzoyl)oxybenzoyl]amino]butanedioic acid dihydrochloride

A) Methyl 4-benzyloxy-2-[2-(5-benzyloxy-2-methoxycarbonylphenoxy)ethoxy]benzoate

Potassium carbonate (8.03 g) was added to a mixture of methyl 4-benzyloxy-2-hydroxybenzoate (5.00 g)

produced in A) of Example 21, ethylene glycol bis(p-toluenesulfonate) (3.59 g), and N,N-dimethylformamide (50 ml) at room temperature, followed by stirring overnight at 50°C. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain a solid. The obtained solid was washed with diisopropyl ether to obtain the title compound (2.31 g).
$^1$H NMR (300 MHz, DMSO-$d_6$) δ 3.66 (6H, s), 4.38 (4H, s), 5.18 (4H, s), 6.70 (2H, dd, J = 8.7, 2.3 Hz), 6.84 (2H, d, J = 2.3 Hz), 7.28-7.51 (10H, m), 7.69 (2H, d, J = 8.7 Hz) .

B) 4-Benzyloxy-2-[2-(5-benzyloxy-2-carboxyphenoxy)ethoxy]benzoic acid

1 M sodium hydroxide aqueous solution (50 ml) was added to a mixture of methyl 4-benzyloxy-2-[2-(5-benzyloxy-2-methoxycarbonylphenoxy)ethoxy]benzoate (2.81 g), tetrahydrofuran (50 ml), and ethanol (50 ml) at room temperature, followed by stirring overnight at 50°C and subsequent concentration under reduced pressure. The residue was neutralized with 1 M hydrochloric acid, and the resulting precipitates were collected by filtration and then washed with water to obtain the title compound (2.81 g).
$^1$H NMR (300 MHz, DMSO-$d_6$) δ 4.39 (4H, s), 5.17 (4H, s), 6.69 (2H, dd, J = 8.7, 2.3 Hz), 6.81 (2H, d, J = 2.2 Hz), 7.30-7.50 (10H, m), 7.70 (2H, d, J = 8.6 Hz), 11.60-12.52 (2H, m).

C) Di-tert-butyl (2R)-2-[[4-benzyloxy-2-[2-[5-benzyloxy-2-[[(1R)-3-tert-butoxy-1-tert-butoxycarbonyl-3-oxopropyl]carbamoyl]phenoxy]ethoxy]benzoyl]amino]butanedi oate

N,N-Diisopropylethylamine (2.54 ml) was added to a mixture of 4-benzyloxy-2-[2-(5-benzyloxy-2-carboxyphenoxy)ethoxy]benzoic acid (1.50 g), di-tert-butyl (2R)-2-aminosuccinate hydrochloride (1.81 g), HATU (2.44 g), and N,N-dimethylformamide (15 ml), followed by stirring overnight at room temperature. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (2.45 g).
MS: $[M+H]^+$ 969.4.

D) Di-tert-butyl (2R)-2-[[2-[2-[2-[[(1R)-3-tert-butoxy-1-tert-butoxycarbonyl-3-oxopropyl]carbamoyl]-5-hydroxyphenoxy]ethoxy]-4-hydroxybenzoyl]amino]butanedioate

A mixture of di-tert-butyl (2R)-2-[[4-benzyloxy-2-[2-[5-benzyloxy-2-[[(1R)-3-tert-butoxy-1-tert-butoxycarbonyl-3-oxopropyl]carbamoyl]phenoxy]ethoxy]benzoyl]amino]butanedi oate (2.45 g), 10% palladium on carbon (water content: about 55%, 0.400 g), ethanol (50 ml), and tetrahydrofuran (50 ml) was stirred under a hydrogen atmosphere at room temperature for 6 hours, and the catalyst was then removed by filtration. The filtrate was concentrated under reduced pressure. A mixture of the residue, 10% palladium on carbon (water content: about 55%, 1.00 g), ethanol (50 ml), and tetrahydrofuran (50 ml) was stirred overnight under a hydrogen atmosphere at room temperature, and the catalyst was then removed by filtration. The filtrate was concentrated under reduced pressure to obtain the title compound (1.96 g).
MS: $[M+H]^+$ 789.4.

E) Di-tert-butyl (2R)-2-[[4-[4-[[N,N'-bis(tert-butoxycarbonyl)carbamimidoyl]amino]benzoyl]oxy-2-[2-[2-[5-[4-[[N,N'-bis(tert-butoxycarbonyl)carbamimidoyl]amino]benzoyl]oxy-2-[[(1R)-3-tert-butoxy-1-tert-butoxycarbonyl-3-oxopropyl]carbamoyl]phenoxy]ethoxy]ethoxy]benzoyl]amino]b utanedioate

HATU (2.27 g) was added to a mixture of di-tert-butyl (2R)-2-[[2-[2-[2-[[(1R)-3-tert-butoxy-1-tert-butoxycarbonyl-3-oxopropyl]carbamoyl]-5-hydroxyphenoxy]ethoxy]-4-hydroxybenzoyl]amino]butanedioate (1.96 g), 4-[[N,N'-bis(tert-butoxycarbonyl)carbamimidoyl]amino]benzoic acid (2.07 g) produced in F) of Example 15, N,N-diisopropylethylamine (2.13 ml), DMAP (15.2 mg), and N,N-dimethylformamide (20 ml) at room temperature, followed by stirring overnight at room temperature. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was washed with diisopropyl ether to obtain the title compound (3.57 g).
$^1$H NMR (300 MHz, DMSO-$d_6$) δ 1.27 (18H, s), 1.28 (18H, s), 1.43 (18H, s), 1.52 (18H, s), 2.71-2.76 (4H, m), 4.65-4.75 (6H, m), 7.01-7.12 (2H, m), 7.18 (2H, s), 7.84 (4H, d, J = 8.9 Hz), 7.97 (2H, d, J = 8.7 Hz), 8.10 (4H, d, J = 9.0 Hz), 8.68 (2H, d, J = 7.8 Hz), 10.26 (2H, s), 11.19 (2H, s).

F) (2R)-2-[[2-[2-[2-[[(1R)-1,2-Dicarboxyethyllcarbamoyl]-5-(4-guanidinobenzoyl)oxyphenoxy]ethoxy]-4-(4-guanidinobenzoyl)oxybenzoyl]amino]butanedioic acid dihydrochloride

A mixture of di-tert-butyl (2R)-2-[[4-[4-[[N,N'-bis(tert-butoxycarbonyl)carbamimidoyl]amino]benzoyl]oxy-2-[2-[2-[5-[4-[[N,N'-bis(tert-butoxycarbonyl)carbamimidoyl]amino]benzoyl]oxy-2-[[(1R)-3-tert-butoxy-1-tert-butoxycarbonyl-3-oxopropyl]carbamoyl]phenoxy]ethoxy]ethoxy]benzoyl]amino]b utanedioate (3.57 g) and trifluoroacetic acid (50 mL) was stirred overnight at room temperature, and the reaction mixture was then concentrated under reduced pressure. 4 M hydrochloric acid in ethyl acetate (50 ml) was added to a mixture of the residue and ethyl acetate (5 mL), followed by stirring at room temperature for 4 hours. The resulting precipitates were collected by filtration and washed with ethyl acetate to obtain the title compound (2.43 g).
$^1$H NMR (300 MHz, DMSO-$d_6$) δ 2.73 (4H, dd, J = 3.6, 1.7 Hz), 4.59 (4H, br s), 4.70-4.82 (2H, m), 7.05 (2H, dd, J = 8.6, 2.0 Hz), 7.24 (2H, d, J = 1.8 Hz), 7.44 (4H, d, J = 8.8 Hz), 7.77 (8H, s), 7.95 (2H, d, J = 8.5 Hz), 8.18 (4H, d, J = 8.7 Hz), 8.65 (2H, d, J = 7.7 Hz), 10.19 (2H, s), 12.52-12.82 (3H, m).

Example 23

(2S)-2-[[2-[2-[2-[2-[[(1S)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid dihydrochloride

A) Di-tert-butyl (2S)-2-[[4-benzyloxy-2-[2-[2-[5-benzyloxy-2-[[(1S)-3-tert-butoxy-1-tert-butoxycarbonyl-3-oxopropyl]carbamoyl]phenoxy]ethoxy]ethoxy]benzoyl]amino]b utanedioate

N,N-Diisopropylethylamine (2.49 ml) was added to a mixture of 4-benzyloxy-2-[2-[2-(5-benzyloxy-2-carboxyphenoxy)ethoxy]ethoxy]benzoic acid (1.00 g) produced in C) of Example 21, di-tert-butyl L-aspartate hydrochloride (1.26 g), HATU (1.70 g), and N,N-dimethylformamide (10 ml), followed by stirring at room temperature for 60 hours. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified by silica gel column chromatography (ethyl acetate/hexane) to obtain the title compound (0.587 g).
MS: $[M+H]^+$ 1013.8.

B) Di-tert-butyl (2S)-2-[[4-[4-[[N,N'-bis(tert-butoxycarbonyl)carbamimidoyl]amino]benzoyl]oxy-2-[2-[2-[5-[4-[[N,N'-bis(tert-butoxycarbonyl)carbamimidoyl]amino]benzoyl]oxy-2-[[(1S)-3-tert-butoxy-1-tert-butoxycarbonyl-3-oxopropyl]carbamoyl]phenoxy]ethoxy]ethoxy]benzoyl]amino]b utanedioate

A mixture of di-tert-butyl (2S)-2-[[4-benzyloxy-2-[2-[2-[5-benzyloxy-2-[[(1S)-3-tert-butoxy-1-tert-butoxycarbonyl-3-oxopropyl]carbamoyl]phenoxy]ethoxy]ethoxy]benzoyl]amino]b utanedioate (586.5 mg), 10% palladium on carbon (water content: about 55%, 250.0 mg), ethanol (10 ml), and tetrahydrofuran (10 ml) was stirred under a hydrogen atmosphere at room temperature for 3.5 hours and then concentrated under reduced pressure. N,N-Diisopropylethylamine (0.495 ml) was added to a mixture of the residue, 4-[[N,N'-bis(tert-butoxycarbonyl)carbamimidoyl]amino]benzoic acid (0.483 g) produced in F) of Example 15, DMAP (7.1 mg), HATU (0.528 g), and N,N-dimethylformamide (20 ml), followed by stirring overnight at room temperature. 4-[[N,N'-Bis(tert-butoxycarbonyl)carbamimidoyl]amino]benzoic acid (0.220 g), HATU (0.220 g), and N,N-diisopropylethylamine (0.198 ml) were added to the reaction mixture, followed by stirring overnight at room temperature. Water and ethyl acetate were added to the reaction mixture, followed by extraction with ethyl acetate. The organic layer was washed with a saturated saline solution, then dried over anhydrous magnesium sulfate, and concentrated under reduced pressure. The residue was purified twice by silica gel column chromatography (ethyl acetate/hexane) to obtain the title compound (0.498 g).
$^1$H NMR (300 MHz, DMSO-$d_6$) δ 1.35 (18H, s), 1.38 (18H, s), 1.43 (18H, s), 1.52 (18H, s), 2.66-2.85 (4H, m), 3.93-4.04 (4H, m), 4.34 (4H, br d, J = 4.5 Hz), 4.70-4.81 (2H, m), 7.02 (2H, dd, J = 8.6, 1.8 Hz), 7.16 (2H, d, J = 1.6 Hz), 7.82 (4H, d, J = 8.8 Hz), 7.97 (2H, d, J = 8.5 Hz), 8.08 (4H, d, J = 8.7 Hz), 8.65 (2H, d, J = 7.8 Hz), 10.25 (2H, s), 11.20 (2H, s).

C) (2S)-2-[[2-[2-[2-[2-[[(1S)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid dihydrochloride

A mixture of di-tert-butyl (2S)-2-[[4-[4-[[N,N'-bis(tert-butoxycarbonyl)carbamimidoyl]amino]benzoyl]oxy-2-[2-[2-[5-[4-[[N,N['-bis(tert-butoxycarbonyl)carbamimidoyl]amino]benzoyl]oxy-2-[[(1S)-3-tert-butoxy-1-tert-butoxycarbonyl-3-oxopropyl]carbamoyl]phenoxy]ethoxy]ethoxy]benzoyl]amino]b utanedioate (498.3 mg) and trifluoroacetic acid (10 mL) was stirred overnight at room temperature, and the reaction mixture was then concentrated under reduced

pressure. 4 M hydrochloric acid in ethyl acetate (10 ml) was added to the residue, followed by stirring at room temperature for 3 hours. Then, the resulting precipitates were collected by filtration and washed with ethyl acetate to obtain a solid. The obtained solid was dissolved in a mixed solution of 2 M hydrochloric acid and acetone (v/v = 1/8) and stirred overnight at room temperature. The resulting precipitates were collected by filtration, washed with acetone, and then dried in air to obtain the title compound (195.1 mg).

$^1$H NMR (300 MHz, DMSO-d6) δ 2.78-2.86 (4H, m), 3.94-4.00 (4H, m), 4.27-4.34 (4H, m), 4.76-4.84 (2H, m), 7.00 (2H, dd, J = 8.6, 2.0 Hz), 7.16 (2H, d, J = 2.0 Hz), 7.43 (4H, d, J = 8.7 Hz), 7.78 (8H, s), 7.99 (2H, d, J = 8.5 Hz), 8.15 (4H, d, J = 8.6 Hz), 8.71 (2H, d, J = 7.6 Hz), 10.25 (2H, s), 12.63-12.84 (3H, m).

Compounds of Examples 2, 3, 9 to 11, 13, 14, 18, and 19 in the following tables were produced by the methods in Examples described above or in accordance with the methods. The example compounds are shown in the following Tables 2 and 3. The column "MS" in the tables shows measured values.

[Table 2]

| Ex. No. | IUPAC name | Salt | MS |
|---|---|---|---|
| **1** | (2S)-2-[[3-[2-[2-[2-[3-[[(1S)-1,2-Dicarboxyethyl]carbamoyl]-5-(4-guanidinobenzoyl)oxyphenoxy]ethoxy]ethoxy]ethoxy]-5-(4-guanidinobenzoyl) oxybenzoyl]amino]butanedioic acid | 2CF3COOH | 975.3 |
| **2** | (2S)-2-[[3-[2-[2-[2-[2-[2-[3-[[(1S)-1,2-Dicarboxyethyl]carbamoyl]-5-(4-guanidinobenzoyl)oxyphenoxy]ethoxy]ethoxy]ethoxy]ethoxy]ethoxy]-5-(4-guanidinobenzoyl)oxybenzoyl]amino]butanedioic acid | 2CF3CO2H | 1063.5 |
| **3** | (2S)-2-[[3-[2-[2-[2-[2-[2-[2-[2-[3-[[(1S)-1,2-Dicarboxyethyl]carbamoyl]-5-(4-guanidinobenzoyl)oxyphenoxy]ethoxy]ethoxy]ethoxy]ethoxy]ethoxy]ethoxy] ethoxy]-5-(4-guanidinobenzoyl)oxybenzoyl]amino]butanedioic acid | 2CF3COOH | 1151 |
| **4** | (2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl) oxyphenyl]propanoylamino]butanedioic acid | 2CF3COOH | 1031. 3 |
| **5** | 2-[[3-[2-[2-[2-[3-[Bis(carboxymethyl)carbamoyl]-5-(4-guanidinobenzoyl) oxyphenoxy]ethoxy]ethoxy]ethoxy]-5-(4-guanidinobenzoyl) oxybenzoyl]-(carboxymethyl)amino]acetic acid | 2CF3COOH | 975.3 |
| **6** | (2R)-2-[[3-[2-[2-[2-[3-[[(1R)-1,2-Dicarboxyethyl]carbamoyl]-5-(4-guanidinobenzoyl)oxyphenoxy]ethoxy]ethoxy]ethoxy]-5-(4-guanidinobenzoyl) oxybenzoyl]amino]butanedioic acid | 2CF3COOH | 975.5 |
| **7** | (2S)-2-[[3-(4-Carbamimidoylphenoxy)carbonyl-5-[2-[2-[2-[3-(4-carbamimidoylphenoxy)carbonyl-5-[[(1S)-1,2-dicarboxyethyl]carbamoyl] phenoxy]ethoxy]ethoxy]ethoxy]benzoyl]amino]butanedioic acid | 2CF3COOH | 945.3 |
| **8** | (2S)-2-[[3-[2-[2-[3-[[(1S)-1,2-Dicarboxyethyl]carbamoyl]-5-(4-guanidinobenzoyl) oxyphenoxy]ethoxy]ethoxy]-5-(4-guanidinobenzoyl)oxybenzoyl]amino] butanedioic acid | 2CF3COOH | 931.4 |
| **9** | (2S)-2-[[3-[2-[3-[[(1S)-1,2-Dicarboxyethyl]carbamoyl]-5-(4-guanidinobenzoyl) oxyphenoxy]ethoxy]-5-(4-guanidinobenzoyl)oxybenzoyl]amino]butanedioic acid | 2CF3COOH | 931.4 |
| **10** | (2R)-2-[[3-[2-[2-[2-[3-[[(1R)-1-Carboxy-2-hydroxyethyl]carbamoyl]-5-(4-guanidinobenzoyl)oxyphenoxy]ethoxy]ethoxy]ethoxy]-5-(4-guanidinobenzoyl) oxybenzoyl]amino]-3-hydroxypropanoic acid | 2CF3COOH | 919.3 |
| **11** | (2R)-6-Amino-2-[[3-[2-[2-[2-[3-[[(1R)-5-amino-1-carboxypentyl]carbamoyl]-5-(4-guanidinobenzoyl)oxyphenoxy]ethoxy]ethoxy]ethoxy]-5-(4-guanidinobenzoyl) oxybenzoyl]amino]hexanoic acid | 4CF3COOH | 975.4 |
| **12** | (2S)-2-[3-[2-[2-[2-[2-[2-[3-[[(1S)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl) oxyphenyl]propanoylamino]butanedioic acid | 2HCl | 1031. 4 |

(continued)

| Ex. No. | IUPAC name | Salt | MS |
|---|---|---|---|
| **13** | (2R)-2-[3-[2-[2-[2-[2-[3-[[ (1R)-1,2-Dicarboxyethyl]amino]-3-oxopropyl]-5-(4-guanidinobenzoyl)oxyphenoxy]ethoxy]ethoxy]-4-(4-guanidinobenzoyl)oxyphenyl] propanoylamino]butanedioic acid | 2CF3COOH | 987.4 |
| **14** | (2R)-2-[3-[2-[2-[2-[2-[2-[3-[[(1R)-1,2-Dicarboxyethyl]amino]-3-oxopropyl]-5-(4-guanidinobenzoyl)oxyphenoxy]ethoxy]ethoxy]ethoxy]-4-(4-guanidinobenzoyl) oxyphenyl]propanoylamino]butanedioic acid | 2CF3COOH | 1031. 5 |
| **15** | (2R)-2-[[3-[2-[2-[3-[[(1R)-1,2-Dicarboxyethyl]carbamoyl]-5-(4-guanidinobenzoyl) oxyphenoxy]ethoxy]ethoxy]-5-(4-guanidinobenzoyl)oxybenzoyl]amino] butanedioic acid | 2HCl | 931.4 |
| **16** | (2R)-2-[[3-[2-[3-[[(1R)-1,2-Dicarboxyethyl]carbamoyl]-5-(4-guanidinobenzoyl) oxyphenoxy]ethoxy]-5-(4-guanidinobenzoyl)oxybenzoyl]amino]butanedioic acid | 2HCl | 887.6 |
| **17** | (2S)-2-[[3-[2-[2-[3-[[(1S)-1,2-Dicarboxyethyl]carbamoyl]-5-(4-guanidinobenzoyl) oxyphenoxy]ethoxy]ethoxy]-5-(4-guanidinobenzoyl)oxybenzoyl]amino] butanedioic acid | 2HCl | 931.6 |
| **18** | (2R)-2-[[3-[2-[2-[3-[[(1R)-1,2-Dicarboxyethyl]carbamoyl]-5-(2-fluoro-4-guanidinobenzoyl)oxyphenoxy]ethoxy]ethoxy]-5-(2-fluoro-4-guanidinobenzoyl) oxybenzoyl]amino]butanedioic acid | 2CF3COOH | 967.3 |
| **19** | (2R)-2-[[2-[2-[2-[2-[[(1R)-1,2-Dicarboxyethyl]carbamoyl]-4-(4-guanidinobenzoyl) oxyphenoxy]ethoxy]ethoxy]-5-(4-guanidinobenzoyl)oxybenzoyl]amino] butanedioic acid | 2CF3COOH | 931.3 |
| **20** | 2-[[3-(4-Guanidinobenzoyl)oxy-5-[2-[2-[3-(4-guanidinobenzoyl)oxy-5-(2-sulfoethylcarbamoyl)phenoxy]ethoxy]ethoxy]benzoyl]amino]ethanesulfonic acid | 2CF3COOH | 916.5 |
| **21** | (2R)-2-[[2-[2-[2-[2-[[(1R)-1,2-Dicarboxyethyl]carbamoyl]-5-(4-guanidinobenzoyl) oxyphenoxy]ethoxy]ethoxy]-4-(4-guanidinobenzoyl)oxybenzoyl]amino] butanedioic acid | 2HCl | 931.4 |
| **22** | (2R)-2-[[2-[2-[2-[[(1R)-1,2-Dicarboxyethyl]carbamoyl]-5-(4-guanidinobenzoyl) oxyphenoxy]ethoxy]-4-(4-guanidinobenzoyl)oxybenzoyl]amino]butanedioic acid | 2HCl | 887.6 |
| **23** | (2S)-2-[[2-[2-[2-[2-[[(1S)-1,2-Dicarboxyethyl]carbamoyl]-5-(4-guanidinobenzoyl) oxyphenoxy]ethoxy]ethoxy]-4-(4-guanidinobenzoyl)oxybenzoyl]amino] butanedioic acid | 2HCl | 931.3 |

[Table 3]

| Example No. | Structural formula | | |
|---|---|---|---|
| 1 | 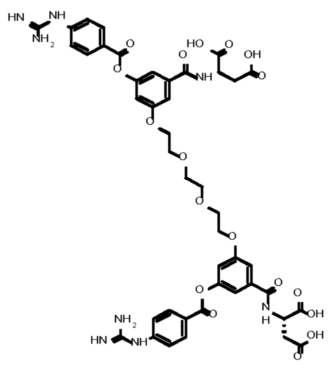 | 2 | 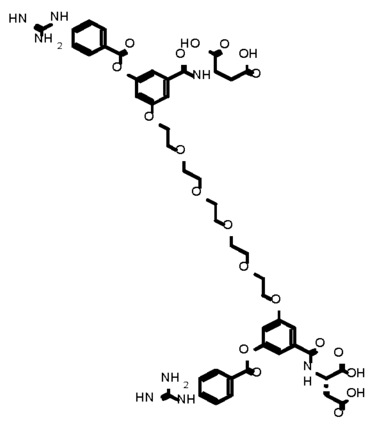 |

(continued)

| Example No. | Structural formula | | |
|---|---|---|---|
| 3 | 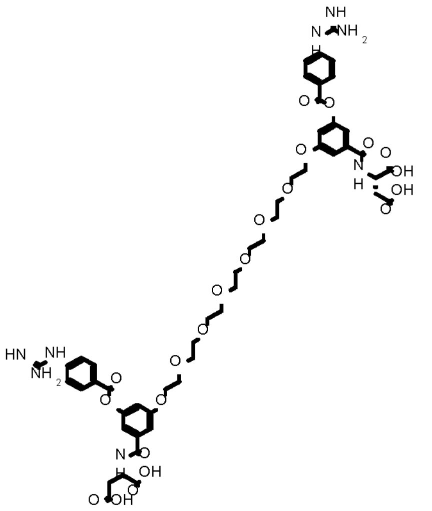 | 4 | 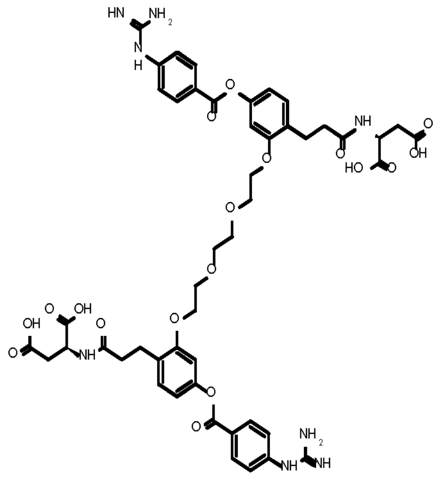 |
| 5 | 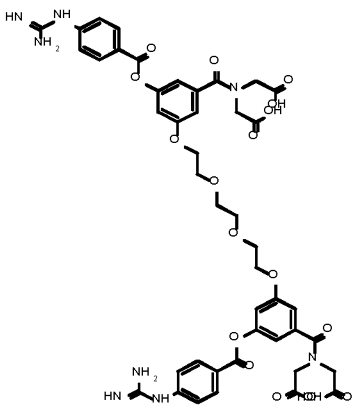 | 6 | 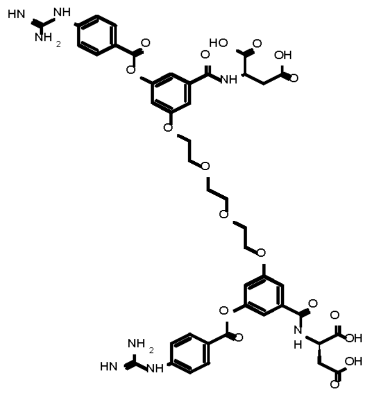 |
| 7 | 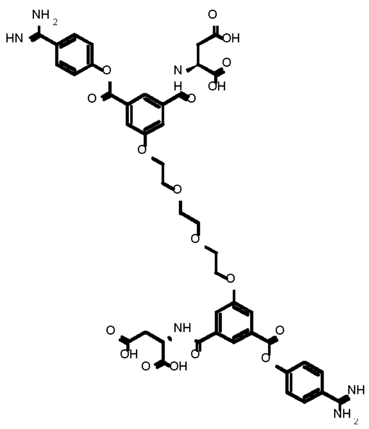 | 8 | 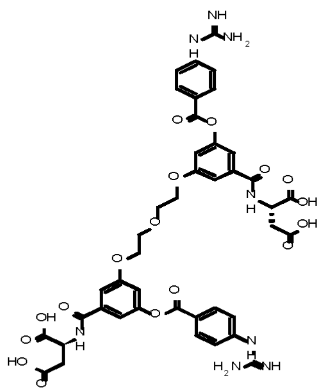 |

(continued)

| Example No. | Structural formula | | |
|---|---|---|---|
| 9 | 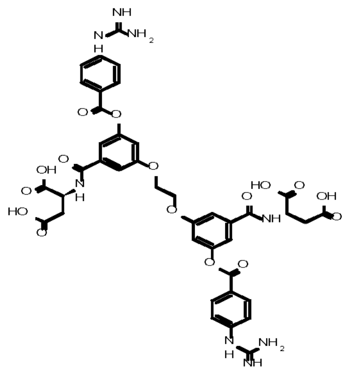 | 10 | 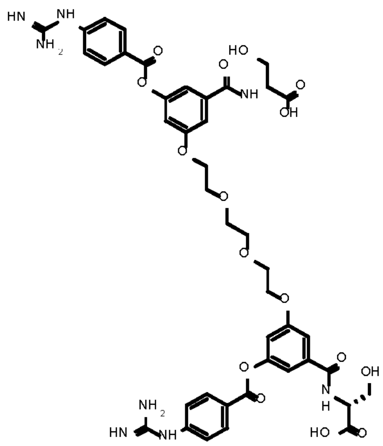 |
| 11 | 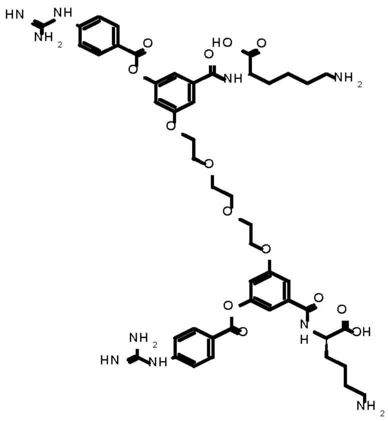 | 12 | 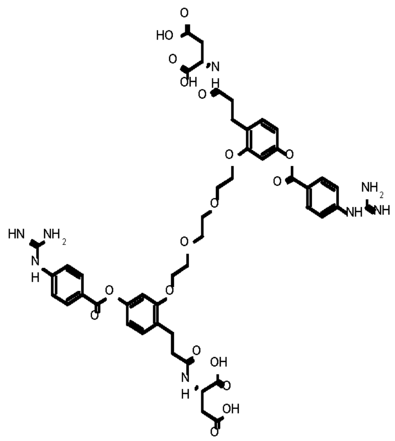 |
| 13 | 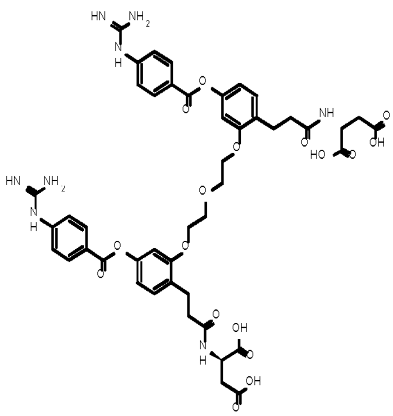 | 14 | 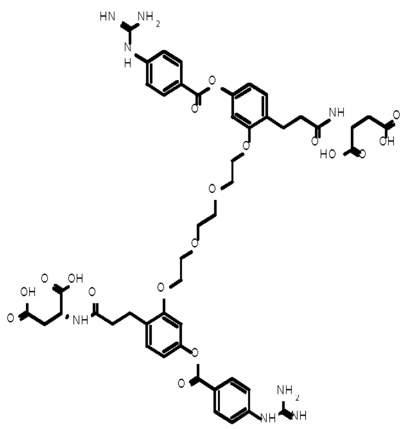 |

(continued)

| Example No. | Structural formula | | |
|---|---|---|---|
| 15 | 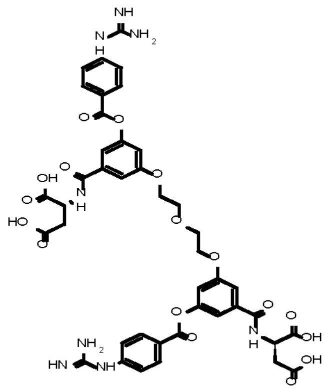 | 16 | 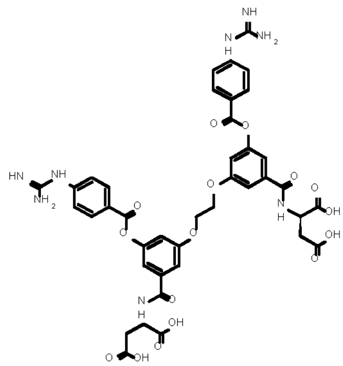 |
| 17 | 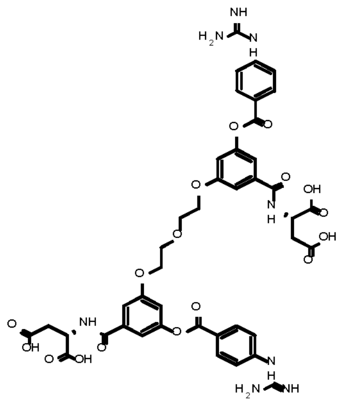 | 18 | 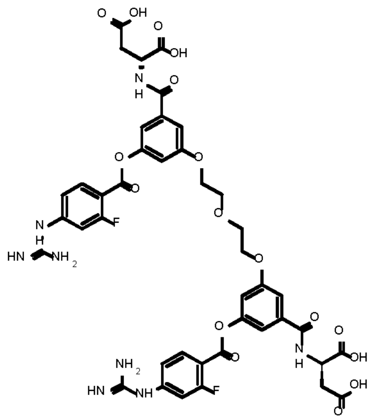 |
| 19 | 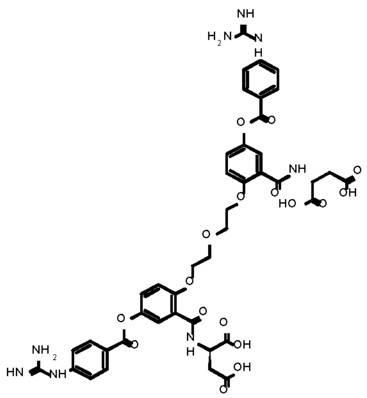 | 20 | 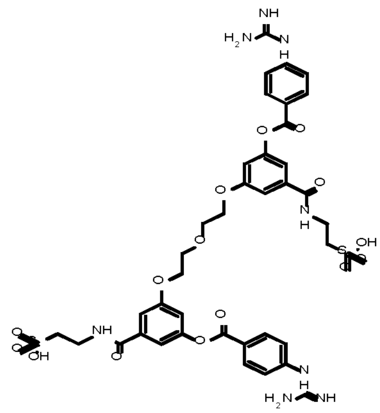 |
| 21 | 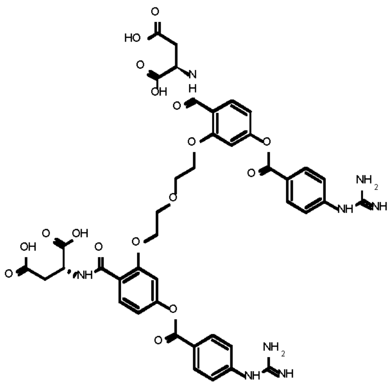 | 22 | 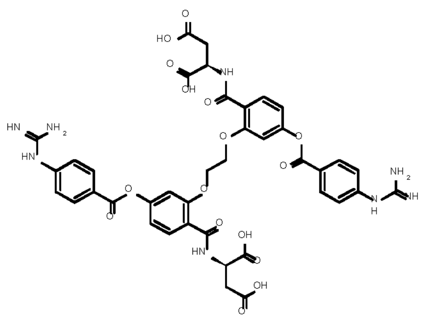 |

(continued)

| Example No. | Structural formula | | |
|---|---|---|---|
| 23 | 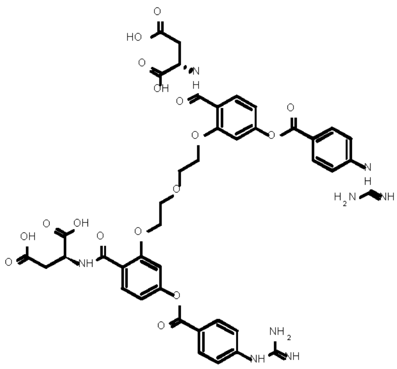 | | |

Test Example 1: Human enteropeptidase inhibitory activity

Human recombinant enteropeptidase (#REN-260, ITSI-Biosciences LLC) was diluted with an assay buffer (50 mM Tricine, pH 8.0, 0.01 (w/v)%, Tween 20, 10 mM $CaCl_2$) to prepare a 24 mU/mL enzyme solution. Subsequently, 5FAM-Abu-Gly-Asp-Asp-Asp-Lys-Ile-Val-Gly-Gly-Lys(CPQ2)-Lys-Lys-$NH_2$ (CPC Scientific Inc.) was diluted with the assay buffer to prepare a 2.1 μM substrate solution. A test compound is dissolved in DMSO into a 10 mM solution, and the solution was diluted with the assay buffer to give a compound solution. To a 384-well black plate (#784076, Greiner Bio-One), 5 μL of the compound solution and 5 μL of the substrate solution were added and mixed, and 5 μL of the enzyme solution was then added to the mixture, followed by mixing to start the reaction. The fluorescence intensity was measured with a fluorescence plate reader EnVision (The Perkin-Elmer Corp.) at an excitation wavelength of 485 nm and a fluorescence wavelength of 535 nm. The same reaction as above was performed without the addition of the test compound (test compound non-addition group). Furthermore, the same reaction as above was performed without the addition of the test compound and the enzyme (control group). The inhibition rate was calculated using the fluorescence intensity at 2 hours after the start of the reaction by the following expression:

Inhibition rate (%) = (1-((fluorescence intensity of test compound addition group) - (fluorescence intensity of control group))/((fluorescence intensity of test compound non-addition group) - (fluorescence intensity of control group))) × 100

The results are shown in Table 4.

[Table 4]

| Test compound (Example No.) | Inhibition rate at 1 μM |
|---|---|
| 1 | 98% |
| 2 | 98% |
| 3 | 98% |
| 4 | 100% |
| 5 | 99% |
| 6 | 100% |
| 7 | 100% |
| 8 | 100% |
| 9 | 100% |
| 10 | 92% |

(continued)

| Test compound (Example No.) | Inhibition rate at 1 μM |
|---|---|
| 11 | 60% |
| 12 | 100% |
| 13 | 100% |
| 14 | 100% |
| 15 | 100% |
| 16 | 100% |
| 17 | 100% |
| 18 | 100% |
| 19 | 100% |
| 20 | 99% |
| 21 | 100% |

As shown above, it was demonstrated that the invention compounds have excellent enteropeptidase inhibitory activities.

Test Example 2: Fecal protein concentration-increasing test using high fat diet-fed mouse

High fat diet-fed mice (D12451 diet, male, 10- to 50-week old) were orally administered with a 0.5% methyl cellulose suspension containing a test compound (compound administration group, five mice per group) or a 0.5% methyl cellulose suspension (compound non-administration group (vehicle group), five mice per group), and whole feces were collected on the first day of administration. Dried feces were dissolved in 0.5 N NaOH, followed by centrifugation at 12,000 rpm. The protein concentration in the supernatant was then quantitatively measured (Lowry method), and the amount of protein contained in 1 g of feces was calculated as the fecal protein concentration (mg/g feces). The fecal protein concentration-increasing rate was calculated by the following expression.

Fecal protein concentration-increasing rate (%) = Fecal protein concentration of compound administration group / Fecal protein concentration of vehicle group × 100

The results are shown in Table 5.

[Table 5]

| Test compound (Example No.) | Dose (mg/kg) | Fecal protein concentration-increasing rate |
|---|---|---|
| 1 | 30 | 159% |
| 2 | 30 | 142% |
| 3 | 30 | 152% |
| 4 | 30 | 181% |
| 5 | 30 | 158% |
| 6 | 30 | 203% |
| 7 | 30 | 115% |
| 8 | 30 | 210% |
| 9 | 30 | 195% |
| 10 | 30 | 179% |

(continued)

| Test compound (Example No.) | Dose (mg/kg) | Fecal protein concentration-increasing rate |
|---|---|---|
| 11 | 30 | 171% |
| 12 | 30 | 197% |
| 13 | 30 | 150% |
| 14 | 30 | 171% |
| 15 | 30 | 212% |
| 16 | 30 | 245% |
| 18 | 30 | 142% |
| 19 | 30 | 215% |
| 20 | 10 | 143% |
| 21 | 30 | 203% |

As shown above, it was demonstrated that the invention compounds have an effect of increasing the fecal protein concentration by enteropeptidase inhibitory activities.

Test Example 3: Anti-obesity effect test using DIO mouse

Diet-induced obesity (DIO) mice (D12451 diet, male, 26-week old) were orally administered with a 0.5% methyl cellulose suspension containing a test compound (30 mg/kg) (compound administration group, five mice per group) or a 0.5% methyl cellulose suspension (compound non-administration group (vehicle), five mice per group) once a day for seven days, and whole feces were collected on the eighth day of administration. Dried feces were dissolved in 0.5 N NaOH, followed by centrifugation at 12,000 rpm. The protein concentration in the supernatant was then quantitatively measured (Lowry method), and the amount of protein contained in 1 g of feces was calculated as the fecal protein concentration (mg/g feces). The fecal protein concentration-increasing rate was calculated by the following expression.

Fecal protein concentration-increasing rate (%) = Fecal protein concentration of compound administration group / Fecal protein concentration of vehicle group × 100

The fecal protein concentration-increasing rate and the averages of the body weights at the start of administration and after administration for seven days are shown in Table 6.

Also, DIO mice (D12451 diet, male, 59-week old) were orally administered with a 0.5% methyl cellulose suspension containing a test compound (20 mg/kg) (compound administration group, five mice per group) or a 0.5% methyl cellulose suspension (compound non-administration group (vehicle), five mice per group) once a day for seven days, and whole feces were collected on the eighth day of administration. The fecal protein concentration (mg/g feces) was calculated by the method described above. The fecal protein concentration-increasing rate and the averages of the body weights at the start of administration and after administration for seven days are shown in Table 7.

[Table 6]

| Test compound (Example No.) | Dose (mg/kg) | Fecal protein concentration-increasing rate (%) | Body weight (g) | |
|---|---|---|---|---|
| | | | At start of administration | After 7-day administration |
| vehicle | 0 | 100 | 46.1 | 45.8 |
| 12 | 30 | 280 | 45.4 | 44.7 |

(continued)

| Test compound (Example No.) | Dose (mg/kg) | Fecal protein concentration-increasing rate (%) | Body weight (g) | |
|---|---|---|---|---|
| | | | At start of administration | After 7-day administration |
| 15 | 30 | 250 | 46.0 | 43.4 |
| 16 | 30 | 271 | 45.7 | 44.7 |
| 17 | 30 | 252 | 45.6 | 44.3 |

[Table 7]

| Test compound (Example No.) | Dose (mg/kg) | Fecal protein concentration-increasing rate (%) | Body weight (g) | |
|---|---|---|---|---|
| | | | At start of administration | After 7-day administration |
| vehicle | 0 | 100 | 57.7 | 57.8 |
| 15 | 20 | 227 | 56.6 | 54.0 |
| 21 | 20 | 248 | 56.7 | 53.6 |
| 22 | 20 | 221 | 56.5 | 55.1 |

As shown above, it was demonstrated that the invention compounds show an effect of decreasing body weight and have an anti-obesity effect by enteropeptidase inhibition.

## Claims

**1.** A compound represented by the formula (I) or a salt thereof:

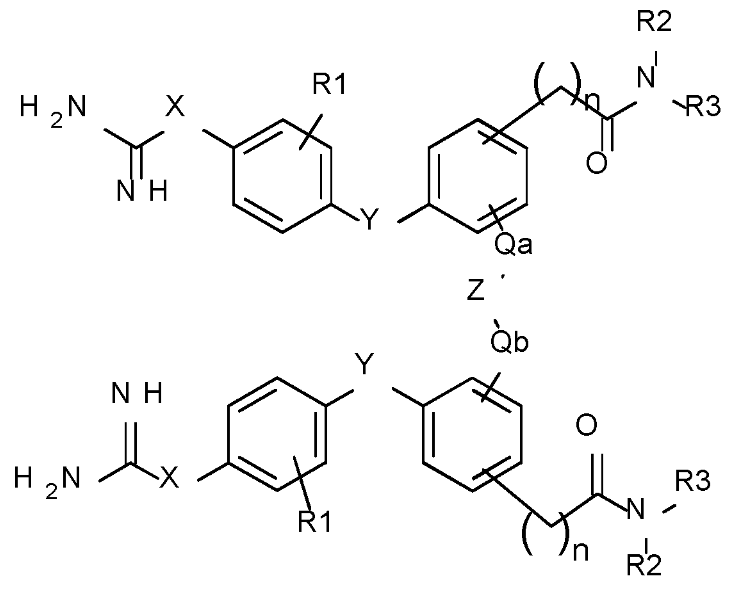

(I)

wherein each symbol represents the following meaning:

R1: H, halogen, a $C_{1-6}$ alkyl group optionally having a substituent(s), or a $C_{1-6}$ alkoxy group optionally having a substituent(s);
R2: H or a $C_{1-6}$ alkyl group optionally substituted by a carboxyl group;
R3: a $C_{1-8}$ alkyl group having one substituent selected from the group consisting of a carboxyl group, a sulfonic acid group, and a phosphoric acid group and optionally further substituted by one substituent selected from the group consisting of a carboxyl group, NHR4 and a hydroxy group;
R4: H or a $C_{1-6}$ alkyl group optionally having a substituent(s);
n: an integer of 0 to 2;
X: a bond or NH;
Y: **-C (=O) O- or **-OC (=O)- (wherein ** is bonded to the benzene ring substituted by a guanidino group or a carbamimidoyl group);
Qa and Qb: the same or different, $-(CH_2)_mO$-*, $-(CH_2)_mNR5$-*, $-(CH_2)_mNR5C(=O)$-*, -C(=O)NR5-*, -S-*, or $-SO_2$-* (wherein * is bonded to Z);
R5: H or a $C_{1-6}$ alkyl group optionally having a substituent(s);
m: an integer of 0 to 6;
Z: $-[(CR6aR6b)_q-A]_r-(CR7aR7b)_s-$;
A: O, S, $SO_2$, NR8, -C (=O) -, -OC(=O)-, -C(=O)O-,-NR8C(=O)-, or C(=O)-NR8;
R6a, R6b, R7a and R7b: the same or different, H, halogen, a hydroxy group, or a $C_{1-6}$ alkyl group optionally having a substituent(s), or a $C_{1-6}$ alkoxy group optionally having a substituent(s);
R8: H or a $C_{1-6}$ alkyl group optionally having a substituent(s);
q: an integer of 1 to 6;
r: an integer of 0 to 50; and
s: an integer of 1 to 6.

2. The compound according to claim 1 or a salt thereof, wherein X is NH, and Y is **-C(=O)O-.

3. The compound according to claim 1 or 2 or a salt thereof, wherein each of R1 and R2 is a hydrogen atom, R3 is a $C_{1-8}$ alkyl group substituted by two carboxyl groups.

4. The compound according to any one of claims 1 to 3 or a salt thereof, wherein each of Qa and Qb is $-(CH_2)_mO$-*, m is an integer of 0 to 3, Z is $-[(CR6aR6b)_q-O]_r-(CR7aR7b)_s-$, each of q and s is an integer of 1 to 6, and r is an integer of 0 to 10.

5. The compound according to claim 1 or a salt thereof, wherein each of Qa and Qb is O, Z is $-[(CH_2)_2-O]r-$ $(CH_2)_2-$,

r is an integer of 0 to 3, X is NH, Y is **-C(=O)O-, each of R1 and R2 is a hydrogen atom, R3 is a $C_{1-8}$ alkyl group substituted by two carboxyl groups, and n is 0.

**6.** (2S)-2-[[3-[2-[2-[2-[3-[[(1S)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof.

**7.** (2R)-2-[[3-[2-[2-[2-[3-[[(1R)-1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof.

**8.** (2S)-2-[[3-[2-[2-[3-[[(1S)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof.

**9.** (2R)-2-[[3-[2-[2-[3-[[(1R)-1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof.

**10.** (2R)-2-[[3-[2-[3-[[(1R)-1,2-Dicarboxyethyl]carbamoyl]-5-(4-guanidinobenzoyl)oxyphenoxy]ethoxy]-5-(4-guanidinobenzoyl)oxybenzoyl]amino]butanedioic acid or a salt thereof.

**11.** (2S)-2-[[3-[2-[3-[[(1S)-1,2-Dicarboxyethyl]carbamoyl]-5-(4-guanidinobenzoyl)oxyphenoxy]ethoxy]-5-(4-guanidinobenzoyl)oxybenzoyl]amino]butanedioic acid or a salt thereof.

**12.** (2R) -2- [[2-[2-[2-[2-[[(1R)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof.

**13.** (2S)-2-[[2-[2-[2-[2-[[(1S)-1,2-Dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof.

**14.** (2R)-2-[[2-[2-[2-[[(1R)-1,2-Dicarboxyethyl]carbamoyl]-5-(4-guanidinobenzoyl)oxyphenoxy]ethoxy]-4-(4-guanidinobenzoyl)oxybenzoyl]amino]butanedioic acid or a salt thereof.

**15.** 2-[[3-[2-[2-[2-[3-[[1,2-Dicarboxyethyl]carbamoyl]-5-(4-guanidinobenzoyl)oxyphenoxy]ethoxy]ethoxy]ethoxy]-5-(4-guanidinobenzoyl)oxybenzoyl]amino]butanedioic acid or a salt thereof, or
2-[[3-[2-[3-[[1,2-dicarboxyethyl]carbamoyl]-5-(4-guanidinobenzoyl)oxyphenoxy]ethoxy]-5-(4-guanidinobenzoyl)oxybenzoyl]amino]butanedioic acid or a salt thereof.

**16.** 2-[[3-[2-[2-[3-[[1,2-Dicarboxyethyl]carbamoyl]-5-(4-guanidinobenzoyl)oxyphenoxy]ethoxy]ethoxy]-5-(4-guanidinobenzoyl)oxybenzoyl]amino]butanedioic acid or a salt thereof;
2-[[2-[2-[2-[2-[[1,2-dicarboxyethyl]carbamoyl]-5-(4-guanidinobenzoyl)oxyphenoxy]ethoxy]ethoxy]-4-(4-guanidinobenzoyl)oxybenzoyl]amino]butanedioic acid or a salt thereof; or
2-[[2-[2-[2-[[1,2-dicarboxyethyl]carbamoyl]-5-(4-guanidinobenzoyl)oxyphenoxy]ethoxy]-4-(4-guanidinobenzoyl)oxybenzoyl]amino]butanedioic acid or a salt thereof.

**17.** A medicament comprising a compound according to any one of claims 1 to 16 or a salt thereof.

**18.** The medicament according to claim 17, wherein the medicament is an enteropeptidase inhibitor.

**19.** The medicament according to claim 17, wherein the medicament is an agent for preventing or treating obesity.

**20.** The medicament according to claim 17, wherein the medicament is an agent for preventing or treating diabetes mellitus.

**21.** A method for preventing or treating obesity in a mammal, comprising administering an effective amount of a compound according to claim 1 or a salt thereof to the mammal.

**22.** A method for preventing or treating diabetes mellitus in a mammal, comprising administering an effective amount of a compound according to claim 1 or a salt thereof to the mammal.

**23.** A method for inhibiting enteropeptidase in a mammal, comprising administering an effective amount of a compound

according to claim 1 or a salt thereof to the mammal.

**24.** Use of a compound according to claim 1 or a salt thereof for manufacturing a medicament for preventing or treating obesity.

**25.** Use of a compound according to claim 1 or a salt thereof for manufacturing a medicament for preventing or treating diabetes mellitus.

**26.** The compound according to claim 1 or a salt thereof for use in the prevention or treatment of obesity.

**27.** The compound according to claim 1 or a salt thereof for use in the prevention or treatment of diabetes mellitus.

**INTERNATIONAL SEARCH REPORT**

International application No.
PCT/JP2019/033215

A. CLASSIFICATION OF SUBJECT MATTER
Int.Cl. C07C279/18(2006.01)i, A61K31/235(2006.01)i, A61P3/04(2006.01)i, A61P3/10(2006.01)i, A61P43/00(2006.01)i, C07C257/18(2006.01)i

According to International Patent Classification (IPC) or to both national classification and IPC

B. FIELDS SEARCHED

Minimum documentation searched (classification system followed by classification symbols)
Int.Cl. C07C279/18, A61K31/235, A61P3/04, A61P3/10, A61P43/00, C07C257/18

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus(STN), REGISTRY(STN), JSTPlus(JDreamIII), JMEDPlus(JDreamIII), JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | MATSUSHIMA, Masashi et al., Biomedical Research, 2001, 22(5), 257-260 | 1-27 |
| A | JP 2017-505823 A (TAKEDA PHARMACEUTICAL COMPANY LIMITED) 23 February 2017, entire text & US 2015/0225354 A1, whole document & US 2016/0347724 A1 & WO 2015/122188 A1 & EP 3105228 A1 & TW 201609677 A & AU 2015216439 A & CA 2939675 A & KR 10-2016-0113299 A & CN 106170487 A | 1-27 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13.09.2019 | Date of mailing of the international search report<br>01.10.2019 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2019/033215

C (Continuation). DOCUMENTS CONSIDERED TO BE RELEVANT

| Category* | Citation of document, with indication, where appropriate, of the relevant passages | Relevant to claim No. |
|---|---|---|
| A | WO 2015/122187 A1 (TAKEDA PHARMACEUTICAL COMPANY LIMITED) 20 August 2015, entire text & JP 2017-506627 A & JP 2018-197254 A & US 2016/0355494 A1, whole document & US 2015/0225361 A1 & EP 3105213 A1 & AU 2015216438 A & CA 2938191 A & CN 105980370 A & KR 10-2016-0116002 A & TW 201607936 A | 1-27 |
| A | WO 2016/158788 A1 (TAKEDA PHARMACEUTICAL COMPANY LIMITED) 06 October 2016, entire text & US 2018/0072694 A1, whole document & EP 3275874 A1 | 1-27 |
| A | WO 2016/104630 A1 (TAKEDA PHARMACEUTICAL COMPANY LIMITED) 30 June 2016, entire text & US 2017/0349605 A1, whole document & EP 3239142 A1 | 1-27 |
| A | WO 2011/071048 A1 (AJINOMOTO CO., INC.) 16 June 2011, entire text & US 2012/0283222 A1, whole document & US 2015/0011511 A1 & US 2015/0313889 A1 & EP 2511271 A1 & CN 102822154 A | 1-27 |
| P, A | WO 2019/088270 A1 (UBE INDUSTRIES, LTD.) 09 May 2019, entire text (Family: none) | 1-27 |
| P, A | YASHIRO, Hiroaki et al., Diabetes, Obesity and Metabolism, 30 May 2019, 21(10), 2228-2239 | 1-27 |
| E, A | SASAKI, Masako et al., Pharmacology Research & Perspectives, 04 September 2019, 7(5), 1-11 | 1-27 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015122187 A
- WO 2016158788 A
- WO 2016104630 A
- WO 2011071048 A
- WO 2007013694 A
- WO 2007018314 A
- WO 2008093639 A
- WO 2008099794 A
- WO 2004041266 A
- WO 2004106276 A
- WO 2005063729 A
- WO 2005063725 A
- WO 2005087710 A
- WO 2005095338 A
- WO 2007013689 A
- WO 2008001931 A
- WO 2006112549 A
- WO 2007028135 A
- WO 2008047821 A
- WO 2008050821 A
- WO 2008136428 A
- WO 2008156757 A
- WO 0114372 A
- WO 2004039365 A
- WO 9710224 A
- WO 0206234 A
- WO 2004048363 A
- WO 2005030740 A
- WO 2005058823 A
- WO 2005113504 A

### Non-patent literature cited in the description

- The Fifth Series of Experimental Chemistry. The Chemical Society of Japan, vol. 13-19
- Shin Jikken Kagaku Koza (New Experimental Chemistry in English. Chemical Society of Japan, vol. 14-15
- **L. F. TIETZE ; TH. EICHER.** Reactions and Syntheses: In the Organic Chemistry Laboratory. Nankodo Co., Ltd,
- The Reaction Mechanism and Essence. Organic Name Reactions. Kodansha Ltd,
- Organic Syntheses Collective. John Wiley & Sons, Inc, vol. I-VII
- **JIE JACK LI.** Modern Organic Synthesis in the Laboratory: A Collection of Standard Experimental Procedures. Oxford University Press
- Comprehensive Heterocyclic Chemistry III. Elsevier, vol. 1-14
- **KIYOSHI TOMIOKA.** Strategic Applications of Named Reactions in Organic Synthesis. Kagaku-Dojin Publishing Company, Inc,
- Comprehensive Organic Transformations. VCH Publishers, Inc, 1989
- **THEODORA W. GREENE ; PETER G. M. WUTS.** Protective Groups in Organic Synthesis. Wiley-Interscience, 2007
- **P.J. KOCIENSKI.** Protecting Groups. Thieme Medical Publishers, 2004
- *The Japan Diabetes Society reported the diagnostic criteria of diabetes mellitus,* 1999
- National Cholesterol Education Program: Executive Summary of the Third Report of National Cholesterol Education Program (NCEP) Expert Panel on Detection, Evaluation, and Treatment of High Blood Cholesterol in Adults (Adults Treatment Panel III). *The Journal of the American Medical Association,* 2001, vol. 285, 2486-2497